# EUROPEAN PATENT SPECIFICATION

(11) **EP 3 523 447 B1**
(45) Date of publication and mention of the grant of the patent: **22.06.2022**
(21) Application number: 17786854.4
(22) Date of filing: 04.10.2017
(51) Int. Cl.: C12Q 1/689

(54) **A METHOD FOR THE DETECTION OF LEGIONELLA**
VERFAHREN ZUR DETEKTION VON LEGIONELLEN
PROCEDE DE DETECTION DE LEGIONELLA

(30) Priority: 05.10.2016 EP 16192489
(43) Date of publication of application: 14.08.2019
(73) Proprietor: National University of Ireland Galway, Galway (IE)
(72) Inventor: BARRY, Thomas, Kinvara Co. Galway (IE); REDDINGTON, Kate, Westport Co. Mayo (IE); MINOGUE, Elizabeth, Whitegate Co. Clare (IE)
(74) Representative: FRKelly
(86) International application number: PCT/EP2017/075265
(87) International publication number: WO 2018/065497

(56) References cited:
- WO-A1-2013/187958
- WO-A2-2005/049642
- WO-A2-2011/133433
- COLLINS S ET AL: "Real-time PCR to supplement gold-standard culture-based detection of Legionella in environmental samples", JOURNAL OF APPLIED MICROBIOLOGY, vol. 119, no. 4, October 2015 (2015-10), pages 1158-1169, XP002768617,
- BENITEZ ALVARO J ET AL: "Clinical application of a multiplex real-time PCR assay for simultaneous detection of Legionella species, Legionella pneumophila, and Legionella pneumophila serogroup 1", JOURNAL OF CLINICAL MICROBIOLOGY, AMERICAN SOCIETY FOR MICROBIOLOGY, UNITED STATES, vol. 51, no. 1, 1 January 2013 (2013-01-01), pages 348-351, XP009169164, ISSN: 1098-660X, DOI: 10.1128/JCM.02510-12
- KIM SEUNG MIN ET AL: "Multiplex real-time PCR assay for Legionella species", MOLECULAR AND CELLULAR PROBES, vol. 29, no. 6, December 2015 (2015-12), pages 414-419, XP002768618, ISSN: 0890-8508
- JANCZAREK MONIKA ET AL: "PCR method for the rapid detection and discrimination ofLegionellaspp. based on the amplification ofpcs, pmtA, and 16S rRNA genes", JOURNAL OF APPLIED GENETICS: AN INTERNATIONAL JOURNAL OF GENETICS AND BREEDING, SPRINGER, GERMANY, vol. 57, no. 2, 30 September 2015 (2015-09-30), pages 251-261, XP035968926, ISSN: 1234-1983, DOI: 10.1007/S13353-015-0317-2 [retrieved on 2015-09-30]
- THUERMER ALEXANDER ET AL: "PCR-based 'serotyping' of Legionella pneumophila", JOURNAL OF MEDICAL MICROBIOLOGY, SOCIETY FOR GENERAL MICROBIOLOGY, GB, vol. 58, no. 5, 1 May 2009 (2009-05-01), pages 588-595,1, XP002567166, ISSN: 0022-2615, DOI: 10.1099/JMM.0.008508-0

## Description

### Field of the invention

This invention relates to the detection of *Legionella,* more specifically *Legionella pneumophila,* more specifically *Legionella pneumophila* serogroup 1, in a sample. Methods of detection, and assays and kits useful for detection are also disclosed.

### Background of the invention

Infections acquired in buildings, hospitals, and healthcare institutions affect approximately 2 million people, result in approximately 98,000 deaths, and cost a reported USD29 billion in the United States alone each year. The economic burden of infections acquired in hospitals in Europe is estimated to be €7 billion per annum.

Water, water distribution, and premise plumbing systems have been identified as a source of many of these infections, and can pose a significant threat to human health, especially in patients with weakened immune systems, and high dependency patients in critical care units. As infection control and prevention teams within clinical settings increasingly recognise the risks to patient health, a variety of testing and treatment technologies are being employed to attempt to eradicate pathogenic microorganisms from water. Despite the range of technologies being applied, the problem of waterborne infection, particularly from *Legionella* species, in the healthcare setting continues to persist.

Legionnaires' disease was first recognised in 1976 during an outbreak of pneumonia at an American Legion convention in Philadelphia. *Legionella* spp., the causative agent of Legionnaires disease, are responsible for outbreaks of both hospital acquired and community acquired pneumonia. In healthcare settings, there are increasing proportions of immunologically compromised patients leading to added potential for infection of patients by such opportunistic pathogenic microorganisms. The most common microorganism associated with Legionnaires disease is *Legionella pneumophila. L. pneumophila* serogroup 1 is the most clinically relevant. Currently, routine testing for *L. pneumophila* serogroup 1 is performed using traditional microbiological based techniques. This method is limited by the fastidious nature and long incubation periods required by *Legionella* spp. and also by the presence of viable but non-culturable Legionellae.

Prior art gold standard, culture-based methodologies are centuries old and are slow to deliver a result for *Legionella,* taking up to 2 weeks. Furthermore, prior art culture-based methodologies alone cannot specifically identify if *L. pneumophila* serogroup 1 is present in a sample. Serogroup 1 is the most important serogroup from a human health perspective, as it causes ~95% of infections. There are also a number of prior art biochemical and immunodetection-based kits for these microorganisms. However, they are not very sensitive and are not truly quantitative.

WO2013187958 discloses methods for detecting Legionella (such as *Legionella spp., Legionella pneumophila, Legionella pneumophila* serogroup 1, *Legionella bozemanii, Legionella dumoffii, Legionella feeleii, Legionella longbeachae,* and/or *Legionella micdadei*). Also disclosed are probes and primers for the detection of *Legionella,* and kits that contain the disclosed probes and/or primers.

WO2011133433 discloses methods for detecting *Mycoplasma pneumoniae, Chlamydophila pneumoniae,* and *Legionella* spp. Also disclosed are probes and primers for the detection of M. *pneumoniae, C. pneumoniae,* and *Legionella* spp., and kits that contain the disclosed probes and/or primers.

### Summary of the invention

According to the present invention, there is provided a method for identifying the presence or quantity of *Legionella pneumophila* serogroup 1 in a sample, the method comprising the steps of:
(a) detecting the presence or quantity of at least part of the nucleic acid sequence of SEQ ID NO. 1, or an expression product thereof; and
(b) correlating the presence or quantity of the at least part of the nucleic acid sequence of SEQ ID NO. 1 or expression product thereof to the presence, or quantity of *Legionella pneumophila* serogroup 1 in the sample.

Optionally, the method further comprises the steps of:
(ai) detecting the presence or quantity of at least part of the nucleic acid sequence of the *smpB* gene, or an expression product thereof; and
correlating the presence or quantity of the at least part of the nucleic acid sequence of the *smpB* gene or expression product thereof to the presence or quantity of *Legionella pneumophila* in the sample.

Optionally or additionally, the method further comprises the steps of:
(aii) detecting the presence or quantity of at least part of the nucleic acid sequence of the *ssrA* gene, or an expression product thereof; and
correlating the presence or quantity of the at least part of the nucleic acid sequence of the *ssrA* gene or expression product thereof to the presence or quantity of *Legionella* genus in the sample.

Optionally, the method comprises the steps of:
(a) detecting the presence or quantity of at least part of the nucleic acid sequence of SEQ ID NO. 1 or an expression product thereof;
   (ai) detecting the presence or quantity of at least part of the nucleic acid sequence of the *smpB* gene or an expression product thereof;
(b) correlating the presence or quantity of the at least part of the nucleic acid sequence of SEQ ID NO. 1 or expression product thereof to the presence, or quantity of *Legionella pneumophila* serogroup 1 in the sample; and correlating the presence or quantity of the at least part of the nucleic acid sequence of the *smpB* gene or expression product thereof to the presence or quantity of *Legionella pneumophila* in the sample.

Optionally, the method comprises the steps of:
(a) detecting the presence or quantity of at least part of the nucleic acid sequence of SEQ ID NO. 1 or an expression product thereof;
   (aii) detecting the presence or quantity of at least part of the nucleic acid sequence of the *ssrA* gene or an expression product thereof;
(b) correlating the presence or quantity of the at least part of SEQ ID NO. 1 or expression product thereof to the presence or quantity of *Legionella pneumophila* serogroup 1 in the sample; and correlating the presence or quantity of the at least part of the nucleic acid sequence of the *ssrA* gene or expression product thereof to the presence or quantity of *Legionella genus* in the sample.

Optionally, the method comprises the steps of:
(a) detecting the presence or quantity of at least part of the nucleic acid sequence of SEQ ID NO. 1 or an expression product thereof;
   (ai) detecting the presence or quantity of at least part of the nucleic acid sequence of the *smpB* gene or an expression product thereof;
   (aii) detecting the presence or quantity of at least part of the nucleic acid sequence of the *ssrA* gene or an expression product thereof;
(b) correlating the presence or quantity of the at least part of the nucleic acid sequence of SEQ ID NO. 1 or expression product thereof to the presence or quantity of *Legionella pneumophila* serogroup 1 in the sample; correlating the presence or quantity of the at least part of the nucleic acid sequence of the *smpB* gene or expression product thereof to the presence or quantity of *Legionella pneumophila* in the sample; and correlating the presence or quantity of the at least part of the nucleic acid sequence of the *ssrA* gene or expression product thereof to the presence or quantity of *Legionella* genus in the sample.

Optionally, the presence of the at least part of the nucleic acid sequence of SEQ ID NO. 1 or an expression product thereof indicates the presence of *Legionella pneumophila* serogroup 1. Optionally, the quantity of the at least part of the nucleic acid sequence of SEQ ID NO. 1 or an expression product thereof indicates the quantity of *Legionella pneumophila* serogroup 1.

Optionally, the at least part of the nucleic acid sequence of SEQ ID NO. 1 comprises at least part of the nucleic acid sequence which encodes spore coat polysaccharide biosynthesis protein E (neuB).

Optionally, the at least part of the nucleic acid sequence of SEQ ID NO. 1 comprises at least part of the nucleic acid sequence defined by Genbank Accession Number AE017354; base pairs 839972 to 840171.

Optionally, the at least part of the nucleic acid sequence of SEQ ID NO. 1 comprises a nucleic acid sequence having at least 85% sequence identity with at least part of the nucleic acid sequence defined by any one of SEQ ID NOs 1 and 21-48.

Optionally, the at least part of the nucleic acid sequence of SEQ ID NO. 1 comprises a nucleic acid sequence having at least 85%, 87.5%, 88%, 88.5%, 94%, 94.5%, 95%, or 100% sequence identity with at least part of the nucleic acid sequence defined by any one of SEQ ID NOs 1 and 21-48.

Optionally, the at least part of the nucleic acid sequence of SEQ ID NO. 1 comprises a nucleic acid sequence defined by any one of SEQ ID NOs 1 and 21-48.

Optionally, the detecting step (a) comprises: contacting the sample with at least one primer capable of hybridizing to at least part of the at least part of the nucleic acid sequence of SEQ ID NO. 1, optionally at least part of the nucleic acid sequence defined by any one of SEQ ID NOs 1 and 21-48; under conditions suitable for an *in vitro* amplification, optionally an *in vitro* amplification selected from the group comprising: polymerase chain reaction (PCR), Nucleic Acid Sequence Based Amplification (NASBA), Rolling Circle Amplification (RCA), Ligase Chain Reaction (LCR), Signal Mediated Amplification of RNA Technology (SMART), Strand Displacement Amplification (SDA), Loop Mediated Isothermal Amplification (LAMP), Isothermal Multiple Displacement Amplification (IMDA), Helicase-Dependent Amplification (HDA), Single Primer Isothermal Amplification (SIPA), circular Helicase Dependent Amplification (cHDA), and Next Generation Sequencing (NGS).

Optionally, polymerase chain reaction (PCR) is selected from real-time PCR and quantitative real-time PCR.

Optionally, the polymerase chain reaction (PCR), optionally the real-time PCR, optionally the quantitative real-time PCR, further comprises pyrosequencing.

Optionally, the detecting step (a) comprises: contacting the sample with at least one primer having a nucleic acid sequence defined by at least one of SEQ ID NO. 2, SEQ ID NO. 3, and the reverse complement each thereof; under conditions suitable for an *in vitro* amplification, optionally an *in vitro* amplification selected from the group comprising: polymerase chain reaction (PCR), Nucleic Acid Sequence Based Amplification (NASBA), Rolling Circle Amplification (RCA), Ligase Chain Reaction (LCR), Signal Mediated Amplification of RNA Technology (SMART), Strand Displacement Amplification (SDA), Loop Mediated Isothermal Amplification (LAMP), Isothermal Multiple Displacement Amplification (IMDA), Helicase-Dependent Amplification (HDA), Single Primer Isothermal Amplification (SIPA), circular Helicase Dependent Amplification (cHDA), and Next Generation Sequencing (NGS).

Optionally, polymerase chain reaction (PCR) is selected from real-time PCR and quantitative real-time PCR.

Optionally, the polymerase chain reaction (PCR), optionally the real-time PCR, optionally the quantitative real-time PCR, further comprises pyrosequencing.

Optionally, the at least one primer has a final concentration of about 0.05-0.5 µM, optionally 0.05 µM, optionally 0.5 µM.

Optionally, the detecting step (a) comprises: contacting the sample with at least one probe capable of hybridizing to at least part of the at least part of SEQ ID NO. 1, optionally at least part of the nucleic acid sequence defined by any one of SEQ ID NOs 1 and 21-48, under conditions suitable for hybridization.

Optionally, the detecting step (a) comprises: contacting the sample with at least one probe having a nucleic acid sequence defined by at least one of SEQ ID NO. 4 and the reverse complement thereof, under conditions suitable for hybridisation.

Optionally, the at least one probe comprises at least one marker, optionally at least one dye, optionally at least one fluorophore, optionally hexachlorofluorescein.

Optionally or additionally, the at least one probe comprises at least one fluorescence quenching dye.

Optionally the polymerase chain reaction, or real-time PCR, or quantitative real-time PCR, cycling parameters comprise about 10 minutes incubation at about 95°C, optionally or additionally about 50 cycles of about 95°C for about 10 seconds and about 60°C for about 30 seconds, optionally or additionally followed by a cooling step at about 40°C for about 10 seconds.

Optionally, the at least one probe has a final concentration of about 0.02-0.8 µM, optionally 0.02 µM, optionally 0.08 µM, optionally 0.2 µM, optionally 0.8 µM.

Optionally, the presence of the at least part of the nucleic acid sequence of the *smpB* gene or expression product thereof indicates the presence of *Legionella pneumophila.* Optionally, the presence of the at least part of the nucleic acid sequence of the *smpB* gene or expression product thereof, and the presence of the at least part of the nucleic acid sequence of SEQ ID NO. 1 or expression product thereof, indicates the presence of each of *Legionella pneumophila* serogroup 1 and *Legionella pneumophila.*

The presence of the at least part of the nucleic acid sequence of the *smpB* gene or expression product thereof, and the absence of the at least part of the nucleic acid sequence of SEQ ID NO. 1 or expression product thereof, can indicate the presence of *Legionella pneumophila* and the absence of *Legionella pneumophila* serogroup 1.

The presence of the at least part of the nucleic acid sequence of the *smpB* gene or expression product thereof, and the absence of the at least part of the nucleic acid sequence of SEQ ID NO. 1 or an expression product thereof, can indicate the presence of at least one organism selected from *Legionella pneumophila* serogroups 2-16.

The absence of the at least part of the nucleic acid sequence of the *smpB* gene or expression product thereof, and the presence of the at least part of SEQ ID NO. 1 or expression product thereof, can indicate at least one false result selected from: a false negative result in detecting step (ai), and a false positive result in detecting step (a).

The absence of the at least part of the nucleic acid sequence of the *smpB* gene or expression product thereof, and the absence of the at least part of the nucleic acid sequence of SEQ ID NO. 1 or expression product thereof, can indicate the absence of each of *Legionella pneumophila and Legionella pneumophila* serogroup 1.

Optionally, the at least part of the nucleic acid sequence of the *smpB* gene comprises at least part of the nucleic acid sequence which encodes small protein B (smpB).

Optionally, the at least part of the nucleic acid sequence of the *smpB* gene comprises at least part of the nucleic acid sequence defined by Genbank Accession number AE017354; base pairs 3213961 to 3214076.

Optionally, the at least part of the nucleic acid sequence of the *smpB* gene comprises at least part of the nucleic acid sequence defined by SEQ ID NO. 6.

Optionally, the detecting step (ai) comprises: contacting the sample with at least one primer capable of hybridizing to at least part of the nucleic acid sequence of the *smpB* gene, optionally at least part of the nucleic acid sequence defined by SEQ ID NO. 6, under conditions suitable for an *in vitro* amplification, optionally an *in vitro* amplification selected from the group comprising: polymerase chain reaction (PCR), Nucleic Acid Sequence Based Amplification (NASBA), Rolling Circle Amplification (RCA), Ligase Chain Reaction (LCR), Signal Mediated Amplification of RNA Technology (SMART), Strand Displacement Amplification (SDA), Loop Mediated Isothermal Amplification (LAMP), Isothermal Multiple Displacement Amplification (IMDA), Helicase-Dependent Amplification (HDA), Single Primer Isothermal Amplification (SIPA), circular Helicase Dependent Amplification (cHDA), and Next Generation Sequencing (NGS).

Optionally, polymerase chain reaction (PCR) is selected from real-time PCR and quantitative real-time PCR.

Optionally, the polymerase chain reaction (PCR), optionally the real-time PCR, optionally the quantitative real-time PCR, further comprises pyrosequencing.

Optionally, the detecting step (ai) comprises: contacting the sample with at least one primer having a nucleic acid sequence defined by at least one of SEQ ID NO. 7, SEQ ID NO. 8, SEQ ID NO. 13, and the reverse complement each thereof, under conditions suitable for an *in vitro* amplification, optionally an *in vitro* amplification selected from the group comprising: polymerase chain reaction (PCR), Nucleic Acid Sequence Based Amplification (NASBA), Rolling Circle Amplification (RCA), Ligase Chain Reaction (LCR), Signal Mediated Amplification of RNA Technology (SMART), Strand Displacement Amplification (SDA), Loop Mediated Isothermal Amplification (LAMP), Isothermal Multiple Displacement Amplification (IMDA), Helicase-Dependent Amplification (HDA), Single Primer Isothermal Amplification (SIPA), circular Helicase Dependent Amplification (cHDA), and Next Generation Sequencing (NGS).

Optionally, the detecting step (ai) comprises: contacting the sample with at least one primer having a nucleic acid sequence defined by at least one of SEQ ID NO. 7, SEQ ID NO. 8, SEQ ID NO. 13, SEQ ID NO. 49, SEQ ID NO. 50, and the reverse complement each thereof, under conditions suitable for an *in vitro* amplification, optionally an *in vitro* amplification selected from the group comprising: polymerase chain reaction (PCR), Nucleic Acid Sequence Based Amplification (NASBA), Rolling Circle Amplification (RCA), Ligase Chain Reaction (LCR), Signal Mediated Amplification of RNA Technology (SMART), Strand Displacement Amplification (SDA), Loop Mediated Isothermal Amplification (LAMP), Isothermal Multiple Displacement Amplification (IMDA), Helicase-Dependent Amplification (HDA), Single Primer Isothermal Amplification (SIPA), circular Helicase Dependent Amplification (cHDA), and Next Generation Sequencing (NGS).

Optionally, polymerase chain reaction (PCR) is selected from real-time PCR and quantitative real-time PCR.

Optionally, the polymerase chain reaction (PCR), optionally the real-time PCR, optionally the quantitative real-time PCR, further comprises pyrosequencing.

Optionally, the at least one primer has a final concentration of about 0.05-0.5 µM, optionally 0.05 µM, optionally 0.5 µM.

Optionally the polymerase chain reaction, or real-time PCR, or quantitative real-time PCR, cycling parameters comprise about 5 minutes incubation at about 95°C, optionally or additionally 50 cycles at about 95°C for about 10 seconds and about 60°C for about 30 seconds, optionally or additionally followed by a cooling step at about 40°C for about 10 seconds.

Optionally, the detecting step (ai) comprises: contacting the sample with at least one probe capable of hybridizing to at least part of the nucleic acid sequence of the *smpB* gene, optionally at least part of the nucleic acid sequence defined by SEQ ID NO. 6, under conditions suitable for hybridisation. Optionally, the detecting step (ai) comprises: contacting the sample with at least one probe having a nucleic acid sequence defined by at least one of SEQ ID NO. 9 or the reverse complement thereof, under conditions suitable for hybridisation.

Optionally, the detecting step (ai) comprises: contacting the sample with at least one probe having a nucleic acid sequence defined by at least one of SEQ ID NO. 9, SEQ ID NO 51, SEQ ID NO. 52, and the reverse complement each thereof, under conditions suitable for hybridisation.

Optionally, the at least one probe comprises at least one marker, optionally at least one dye, optionally at least one fluorophore, optionally rhodamine.

Optionally, or additionally, the at least one probe comprises at least one fluorescence quenching dye.

Optionally, the at least one probe has a final concentration of about 0.02-0.8 µM, optionally 0.02 µM, optionally 0.08 µM, optionally 0.2 µM, optionally 0.8 µM.

Optionally, the presence of the at least part of the nucleic acid sequence of the *ssrA* gene or expression product thereof indicates the presence of *Legionella* genus. Optionally, the presence of the at least part of the nucleic acid sequence of the *ssrA* gene or expression product thereof, and the presence of the at least part of SEQ ID NO. 1 or expression product thereof, indicates the presence of each of the *Legionella* genus and *Legionella pneumophila* serogroup 1.

The presence of the at least part of the nucleic acid sequence of the *ssrA* gene or expression product thereof, and the absence of the at least part of the nucleic acid sequence of SEQ ID NO. 1 or expression product thereof, can indicate the presence of the *Legionella* genus and the absence of *Legionella pneumophila* serogroup 1.

The presence of the at least part of the nucleic acid sequence of the *ssrA* gene or expression product thereof, and the absence of the at least part of the nucleic acid sequence of SEQ ID NO. 1 or expression product thereof, indicates the presence of at least one organism selected from *Legionella pneumophila* serogroups 2-16, *Legionella anisa, L. birminghamensis, L. birminghamensis, L. bozemanii, L. bozemanii, L. cincinnatiensis, L. dumoffii, L. dumoffii, L. erythra, L. feeleii-1, L. feelii-2, L. gormanii, L. gormanii, L. hackeliae-1, L. hackeliae-2, L. jordanis, L. jordanis, L. lansingensis, L. longbeachae-1, L. maceachemii, L. miodadei, L. oakridgensis, L. oakridgensis, L. parisiensis, L. wadworthii, L. cherrii, L. jamestowniensis, L. londoniensis, L. taurinsis, L. moravica, L. adelaidensis, L. donaldsonii, L. gratiana, L. gresilensis, L. fairfieldensis, L. israelensis, L. fallonii, L. brunensis, L. busanensis, L. quinlivanii,* and *L. rubrilicens or other non-legionella pneumophila species.*

The absence of the at least part of the nucleic acid sequence of the *ssrA* gene or expression product thereof, and the presence of the at least part of the nucleic acid sequence of SEQ ID NO. 1 or expression product thereof, can indicate at least one false result selected from: a false negative result in detecting step (aii), and a false positive result in detecting step (a).

The absence of the at least part of the nucleic acid sequence of the *ssrA* gene or expression product thereof, and the absence of the at least part of the nucleic acid sequence of SEQ ID NO. 1 or expression product thereof, can indicate the absence of each of the *Legionella* genus and *Legionella pneumophila* serogroup 1.

Optionally, the presence of the at least part of the nucleic acid sequence of the *ssrA* gene or expression product thereof, and the presence of the at least part of the nucleic acid sequence of the *smpB* gene or expression product thereof, and the presence of the at least part of the nucleic acid sequence of SEQ ID NO. 1 or expression product thereof, indicates the presence of each of the *Legionella* genus, *Legionella pneumophila,* and *Legionella pneumophila* serogroup 1.

The presence of the at least part of the nucleic acid sequence of the *ssrA* gene or expression product thereof, and the presence of the at least part of the nucleic acid sequence of the *smpB* gene or expression product thereof, and the absence of the at least part of the nucleic acid sequence of SEQ ID NO. 1 or an expression product thereof, can indicate the presence of *Legionella* genus and *Legionella pneumophila,* and the absence of *Legionella pneumophila* serogroup 1.

The presence of the at least part of the nucleic acid sequence of the *ssrA* gene or expression product thereof, and the presence of the at least part of the nucleic acid sequence of the *smpB* gene or expression product thereof, and the absence of the at least part of the nucleic acid sequence of SEQ ID NO. 1 or expression product thereof, can indicate the presence of the *Legionella* genus and at least one organism selected from *Legionella pneumophila* serogroup 2-16.

The presence of the at least part of the nucleic acid sequence of the *ssrA* gene or expression product thereof, and the absence of the at least part of the nucleic acid sequence of the *smpB* gene or expression product thereof, and the presence of the at least part of SEQ ID NO. 1 or expression product thereof, can indicate the presence of the *Legionella* genus, and also indicates at least one false result selected from: a false negative result in detecting step (ai), a false positive result in detecting step (a).

The presence of the at least part of the nucleic acid sequence of the *ssrA* gene or expression product thereof, and the absence of the at least part of the nucleic acid sequence of the *smpB* gene or expression product thereof, and the presence of the at least part of the nucleic acid sequence of SEQ ID NO. 1 or expression product thereof, can indicatethe presence of at least one organism selected from *Legionella anisa, L. birminghamensis, L.. birminghamensis, L. bozemanii, L. bozemanii, L. cincinnatiensis, L. dumoffii, L. dumoffii, L. erythra, L. feeleii-1, L. feelii-2, L. gormanii, L. gormanii, L. hackeliae-1, L. hackeliae-2, L. jordanis, L. jordanis, L. lansingensis, L. longbeachae-1, L. maceachemii, L. miodadei, L. oakridgensis, L. oakridgensis, L. parisiensis, L. wadworthii, L. cherrii, L. jamestowniensis, L. londoniensis, L. taurinsis, L. moravica, L. adelaidensis, L. donaldsonii, L. gratiana, L. gresilensis, L. fairfieldensis, L. israelensis, L. fallonii, L. brunensis, L. busanensis, L. quinlivanii, L. rubrilicens,* or other non-legionella pneumophila species, and also can indicate at least one false result selected from: a false negative result in detecting step (ai), a false positive result in detecting step (a).

The presence of the at least part of the nucleic acid sequence of the *ssrA* gene or expression product thereof, and the absence of the at least part of the nucleic acid sequence of the *smpB* gene or expression product thereof, and the absence of the at least part of the nucleic acid sequence of SEQ ID NO. 1 or expression product thereof, can indicate the presence of at least one organism selected from the *Legionella* genus, and the absence of each of *Legionella pneumophila* species and *Legionella pneumophila* serogroup 1.

The presence of the at least part of the nucleic acid sequence of the *ssrA* gene or expression product thereof, and the absence of the at least part of the nucleic acid sequence of the *smpB* gene or expression product thereof, and the absence of the at least part of the nucleic acid sequence of SEQ ID NO. 1 or expression product thereof, can indicate the presence of at least one organism selected from *Legionella anisa, L. birminghamensis, L.. birminghamensis, L. bozemanii, L. bozemanii, L. cincinnatiensis, L. dumoffii, L. dumoffii, L. erythra, L. feeleii-1, L. feelii-2, L. gormanii, L. gormanii, L. hackeliae-1, L. hackeliae-2, L. jordanis, L. jordanis, L. lansingensis, L. longbeachae-1, L. maceachemii, L. miodadei, L. oakridgensis, L. oakridgensis, L. parisiensis, L. wadworthii, L. cherrii, L. jamestowniensis, L. londoniensis, L. taurinsis, L. moravica, L. adelaidensis, L. donaldsonii, L. gratiana, L. gresilensis, L. fairfieldensis, L. israelensis, L. fallonii, L. brunensis, L. busanensis, L. quinlivanii, L. rubrilicens,* or other non-legionella pneumophila species, and the absence of *Legionella pneumophila* species or *Legionella pneumophila* serogroup 1.

The absence of the at least part of the nucleic acid sequence of the *ssrA* gene or expression product thereof, the presence of the at least part of the nucleic acid sequence of the *smpB* gene or expression product thereof, and the presence of the at least part of the nucleic acid sequence of SEQ ID NO. 1 or expression product thereof, can indicate at least one false result selected from: a false negative result in detecting step (aii), a false positive result in detecting step (ai), and a false positive result in detecting step (a).

The absence of the at least part of the nucleic acid sequence of the *ssrA* gene or expression product thereof, the presence of the at least part of the nucleic acid sequence of the *smpB* gene or expression product thereof, and the absence of the at least part of the nucleic acid sequence of SEQ ID NO. 1 or expression product thereof, can indicateat least one false result selected from: a false negative result in detecting step (aii), a false positive result in detecting step (ai), and a false negative result in detecting step (a).

The absence of the at least part of the nucleic acid sequence of the *ssrA* gene or expression product thereof, the absence of the at least part of the nucleic acid sequence of the *smpB* gene or expression product thereof, and the absence of the at least part of the nucleic acid sequence of SEQ ID NO. 1 or expression product thereof, can indicate the absence of each of the *Legionella* genus, *Legionella pneumophila* species, and *Legionella pneumophila* serogroup 1.

Optionally, the at least part of the nucleic acid sequence of the ssrA gene comprises at least part of the nucleic acid sequence defined by Genbank Accession number AE017354; base pairs 172917 to 173144.

Optionally, the at least part of the nucleic acid sequence of the *ssrA* gene comprises at least part of the nucleic acid sequence defined by SEQ ID NO. 5.

Optionally, the detecting step (aii) comprises: contacting the sample with at least one primer capable of hybridizing to at least part of the nucleic acid sequence of the *ssrA* gene, optionally at least part of the nucleic acid sequence defined by SEQ ID NO. 5, under conditions suitable for an *in vitro* amplification, optionally an *in vitro* amplification selected from the group comprising: polymerase chain reaction (PCR), Nucleic Acid Sequence Based Amplification (NASBA), Rolling Circle Amplification (RCA), Ligase Chain Reaction (LCR), Signal Mediated Amplification of RNA Technology (SMART), Strand Displacement Amplification (SDA), Loop Mediated Isothermal Amplification (LAMP), Isothermal Multiple Displacement Amplification (IMDA), Helicase-Dependent Amplification (HDA), Single Primer Isothermal Amplification (SIPA), circular Helicase Dependent Amplification (cHDA), and Next Generation Sequencing (NGS).

Optionally, polymerase chain reaction (PCR) is selected from real-time PCR and quantitative real-time PCR.

Optionally, the polymerase chain reaction (PCR), optionally the real-time PCR, optionally the quantitative real-time PCR, further comprises pyrosequencing.

Optionally, the detecting step (aii) comprises: contacting the sample with at least one primer having a nucleic acid sequence defined by at least one of SEQ ID NO. 10, SEQ ID NO. 11, and the reverse complement each thereof, under conditions suitable for an *in vitro* amplification, optionally an *in vitro* amplification selected from the group comprising: polymerase chain reaction (PCR), Nucleic Acid Sequence Based Amplification (NASBA), Rolling Circle Amplification (RCA), Ligase Chain Reaction (LCR), Signal Mediated Amplification of RNA Technology (SMART), Strand Displacement Amplification (SDA), Loop Mediated Isothermal Amplification (LAMP), Isothermal Multiple Displacement Amplification (IMDA), Helicase-Dependent Amplification (HDA), Single Primer Isothermal Amplification (SIPA), circular Helicase Dependent Amplification (cHDA), and Next Generation Sequencing (NGS).

Optionally, polymerase chain reaction (PCR) is selected from real-time PCR and quantitative real-time PCR.

Optionally, the polymerase chain reaction (PCR), optionally the real-time PCR, optionally the quantitative real-time PCR, further comprises pyrosequencing.

Optionally, the at least one primer has a final concentration of about 0.05-0.5 µM, optionally 0.05 µM, optionally 0.5 µM.

Optionally the polymerase chain reaction, or real-time PCR, or quantitative real-time PCR, cycling parameters comprise about 10 minutes incubation at about 95°C, optionally or additionally 50 cycles at about 95°C for about 10 seconds and about 63°C for about 30 seconds, optionally or additionally followed by a cooling step at about 40°C for about 10 seconds.

Optionally, the detecting step (ai) comprises: contacting the sample with a probe capable of hybridizing to at least part of the nucleic acid sequence of the *ssrA* gene, optionally at least part of the nucleic acid sequence defined by SEQ ID NO. 5, under conditions suitable for hybridisation.

Optionally, the detecting step (ai) comprises: contacting the sample with a probe having a nucleic acid sequence defined by SEQ ID NO. 12 or the reverse complement thereof, under conditions suitable for hybridisation.

Optionally, the at least one probe comprises at least one marker, optionally at least one dye, optionally at least one fluorophore, optionally fluorescein.

Optionally, or additionally, the at least one probe comprises at least one fluorescence quenching dye.

Optionally, the at least one probe has a final concentration of about 0.02-0.8 µM, optionally 0.02 µM, optionally 0.08 µM, optionally 0.2 µM, optionally 0.8 µM.

Optionally, the method is a multiplex method.

Optionally, the method is a multiplex polymerase chain reaction method.

This invention provides a multiplex *in vitro* nucleic acid amplification method for identifying a serogroup of *Legionella pneumophila* present in a sample, wherein the method comprises detecting the presence of a plurality of nucleic acid molecule targets in the sample in one reaction, wherein at least one of the nucleic acid molecule targets is present in the genome of one or more, but not all, of the serogroups of *Legionella pneumophila.*

Previous methods for the detection of *Legionella pneumophila* have not been capable of specifically identifying *Legionella pneumophila* serogroup 1 in a manner that is practically useful. This means that diagnosis and treatment provision is not tailored to the specific serogroup present unless extensive experimentation is carried out. This requires significant time and effort that is incompatible with rapid and effective diagnosis and treatment. This invention, for the first time, provides a method that is able to identify members of *Legionella pneumophila* serogroup 1 in a rapid and easily-interpretable manner. The inventors have surprisingly found that there is sufficient variation between serogroups yet sufficient conservation between isolates of the same serogroup to specifically identify *Legionella pneumophila* serogroup 1 in a single reaction multiplex nucleic acid amplification assay. By identifying and characterising a series of sequences that are either shared or not shared between the different members of the Legionella, the present invention thus allows the use of multiplex nucleic acid amplification methods to specifically detect *Legionella pneumophila* serogroup 1.

The serogroups that make up *Legionella pneumophila* are closely related but differ significantly in their pathology and susceptibility to certain treatments. Therefore, although it is important to be able to distinguish between the different serogroups, it has previously not been possible to do this in any straightforward way that is amenable to use in rapid diagnosis. The different members of *Legionella pneumophila* share a significant proportion of their genetic material. The present invention has successfully identified sufficient differences between the genomes of the members of *Legionella pneumophila* to be able to distinguish between them in multiplex *in vitro* nucleic acid amplification assays. In contrast to the methods of the prior art, the methods of the present invention allow a single multiplex reaction to be performed that gives clear signals to identify which serogroup is present in the tested sample. It is not necessary to run gels or to undertake complex interpretation of the results.

The present inventors have surprisingly found that it is possible to identify serogroup 1 of *Legionella pneumophila,* despite the high sequence homology that exists between the serogroups of *Legionella pneumophila.* In particular, the present invention provides a multiplex *in vitro* nucleic acid amplification assay that is capable of identifying serogroups of *Legionella pneumophila,* such as *Legionella pneumophila* serogroup 1 in a single reaction. The inventors have also discovered that the presence of at least one organism selected from the other serogroups in the *Legionella pneumophila* species, including any of serogroups 1-16, can also be identified in the same single reaction. Furthermore, the inventors have devised a method for detecting the presence of other members of the Legionella genus, including *Legionella anisa, L. birminghamensis, L. birminghamensis, L. bozemanii, L. bozemanii, L. cincinnatiensis, L. dumoffii, L. dumoffii, L. erythra, L. feeleii-1, L. feelii-2, L. gormanii, L. gormanii, L. hackeliae-1, L. hackeliae-2, L. jordanis, L. jordanis, L. lansingensis, L. longbeachae-1, L. maceachemii, L. miodadei, L. oakridgensis, L. oakridgensis, L. parisiensis, L. wadworthii, L. cherrii, L. jamestowniensis, L. londoniensis, L. taurinsis, L. moravica, L. adelaidensis, L. donaldsonii, L. gratiana, L. gresilensis, L. fairfieldensis, L. israelensis, L. fallonii, L. brunensis, L. busanensis, L. quinlivanii,* and *L. rubrilicens.*

The present invention contemplates the use of any appropriate method for amplification of target regions. The method of amplification will in general be PCR. Real-time PCR (RT-PCR) is one example of this. In certain embodiments RT-PCR using fluorescent reporter probes is used. The use of the reporter probes significantly increases specificity, and enables quantification even in the presence of non-specific DNA amplification. Fluorescent probes are particularly suited to multiplex assays, as specific probes with different-coloured labels can be used. RT-PCR relies on a DNA-based probe with a fluorescent reporter at one end and a quencher of fluorescence at the opposite end of the probe. The close proximity of the reporter to the quencher prevents detection of its fluorescence; breakdown of the probe by the 5' to 3' exonuclease activity of the DNA polymerase (for example Taq polymerase) breaks the reporter-quencher proximity, and results in emission of fluorescence. An increase in the product targeted by the reporter probe at each PCR cycle therefore causes a proportional increase in fluorescence due to the breakdown of the probe and release of the reporter.

Further, the methods of the invention may therefore be configured to allow quantitation, i.e. the method comprises determining the amount of a member of a group of bacterial organisms in a sample. This may be achieved e.g. by selecting appropriate parameters for amplification (e.g. the number of cycles of amplification).

Other appropriate methods of amplification are referred to below.

Also contemplated for use in the present invention is Nucleic Acid Sequence Based Amplification (NASBA). Nucleic acid sequence-based amplification (NASBA) is an isothermal amplification technique which uses three enzymes (RNase H, AMV reverse transcriptase and T7 RNA polymerase) working in concert at a low isothermal temperature (generally 41 °C). The product of a NASBA reaction is mainly single-stranded RNA, which can be detected by gel electrophoresis, enzyme-linked gel assay (ELGA) or electrochemiluminescent detection (ECL). Alternatively, NASBA products can be detected in real time using molecular beacons included in the reaction. In microbial diagnostics, NASBA has been successfully combined with electrochemiluminescent (ECL), ELISA labelled dendrimer and molecular beacon-based methods to detect and identify viral and bacterial pathogens (Scheler *et al*., 2009).

Also contemplated for use in the present invention is Rolling Circle Amplification (RCA). RCA describes a process of unidirectional nucleic acid replication that can rapidly synthesize multiple copies of circular molecules of DNA or RNA. RCA is a technology that is adaptable to an on-chip signal amplification format. RCA is well suited to solid phase formats such as microarrays for generating localized signals at specific microarray locations. This distinctive property of RCA should allow many assays to be performed simultaneously (multiplexing) without interference.

Also contemplated for use in the present invention is the Ligase Chain Reaction (LCR). LCR uses two complementary pairs of probes which, when the correct template is available, hybridize next to each other and then are ligated together. These ligated probes plus the original template serve as the template for the next cycle of hybridization and ligation. As subsequent cycles are performed, the amplification proceeds exponentially. A commercially available kit using this technology is the LCx M. *tuberculosis* complex specific kit available from Abbott Diagnostics.

Further isothermal amplification technologies that are contemplated for use with the present invention are provided in and include signal mediated amplification of RNA technology (SMART), strand displacement amplification (SDA), loop mediated isothermal amplification (LAMP), isothermal multiple displacement amplification (IMDA), helicase-dependent amplification (HDA), single primer isothermal amplification (SIPA) and circular helicase dependent amplification (cHDA). As exemplified by SMART, the amplification method used with the invention may comprise signal amplification rather than target amplification.

Also contemplated for use in the present invention is Next Generation Sequencing (NGS). Next generation sequencing is a relatively new field of sequencing which allows for the rapid high throughput process. NGS has the capacity to generate gigabases of nucleotide sequence, depending on the instrument used, in a single run. A recently described assay combines the use of real-time PCR in combination with pyrosequencing which allows for the rapid detection of MTC DNA in addition to sequencing of an 81-bp core region of the rpoB gene associated with rifampin resistance (Halse *et al*., 2010).

Optionally the sample substantially comprises a water sample.

Optionally the sample substantially comprises an environmental water sample.

Optionally the sample substantially comprises a sample from a water distribution system.

Optionally the sample substantially comprises a sample from a plumbing system.

Optionally the sample substantially comprises a sample from a healthcare facility water system.

Also disclosed is a *Legionella* detection kit comprising at least one primer or probe capable of hybridizing to at least one nucleic acid sequence selected from: at least part of the nucleic acid sequence of the *Ipg0768* gene or an expression product thereof, at least part of the nucleic acid sequence of the *smpB* gene or an expression product thereof, at least part of the nucleic acid sequence of the *ssrA* gene or an expression product thereof; and instructions for use.

Optionally, the kit comprises at least one primer or probe capable of hybridizing to at least one nucleic acid sequence defined by any of SEQ ID NOs 1, 5, 6, and 21-48, or any expression product each thereof; and instructions for use.

Optionally, the kit comprises at least one primer or probe having the nucleic acid sequence defined by any of SEQ ID NOs 2, 3, 4, 7, 8, 9 and 13; and instructions for use.

Also disclosed is a *Legionella* detection assay comprising a solid support and at least one primer or probe capable of hybridizing to at least one nucleic acid sequence selected from: at least part of the nucleic acid sequence of the *Ipg0768* gene or an expression product thereof, at least part of the nucleic acid sequence of the *smpB* gene or an expression product thereof, at least part of the nucleic acid sequence of the *ssrA* gene or an expression product thereof.

Optionally, the assay comprises a solid support and at least one primer or probe capable of hybridizing to at least one nucleic acid sequence defined by any of SEQ ID NOs 1, 5, 6, and 21-48, or any expression product each thereof.

Optionally, the assay comprises a solid support and at least one primer or probe having the nucleic acid sequence defined by any of SEQ ID NOs 2, 3, 4, 7, 8, 9 and 13.

Optionally, the assay is a microarray comprising at least one primer or probe capable of hybridizing to at least one nucleic acid sequence selected from: at least part of the nucleic acid sequence of the *Ipg0768* gene or an expression product thereof, the at least part of the nucleic acid sequence of the *smpB* gene or an expression product thereof, the at least part of the nucleic acid sequence of the *ssrA* gene or an expression product thereof.

Optionally, the microarray comprises at least one primer or probe capable of hybridizing to at least one nucleic acid sequence defined by any of SEQ ID NOs 1, 5, 6, and 21-48, or any expression product each thereof.

Optionally, the microarray discloses at least one primer or probe having the nucleic acid sequence defined by any of SEQ ID NOs 2, 3, 4, 7, 8, 9 and 13.

Also provided is a method for detecting Legionnaires' disease comprising a method according to the invention, wherein the presence of at least part of the nucleic acid sequence of SEQ ID NO. 1 or expression product thereof indicates the presence of Legionnaires' disease.

Optionally, the method for detecting Legionnaires' disease comprises the steps of:
(a) detecting the presence or quantity of at least part of the nucleic acid sequence of SEQ ID NO. 1 or an expression product thereof; and
(b) correlating the presence or quantity of the at least part of the nucleic acid sequence of SEQ ID NO. 1 or expression product thereof, to the presence or quantity of Legionnaires' disease.

### Optionally, the method is an in vitro method.

Optionally, any probe used in the method for detecting Legionnaires' disease may further comprise at least one dye, optionally at least one fluorophore, optionally hexachlorofluorescein, optionally rhodamine, optionally fluorescein.

Optionally or additionally, the probe may further comprise at least one fluorescence quenching dye.

The term "expression product" refers to any molecule that may be obtained from a nucleic acid sequence by transcription, translation, post-translational modification, or reverse transcription; including but not limited to RNA, amino acid polypeptide, protein, and complementary DNA.

The term "sg1" is synonymous with the term "serogroup 1".

The term "terminal quencher" refers to a fluorescence quenching dye, which can be located at or adjacent at least one end, optionally located at or adjacent at least one terminal end, of a probe; for example, located at or adjacent at least the 3' end of an oligonucleotide probe.

The term "internal quencher" refers to a fluorescence quenching dye, which can be located between the ends, optionally located between the terminal ends of a probe; for example, located between the 3' and 5' ends of an oligonucleotide probe.

### Brief description of the drawings

Embodiments of the invention will now be described with reference to the accompanying drawings in which:
Figure 1(a) illustrates quantitative real-time PCR amplification curves, showing inclusivity testing of the *L. pneumophila* serogroup 1 assay targeting a region of the *Ipg0768* gene for detection of *L. pneumophila* serogroup 1 strains;
Figure 1(b) illustrates quantitative real-time PCR amplification curves, showing exclusivity testing of the *L. pneumophila* serogroup 1 assay targeting a region of the *Ipg0768* gene. No cross reactions were observed with strains closely related to *L. pneumophila* serogroup 1*;*
Figure 2(a) illustrates quantitative real-time PCR amplification curves, showing inclusivity testing of the *L. pneumophila* species assay targeting a region of the *smpB* gene for detection of *L. pneumophila* strains;
figure 2(b) illustrates quantitative real-time PCR amplification curves, showing exclusivity testing of the *L. pneumophila* species assay targeting a region of the *smpB* gene. No cross reactions were observed with non-*legionella pneumophila* species and strains tested for.
Figure 3(a) illustrates quantitative real-time PCR amplification curves, showing inclusivity testing of species of the *Legionella* genus assay targeting a region of the *ssrA* gene for detection of *Legionella* strains, using the multiplex assay;
Figure 3(b) illustrates quantitative real-time PCR amplification curves, showing the specificity of the *L*. *pneumophila* serogroup 1 assay targeting a region of the *Ipg0768* gene for detection of *L. pneumophila* serogroup 1 isolates;
Figure 3(c) illustrates quantitative real-time PCR amplification curves, showing the specificity of the *L*. *pneumophila* species assay targeting a region of the *smpB* gene for detection of all *L. pneumophila* strains and non-detection of other *Legionella* species; and
Figure 3(d) illustrates quantitative real-time PCR amplification curves, showing detection of the Internal Amplification Control (IAC) assay in each sample tested for.
Figure 4(A) illustrates quantitative real-time PCR amplification curves, showing inclusivity testing of species of the *Legionella* genus assay targeting a region of the *ssrA* gene for detection of *Legionella* strains, using the multiplex assay;
Figure 4(B) illustrates quantitative real-time PCR amplification curves, showing the specificity of the *L. pneumophila* serogroup 1 assay targeting a region of the *Ipg0768* gene for detection of *L. pneumophila* serogroup 1 isolates;
Figure 4(C) illustrates quantitative real-time PCR amplification curves, showing the specificity of the *L. pneumophila* species assay targeting a region of the *smpB* gene for detection of all *L. pneumophila* strains and non-detection of other *Legionella* species; and
Figure 4(D) illustrates quantitative real-time PCR amplification curves, showing detection of the Internal Amplification Control (IAC) assay in each sample tested for.

### Examples

Embodiments of the present invention will now be described with reference to the following nonlimiting examples:

### Example 1 - real-time PCR assay for the detection of Legionella pneumophila serogroup 1

### In silico target identification

Publicly available whole genome sequence information was retrieved from the National Center for Biotechnology Information *website (www.ncbi.nlm.nih.gov*/*) for Legionella pneumophila serogroup 1 and Legionella pneumophila serogroup 6 and serogroup 12. Regions potentially unique to L. pneumophila serogroup 1 were identified using WEBACT (www.webact.org*/*). This resulted in a number of regions of difference in L. pneumophila serogroup 1. Each identified putative target nucleotide sequence was BLAST analysed (blast.ncbi.nlm.nih.gov*/*Blast.cgi) to determine if they were present in all L. pneumophila serogroup 1 and absent in all other L. pneumophila serogroups or closely related Legionella species. One region was present in all L. pneumophila serogroup 1 analysed with very low or no sequence similarity to other L. pneumophila serogroups or closely related Legionella species. This region was the Ipg0768* gene (SEQ ID NOs 1 and 21-48) which codes for spore coat polysaccharide biosynthesis protein E, NeuB. Closely related nucleotide sequences *were aligned using ClustalW* *(*www.ebi.ac.uk/Tools/msa/clustalw2/*)*.

In this example, the following exemplary real-time PCR assay primers and probe for the *Ipg0768* gene (SEQ ID NOs 1 and 21-48) were used: spore coat polysaccharide biosynthesis protein E forward primer (SEQ ID NO. 2), spore coat polysaccharide biosynthesis protein E reverse primer (SEQ ID NO. 3), spore coat polysaccharide biosynthesis protein E probe (SEQ ID NO. 4). The spore coat probe (SEQ ID NO. 4) comprised at least one marker, optionally at least one dye, optionally at least one fluorophore, optionally hexachlorofluorescein (5' HEX, Integrated DNA Technologies). Optionally, the spore coat polysaccharide biosynthesis protein E probe (SEQ ID NO. 4) comprises at least one fluorescence quenching dye, optionally an internal quencher, such as ZEN^{™} quencher, Integrated DNA Technologies; and/or a terminal quencher, such as 3' Iowa Black^{®} FQ, Integrated DNA Technologies; or Black Hole Quencher 1^{®}, LGC Biosearch.

### Bacterial strains, culture media, and growth conditions

A panel of 26 *Legionella pneumophila* strains and 41 other *Legionella* species were used in this study (Table 1(a) and 1(b)). These species and strains were purchased from the German Collection of Microorganisms and Cell Cultures (Deutsche Sammlung von Mikroorganismen und Zellkulturen GmbH, DSMZ. Braunschweig, Germany), the National Collection of Type Cultures (NCTC, Public Health England, Salisbury, United Kingdom), American Type Culture Collection (ATCC, provided by LGC standards, Middlesex United Kingdom). All Legionella species and strains were cultured on buffered charcoal yeast extract (BCYE) media at 37°C 18-24 hours or until sufficient growth was observed.

**Table 1(a) Inclusivity panel**

| **Strain** | **Culture collection number** |
|---|---|
| *Legionella pneumophila subsp. pneumophila-1* | DSM 7513 |
| *Legionella pneumophila subsp. pneumophila-1* | NCTC 11191 |
| *Legionella pneumophila subsp. pneumophila-1* | NCTC 11231 |
| *Legionella pneumophila subsp. pneumophila-1* | NCTC 11286 |
| *Legionella pneumophila subsp. pneumophila-1* | NCTC 11378 |
| *Legionella pneumophila subsp. pneumophila-1* | NCTC 11404 |
| *Legionella pneumophila subsp. pneumophila-1* | DSM 25019 |
| *Legionella pneumophila subsp. pneumophila-1* | DSM 25021 |
| *Legionella pneumophila subsp. pneumophila-1* | DSM 25199 |
| *Legionella pneumophila subsp. pneumophila-1* | DSM 25200 |
| *Legionella pneumophila subsp. pneumophila-1* | DSM 25213 |
| *Legionella pneumophila subsp. pneumophila-1* | DSM 37564 |

**Table 1(b) Exclusivity panel**

| **Species** | **Culture collection number** |
|---|---|
| *Legionella pneumophila subsp. fraseri-4* | DSM 7514 |
| *Legionella pneumophila subsp. pasculei-5* | DSM 7515 |
| *Legionella pneumophila subsp. pneumophila-1* | NCTC 11231 |
| *Legionella pneumophila subsp. pneumophila-3* | NCTC 11232 |
| *Legionella pneumophila subsp. pneumophila-6* | NCTC 11287 |
| *Legionella pneumophila subsp. pneumophila-5* | NCTC 11417 |
| *Legionella pneumophila subsp. pneumophila-7* | NCTC 11984 |
| *Legionella pneumophila subsp. pneumophila-8* | NCTC 11985 |
| *Legionella pneumophila subsp. pneumophila-9* | NCTC 11986 |
| *Legionella pneumophila subsp. pneumophila-10* | NCTC 12000 |
| *Legionella pneumophila subsp. pneumophila-14* | NCTC 12174 |
| *Legionella pneumophila subsp. pneumophila-11* | NCTC 12179 |
| *Legionella pneumophila subsp. pneumophila-12* | NCTC 12180 |
| *Legionella pneumophila subsp. pneumophila-13* | NCTC 12181 |
| *Legionella anisa* | DSM 17627 |
| *Legionella birminghamensis* | DSM 19232 |
| *Legionella. birminghamensis* | ATCC 700709 |
| *Legionella bozemanii* | NCTC 11975 |
| *Legionella bozemanii* | DSM 16523 |
| *Legionella cincinnatiensis* | DSM 19233 |
| *Legionella dumoffii* | DSM 17625 |
| *Legionella dumoffii* | ATCC 33343 |
| *Legionella erythra* | DSM 17644 |
| *Legionella feeleii-1* | DSM 17645 |
| *Legionella feelii-2* | NCTC 11978 |
| *Legionella gormanii* | DSM 16641 |
| *Legionella gormanii* | ATCC 43769 |
| *Legionella hackeliae-1* | DSM 19214 |
| *Legionella hackeliae-2* | NCTC 11980 |
| *Legionella jordanis* | DSM 19212 |
| *Legionella jordanis* | ATCC 700762 |
| *Legionella lansingensis* | DSM 19556 |
| *Legionella longbeachae-1* | DSM 10572 |
| *Legionella maceachemii* | DSM 16642 |
| *Legionella miodadei* | NCTC 11403 |
| *Legionella oakridgensis* | NCTC 11531 |
| *Legionella oakridgensis* | ATCC 700515 |
| *Legionella parisiensis* | DSM 19216 |
| *Legionella wadworthii* | NCTC 11532 |
| *Legionella cherrii* | DSM 19213 |
| *Legionella jamestowniensis* | DSM 19215 |
| *Legionella londoniensis* | DSM 21234 |
| *Legionella taurinsis* | CCUG 44901 |
| *Legionella moravica* | DSM 19234 |
| *Legionella adelaidensis* | DSM 19888 |
| *Legionella donaldsonii* | ATCC BAA-693 |
| *Legionella gratiana* | DSM 21233 |
| *Legionella gresilensis* | DSM 21218 |
| *Legionella fairfieldensis* | NCTC 12488 |
| *Legionella israelensis* | DSM 19235 |
| *Legionella fallonii* | CCUG 43887 |
| *Legionella brunensis* | DSM 19236 |
| *Legionella busanensis* | ATCC BAA-518 |
| *Legionella quinlivanii* | NCTC 12434 |
| *Legionella rubrilicens* | DSM 11884 |

### DNA isolation and quantification

Genomic DNA from Legionella cultures were isolated using a modified procedure combining mechanical lysis (IDI lysis kit; Becton Dickenson, Canada) and purification using a DNA purification kit (quick gDNA kit; Zymo Research, Irvine, CA, USA). Briefly, a loop of culture was resuspended in 250 ul lysis buffer (IDI lysis buffer). The suspension was transferred to a cell lysis tube (GeneOhm) and bead beaten (Mini-Bead-Beater-16; Stratech, United Kingdom) for 3 minutes. After bead beating, 200 µl was transferred to a nucleic acid purification spin column (Zymo-Spin^{™} Column) in a collection tube and steps 2 to 5 of the procedure for purification of total DNA from cell suspensions were followed, according to the manufacturer's instructions.

### Assay design

All oligonucleotide primers and probes used in this study were designed in accordance with published guidelines.

### Real-time PCR

This *Legionella pneumophila* serogroup 1 specific assay was then tested in a real-time PCR format against a panel of well characterised *Legionella* species to determine assay specificity (Inclusivity and Exclusivity testing) and suitability of the target nucleotide sequence as a diagnostics marker.

Real-time PCR was performed on a real-time PCR amplification device (LightCycler^{®} 480 Instrument, Roche Diagnostics) using a real-time PCR probes kit (LightCycler^{®} 480 Probes Master kit, Roche Diagnostics). The reaction mixture comprised: master mix (LightCycler^{®} 480 Probes Master; 6.4 mM MgCl₂) forward and reverse primer (0.5 µM final conc.), carboxyfluorescein (FAM) labelled probe (0.2 µM final conc.), template DNA (5 µl) and nuclease free dH₂O to a final volume of 20 µl. The cycling parameters consisted of 10 minutes incubation at 95°C to activate the polymerase (Taq), 50 cycles of 95°C for 10 seconds and 60°C for 30 s, followed by a cooling step at 40°C for 10 s. The temperature transition rate (ramp rate on the LightCycler^{®} 480 Instrument) was 4.4°C/s while heating and 2.2°C/s while cooling.

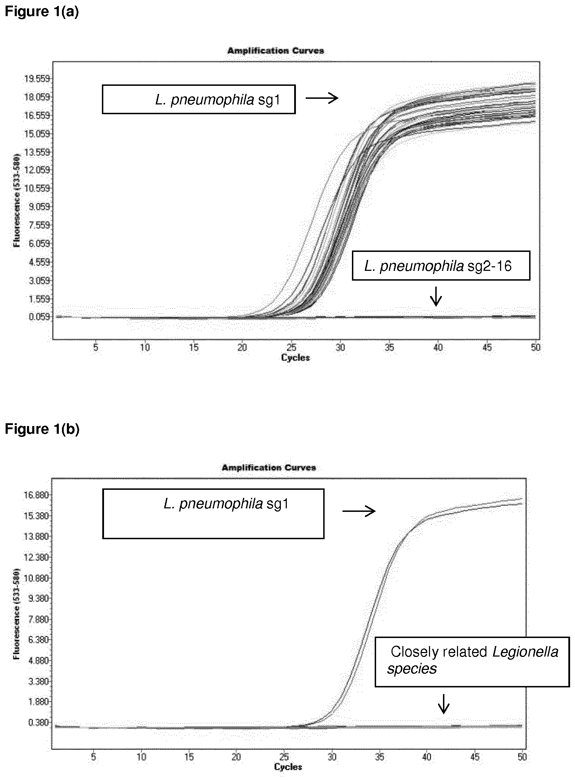

As demonstrated (Figure 1(a)), the assay targeting a region of the *Ipg0768* gene detected all 12 *Legionella pneumophila* serogroup 1 strains tested for (Table 1(a)); but the assay did not detect *Legionella pneumophila* serogroup 2-16 strains, or the no template control. The remaining *Legionella* species tested for (Table 1(b)) were not detected by the assay targeting a region of the *Ipg0768* gene (Figure 1(b)).

### Example 2 -assay for the detection of Legionella pneumophila

### In silico analysis

Publicly available *smpB* gene nucleotide sequence information was retrieved from the National Center for Biotechnology Information website (www.ncbi.nlm.nih.gov/) for *Legionella pneumophila* and aligned using ClustalW (www.ebi.ac.uk/Tools/msa/clustalw2/). Partial gene sequence was also generated for a number of culture collection strains using sequencing primers and then aligned. All publicly available and generated nucleotide sequences for *Legionella pneumophila* sequences were subsequently aligned which demonstrated sufficient nucleotide similarity to design oligonucleotide primers and probes.

In this example, the following exemplary real-time PCR assay primers and probe for the *smpB* gene nucleotide sequence (SEQ ID NO. 6) were used: smpB forward primer (SEQ ID NO. 7), smpB reverse primer (SEQ ID NO. 8), smpB probe (SEQ ID NO. 9). Optionally an alternative smpB reverse primer (SEQ ID No. 13) could be used. The smpB probe (SEQ ID NO. 9) comprised at least one marker, optionally at least one dye, optionally at least one fluorophore, optionally rhodamine (5' ROX, Integrated DNA Technologies). Optionally, the smpB probe (SEQ ID NO. 9) comprised at least one fluorescence quenching dye, optionally a terminal quencher (3' Iowa Black^{®} RQ, Integrated DNA Technologies; or Black Hole Quencher 2^{®}, LGC Biosearch).

### Nucleotide sequencing

To generate a partial *smpB* gene nucleotide sequence for *L. pneumophila,* conventional PCR was performed using sequencing primers on a thermal cycler (iCycler iQ thermal cycler; Bio-Rad Laboratories Inc., California, USA). All reactions were carried out in a final volume of 50 µl, containing 5 µl 10x buffer (15 mM MgCl₂), 1 µl forward and reverse primers (0.2 µM final conc.), 2 µl DNA polymerase (Taq; 1 U/µl, Roche Diagnostics, Basel, Switzerland), 1 µl dNTP mix (10 mM deoxynucleoside triphosphate set; Roche Diagnostics), 5 µl of template DNA and 35 µl nuclease free water (Applied Biosystems/Ambion, Texas, USA). The cycling parameters consisted of initial denaturation at 95°C for 5 minutes, followed by 35 cycles of denaturation at 95°C (1 minutes), annealing at 50°C (1 minutes), and extension at 72°C (1 minutes), with a final elongation step at 72°C for 10 minutes.

PCR products were visualised on a 1.5% agarose gel and subsequently purified using a PCR product purification kit (High Pure PCR Product Purification Kit, Roche Diagnostics). Purified PCR products were sent for sequencing externally (Sequiserve, Vaterstetten, Germany).

### DNA isolation and quantification

Genomic DNA from Legionella cultures were isolated using a modified procedure combining mechanical lysis (IDI lysis kit; Becton Dickenson, Canada) and purification using a DNA purification kit (quick gDNA kit, Zymo Research, Irvine, CA, USA). Briefly, a loop of culture was resuspended in 250 ul lysis buffer (IDI lysis buffer). The suspension was transferred to a cell lysis tube (GeneOhm) and bead beaten (Mini-Bead-Beater-16; Stratech, United Kingdom) for 3 minutes. After bead beating, 200 µl was transferred to a nucleic acid purification spin column (Zymo-Spin^{™} Column) in a collection tube and steps 2 to 5 of the procedure for purification of total DNA from cell suspensions were followed, according to the manufacturer's instructions.

### Bacterial strains, culture media, and growth conditions

A panel of 26 Legionella pneumophila strains and 41 other Legionella species and 14 other bacteria were used in this study (Table 2(a) and Table 2(b)). These species and strains were purchased from the German Collection of Microorganisms and Cell Cultures (Deutsche Sammlung von Mikroorganismen und Zellkulturen GmbH, DSMZ. Braunschweig, Germany), the National Collection of Type Cultures (NCTC, Public Health England, Salisbury, United Kingdom), American Type Culture Collection (ATCC, provided by LGC standards, Middlesex United Kingdom) and Culture Collection, University of Göteborg, (CCUG, Sweden). All *Legionella* species and strains were cultured on BCYE media at 37°C 18-24 hours or until sufficient growth was observed.

**Table 2(a): Legionella pneumophila strains used in this study**

| **Strain** | **Culture collection** |
|---|---|
| *Legionella pneumophila* subsp. pneumophila-1 | DSM 7513 |
| *Legionella pneumophila* subsp. fraseri-4 | DSM 7514 |
| *Legionella pneumophila* subsp. pasculei-5 | DSM 7515 |
| *Legionella pneumophila* subsp. pneumophila-1 | NCTC 11191 |
| *Legionella pneumophila* subsp. pneumophila-2 | NCTC 11230 |
| *Legionella pneumophila* subsp. pneumophila-1 | NCTC 11231 |
| *Legionella pneumophila* subsp. pneumophila-3 | NCTC 11232 |
| *Legionella pneumophila* subsp. pneumophila-1 | NCTC 11286 |
| *Legionella pneumophila* subsp. pneumophila-6 | NCTC 11287 |
| *Legionella pneumophila* subsp. pneumophila-1 | NCTC 11378 |
| *Legionella pneumophila* subsp. pneumophila-1 | NCTC 11404 |
| *Legionella pneumophila* subsp. pneumophila-5 | NCTC 11417 |
| *Legionella pneumophila* subsp. pneumophila-7 | NCTC 11984 |
| *Legionella pneumophila* subsp. pneumophila-8 | NCTC 11985 |
| *Legionella pneumophila* subsp. pneumophila-9 | NCTC 11986 |
| *Legionella pneumophila* subsp. pneumophila-10 | NCTC 12000 |
| *Legionella pneumophila* subsp. pneumophila-14 | NCTC 12174 |
| *Legionella pneumophila* subsp. pneumophila-11 | NCTC 12179 |
| *Legionella pneumophila* subsp. pneumophila-12 | NCTC 12180 |
| *Legionella pneumophila* subsp. pneumophila-13 | NCTC 12181 |
| *Legionella pneumophila* subsp. pneumophila-1 | DSM 25019 |
| *Legionella pneumophila* subsp. pneumophila-1 | DSM 25071 |
| *Legionella pneumophila* subsp. pneumophila-1 | DSM 25199 |
| *Legionella pneumophila* subsp. pneumophila-1 | DSM 25200 |
| *Legionella pneumophila* subsp. pneumophila-1 | DSM 25213 |
| *Legionella pneumophila* subsp. pneumophila-1 | DSM 27564 |

| | |
|---|---|
| *DSM = The German Collection of Microorganisms; *NCTC = National Collection of Type Cultures | |

**Table 2(b): Other Legionella species tested in this study**

| **Species** | **culture collection number** |
|---|---|
| *Legionella anisa* | DSM 17627 |
| *Legionella birminghamensis* | DSM 19232 |
| *Legionella. birminghamensis* | ATCC 700709 |
| *Legionella bozemanii* | NCTC 11975 |
| *Legionella bozemanii* | DSM 16523 |
| *Legionella cincinnatiensis* | DSM 19233 |
| *Legionella dumoffii* | DSM 17625 |
| *Legionella dumoffii* | ATCC 33343 |
| *Legionella erythra* | DSM 17644 |
| *Legionella feeleii-1* | DSM 17645 |
| *Legionella feelii-2* | NCTC 11978 |
| *Legionella gormanii* | DSM 16641 |
| *Legionella gormanii* | ATCC 43769 |
| *Legionella hackeliae-1* | DSM 19214 |
| *Legionella hackeliae-2* | NCTC 11980 |
| *Legionella jordanis* | DSM 19212 |
| *Legionella jordanis* | ATCC 700762 |
| *Legionella lansingensis* | DSM 19556 |
| *Legionella longbeachae-1* | DSM 10572 |
| *Legionella maceachemii* | DSM 16642 |
| *Legionella miodadei* | NCTC 11403 |
| *Legionella oakridgensis* | NCTC 11531 |
| *Legionella oakridgensis* | ATCC 700515 |
| *Legionella parisiensis* | DSM 19216 |
| *Legionella wadworthii* | NCTC 11532 |
| *Legionella cherrii* | DSM 19213 |
| *Legionella jamestowniensis* | DSM 19215 |
| *Legionella londoniensis* | DSM 21234 |
| *Legionella taurinsis* | CCUG 44901 |
| *Legionella moravica* | DSM 19234 |
| *Legionella adelaidensis* | DSM 19888 |
| *Legionella donaldsonii* | ATCC BAA-693 |
| *Legionella gratiana* | DSM 21233 |
| *Legionella gresilensis* | DSM 21218 |
| *Legionella fairfieldensis* | NCTC 12488 |
| *Legionella israelensis* | DSM 19235 |
| *Legionella fallonii* | CCUG 43887 |
| *Legionella brunensis* | DSM 19236 |
| *Legionella busanensis* | ATCC BAA-518 |
| *Legionella quinlivanii* | NCTC 12434 |
| *Legionella rubrilicens* | DSM 11884 |
| *Burkholderia cepacia* | DSM 7522 |
| *Ralstonia pickettii* | DSM 6297 |
| *Serratia marcescens* | DSM 30121 |
| *Cupriavidus pauculus* | DSM 17313 |
| *Stenotrophomonas maltophilia* | DSM 21874 |
| *Pseudomonas maltophila* | DSM 50071 |
| *Klebsiella pneumoniae* | DSM 12059 |
| *Proteus mirabilis* | DSM 4479 |
| *Enterobacter aerogenes* | DSM 2969 |
| *Acinetobacter baumannii* | LMG 984 |
| *Staphylococcus aureus* | DSM 11822 |
| *Bacillus cereus* | DSM 30584 |
| *Clostridium difficile* | DSM 1296 |
| *Sphingomonas paucimobilis* | DSM 1098 |

| | |
|---|---|
| *DSM = The German Collection of Microorganisms; *ATCC = American Type Culture Collection; *NCTC = National Collection of Type Cultures; *CCUG=Culture Collection, University of Göteborg | |

### Real-time PCR

The *Legionella pneumophila* specific assay was then tested in a real-time PCR format against a panel of well characterised *Legionella* species to determine assay specificity (Inclusivity and Exclusivity testing) and suitability of the *smpB* gene nucleotide sequence as a diagnostics marker.

Real-time PCR was performed on a real-time PCR amplification device (LightCycler^{®} 480 Instrument, Roche Diagnostics) using a real-time PCR probes kit (LightCycler^{®} 480 Probes Master kit, Roche Diagnostics). The reaction mixture comprised: master mixture (LightCycler^{®} 480 Probes Master) in 6.4 mM MgCl₂, forward and reverse primer (0.5 µM final conc.), carboxyfluorescein (FAM) labelled probe (0.2 µM final conc.), template DNA (5 µl) and nuclease free dH₂O to a final volume of 20 µl. The cycling parameters consisted of 10 minutes incubation at 95°C to activate the polymerase (Taq), 50 cycles of 95°C for 10 seconds and 60°C for 30 s, followed by a cooling step at 40°C for 10 s. The temperature transition rate, (ramp rate on the LightCycler^{®} 480 Instrument) was 4.4°C/s while heating and 2.2°C/s while cooling.

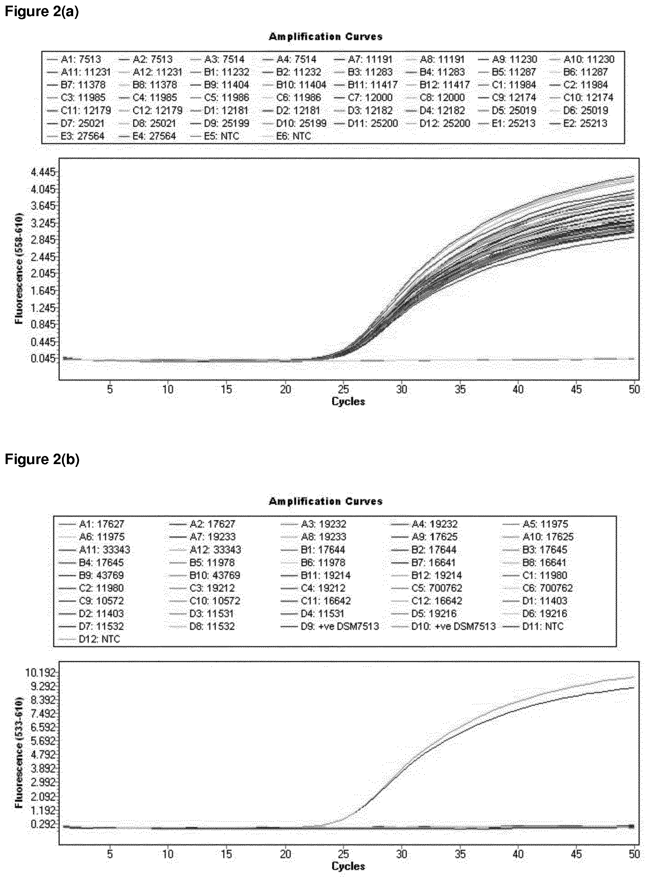

As demonstrated in Figure 2(a), all 26 *Legionella pneumophila* strains tested for (Table 2(a)) were detected by the *L. pneumophila* species assay targeting a region of the *smpB* gene and the no-template control was not detected by the assay. As shown in Figure 2(b), the *L. pneumophila* species assay targeting a region of the *smpB* gene detected the one *L. pneumophila* isolate tested for and did not detect the 41 other *Legionella* species and other bacteria that were tested for (Table 2(b)).

### Example 3: An internally controlled multiplex real-time PCR for the detection and identification of Legionella species, Legionella pneumophila (serogroup 1-16) and Legionella pneumophila serogroup 1

### Diagnostic target identification

A number of housekeeping genes which are highly conserved throughout the *Legionella* genus were evaluated to identify, *in silico,* a potential diagnostic target which could be used to detect all members of this genus and for the detection of *Legionella pneumophila* strains. The optimal diagnostic target chosen in this study for detection of the *Legionella* genus was the *ssrA* gene. The *ssrA* gene codes for tmRNA and has been identified in all bacterial species. The function of tmRNA in bacteria is to rescue stalled ribosomes and to clear the cell of incomplete polypeptides. *In silico* alignment of publically available nucleotide sequences indicated that while sufficient nucleotide sequence heterogeneity exists between different *Legionella* species, sufficient nucleotide sequence similarity existed to design genus oligonucleotide primers and probes.

In this example, the following exemplary real-time PCR assay primers and probe for the *ssrA* gene nucleotide sequence (SEQ ID NO. 5) were used: ssrA forward primer (SEQ ID NO. 10), ssrA reverse primer (SEQ ID NO. 11), ssrA probe (SEQ ID NO. 12). The ssrA probe (SEQ ID NO. 12) comprised at least one marker, optionally at least one dye, optionally at least one fluorophore, optionally fluorescein (5' 6-FAM, Integrated DNA Technologies). Optionally, the ssrA probe (SEQ ID NO. 12) comprised at least one fluorescence quenching dye, optionally an internal quencher (ZEN^{™} quencher, Integrated DNA Technologies), optionally a terminal quencher (3' Iowa Black^{®} FQ, Integrated DNA Technologies; or Black Hole Quencher 1^{®}, LGC Biosearch).

By *in silico* alignment, it was demonstrated that there is perfect, or near-perfect, conservation of the each of the following nucleotide sequences: the exemplary ssrA forward primer (SEQ ID NO. 10), the exemplary ssrA reverse primer (SEQ ID NO. 11), and the exemplary ssrA probe (SEQ ID NO. 12); among the ssrA gene nucleotide sequences of the following *Legionella* species:
*L. pneumophilaATCC43290, L. pneumophila_*ThunderBay, *L. pneumopnila_*HL06041035,
*L. pneumophila*_str. Philadelphia, *L. pneumophila*_Lens, *L. pneumophila_*Paris,
*L. pneumophila_Corby, L. pneumophila_*2300/99Alcoy, *L. pneumophila_*Lorraine, *L. longbeachaeD-*4968, *L. longbeachae*NSW150, *L. fallonii_*LLAP-10, *L. hackeliae_LHA, L. oakridgensis_*ATCC33761
*L. micdadei_*LMI, *L. sainthelensi_*ATCC35248, *L. wadsworthit*DSM21896, *L. cherrii_*DSM19213,
*L. moravica*DSM19234, *L. norrlandica, L. shakespearei*DSM23087, *L. drancourtii*LLAP12
*L. lansingensis_*DSM19556, *L. fairfieldensis_*ATCC_49588, *L. massiliensis_*PRJEB110, and
*L. geestiana*DSM21217*.*

The optimal diagnostic target chosen in this study for detection of the *Legionella pneumophila* was the *smpB* gene. The *smpB* gene encodes the RNA binding protein Small Protein B (SmpB) and has been identified in all bacterial species to date. It is considered an essential component of quality control in bacteria as it facilitates the binding of tmRNA to stalled ribosomes which in turn allows for the removal of incomplete polypeptides from the cell. *In silico* alignment of publically available nucleotide sequences indicated that sufficient nucleotide sequence similarity exists between different serogroups of *Legionella pneumophila* to allow for the design of species specific oligonucleotide primers and probes.

In this example, the following exemplary real-time PCR assay primers and probe for the *smpB* gene nucleotide sequence (SEQ ID NO. 6) were used: smpB forward primer (SEQ ID NO. 7), smpB reverse primer (SEQ ID NO. 8), smpB probe (SEQ ID NO. 9). Optionally an alternative smpB reverse primer (SEQ ID No. 13) could be used. The smpB probe (SEQ ID NO. 9) comprised at least one marker, optionally at least one dye, optionally at least one fluorophore, optionally rhodamine (5' ROX, Integrated DNA Technologies). Optionally, the smpB probe (SEQ ID NO. 9) comprised at least one fluorescence quenching dye, optionally a terminal quencher (3' Iowa Black^{®} RQ, Integrated DNA Technologies; or Black Hole Quencher 2^{®}, LGC Biosearch).

By *in silico* alignment, it was demonstrated that there is perfect, or near-perfect, conservation of the each of the following nucleotide sequences: the exemplary *smpB* forward sequencing primer (SEQ ID NO. 14), the exemplary *smpB* forward primer (SEQ ID NO. 7), the exemplary *smpB* reverse sequencing primer (SEQ ID NO. 15), the exemplary *smpB* reverse primer (SEQ ID NO. 8), and the exemplary *smpB* probe (SEQ ID NO. 9); among the *smpB* nucleotide sequences of the following
*Legionella* species: *L. pneumophila_*Paris, *L. pneumophila_Corby, L. pneumophila_*Pneu*,*
*L. pneumophila_*Lens*, L. pneumophila_*Alcoy, *L. pneumophila_2868, L. pneumophila_*3140,
*L. pneumophila_*3194, *L. pneumophila_*00189, *L. pneumophila_2829, L. pneumophila_*NCTC11986,
*L. pneumophila_*NCTC12000, *L. pneumophila_NCTC12179, L. pneumophila_*DSM7513,
*L. pneumophila*DSM7514, *L. pneumophila*DSM7515, *L. pneumophila_*NCTC11191,
*L. pneumophila_*NCTC11230, *L. pneumophila_NCTC11232, L. pneumophila_*NCTC11287,
*L. pneumophila_*NCTC11984, *L. pneumophila_*NCTC11985.

Based on the evaluation of housekeeping genes performed in this study, it was not possible to identify a *Legionella pneumophila* serogroup 1 specific target. As such whole genome comparisons were performed to identify a novel diagnostics target *in silico.* Publicly available whole genome sequence information was retrieved from the National Center for Biotechnology Information website (www.ncbi.nlm.nih.gov/) for *Legionella pneumophila* serogroup 1 and *Legionella pneumophila* serogroup 6 and serogroup 12. Regions potentially unique to *L. pneumophila* serogroup 1 were identified using WEBACT (www.webact.org/). This resulted in a number of regions of difference in *L. pneumophila* serogroup 1. Each identified putative target nucleotide sequence was BLAST analysed (blast.ncbi.nlm.nih.gov/Blast.cgi) to determine if they were present in all *L. pneumophila* serogroup 1 and absent in all other *L. pneumophila* serogroups or closely related *Legionella* species. One region was present in all *L. pneumophila* serogroup 1 analysed with very low or no sequence similarity to other *L. pneumophila* serogroups or closely related *Legionella* species. This region was the *Ipg0768* gene (SEQ ID NOs 1 and 21-48) which encodes the spore coat polysaccharide biosynthesis protein E, NeuB. Closely related nucleotide sequences from *Legionella pneumophila* serogroup 1 strains were aligned using ClustalW (www.ebi.ac.uk/Tools/msa/clustalw2/).

By *in silico* alignment, it was demonstrated that there is partial, perfect, or near-perfect, conservation of the each of the following nucleotide sequences: the exemplary *Ipg0768*forward sequencing primer (SEQ ID NO. 16), the exemplary *Ipg0768* forward primer (SEQ ID NO. 2), the exemplary *Ipg0768* reverse sequencing primer (SEQ ID NO. 17), the exemplary *Ipg0768* reverse primer (SEQ ID NO. 3), and the exemplary *Ipg0768* probe (SEQ ID NO. 4); among the *Ipg0768* nucleotide sequences of the following *Legionella pneumophila* serogroup 1 strains: gi|395129037 *L. pneumophila_*extended,
*L. pneumophila_*str.Philadelphia1(sg1), *L. pneumophila_*str.Paris(sg1),
*L. pneumophila_*LPE509(SG1), gi|6688579 *L. pneumophila(sg1), L. pneumophila_*str.Lens(sg1), gi|410173912 *L. pneumophila(sg1), L. pneumophila_*str.Camperdown1(sg1),
*L. pneumophila_*str.Heysham1(sg1), *L. pneumophila_*str.Uppsala3(sg1),
*L. pneumophila*2300/99Alcoy(sg1), *L. pneumophila_*str.Corby(sg1),
*L. pneumophila_*str.uppsala3.b(sg1), *L. pneumophila_*str.Lorraine(sg1),
*L. pneumophila_*str.Goerlitz6543(sg1), *L. pneumophila_*str.HL06041035(sg1),
*L. pneumophila_*str.Hextuple_3a(sg1), *L. pneumophila_*str.Hextuple_2q(sg1),
*L. pneumophila*(sg1)_DSM7513, *L. pneumophila*(sg1)_NCTC11191,
*L. pneumophila*(sg1)_NCTC11231, *L. pneumophila*(sg1)_NCTC11378,
*L. pneumophila*(sg1)_NCTC11404, *L. pneumophila*(sg1)_DSM25019,
*L. pneumophila*(sg1)_DSM25021, *L. pneumophila*(sg1)_DSM25199,
*L. pneumophila*(sg1)*_*DSM25200, *L. pneumophila*(sg1)_DSM25213, and
*L. pneumophila*(sg1)_DSM37564.

### Bacterial strains, culture media, and growth conditions

A panel of 26 *Legionella pneumophila* strains and 41 other *Legionella* species were used in this study (Tables 2.1 and 2.2). These species and strains were purchased from the German Collection of Microorganisms and Cell Cultures (Deutsche Sammlung von Mikroorganismen und Zellkulturen GmbH, DSMZ. Braunschweig, Germany), the National Collection of Type Cultures (NCTC, Public Health England, Salisbury, United Kingdom), American Type Culture Collection (ATCC, provided by LGC standards, Middlesex United Kingdom) and Culture Collection, University of Göteborg, (CCUG, Sweden). All *Legionella* species and strains were cultured on BCYE media at 37°C for 18-24 hours or until sufficient growth was observed.

### Genomic DNA isolation and quantification.

Genomic DNA from *Legionella* cultures were isolated using a modified procedure combining mechanical lysis (IDI lysis kit; Becton Dickenson, Canada) and purification using a DNA purification kit (quick gDNA kit; Zymo Research, Irvine, CA, USA). Briefly, a loop of culture was resuspended in 250 ul lysis buffer (IDI lysis buffer). The suspension was transferred to a cell lysis tube (GeneOhm) and bead beaten (Mini-Bead-Beater-16; Stratech, United Kingdom) for 3 minutes. After bead beating, 200 µl was transferred to a nucleic acid purification spin column (Zymo-Spin^{™} Column) in a collection tube and steps 2 to 5 of the procedure for purification of total DNA from cell suspensions were followed, according to the manufacturer's instructions.

### Nucleotide Sequencing

To generate partial *smpB* nucleotide sequence for *L. pneumophila,* conventional PCR was performed using the sequencing primers outlined in Table 3(a) on a thermal cycler (iCycler iQ thermal cycler; Bio-Rad Laboratories Inc., California, USA). All reactions were carried out in a final volume of 50 µl, containing 5 µl 10x buffer (15 mM MgCl₂), 1 µl forward and reverse primers (0.2 µM final conc.), 2 µl DNA polymerase (Taq; 1 U/µl, Roche Diagnostics, Basel, Switzerland), 1 µl dNTP mix (10 mM deoxynucleoside triphosphate set; Roche Diagnostics), 5 µl of template DNA and 35 µl nuclease free water (Applied Biosystems/Ambion, Texas, USA). The cycling parameters consisted of initial denaturation at 95°C for 5 minutes, followed by 35 cycles of denaturation at 95°C (1 minutes), annealing at 50°C (1 minutes), and extension at 72°C (1 minutes), with a final elongation step at 72°C for 10 minutes.

**Table 3(a): Oligonucleotides used in this study**

| **Name** | **Target gene** | **Function** | **5'-3' Sequence** | **SEQ ID NO.** |
|---|---|---|---|---|
| LegGen-F1 | ssrA | Genus real-time PCR assay forward primer | GGCGACCTGGCTTC | 10 |
| LegGen-R1 | ssrA | Genus real-time PCR assay reverse primer | TATGACCGTTGATTCGATACC | 11 |
| LegGen-P1 | ssrA | Genus real-time PCR assay probe | | 12 |
| LgensmpB_F | smpB | *L. pneumophila* forward Seqeuncing primer | GATCAATACGAAGCAGGC | 14 |
| LgensmpB_R | smpB | *L. pneumophila* | GCCATTCTCTGTCTTTGATC | 15 |
| | | reverse Seqeuncing primer | | |
| LegPneuF1 | smpB | *L. pneumophila* real-time PCR assay forward primer | CACGTGATAATMAAATACGGT | 7 |
| LegPneuR1 | smpB | *L. pneumophila* real-time PCR assay reverse primer | TTCATCAATAGCTTGCGYG | 8 |
| LegPneuR12 | smpB | *L. pneumophila* real-time PCR assay reverse primer 2 | TTCATCAACAGCTTGCGTG | 13 |
| LegPneuP1 | smpB | *L. pneumophila* real-time PCR assay probe | | 9 |
| L.pneu(sg1)_Fseq | region 5' Ipg0768 | *L. pneumophila* sg1 forward Seqeuncing primer | CCAAGTAGGGTTTCTAACAATC | 16 |
| L.pneu(sg1)_Rseq | region 3' Ipg0768 | *L. pneumophila* sg1 reverse Seqeuncing primer | GAGTCAGCCTTTTACAATTGG | 17 |
| L.pneu(sg1)_F | Ipg0768 | *L. pneumophila* sg1 real- | AGCTCCCAATGCAATAGAC | 2 |
| | | time PCR assay forward primer | | |
| L.pneu(sg1)_R | Ipg0768 | *L. pneumophila* sg1 real-time PCR assay reverse primer | GCAAAGCAGTTGAAACAGC | 3 |
| L.pneu(sg1)_P | Ipg0768 | *L. pneumophila* sg1 real-time PCR assay probe | | 4 |
| SIACF1 | synthetic construct | IAC real-time PCR assay forward primer | ATGCCAGTCAGCATAAGGA | 18 |
| SIACF2 | synthetic construct | IAC real-time PCR assay reverse primer | CAGACCTCTGGTAGGATGTAC | 19 |
| SIACP1 | synthetic construct | IAC real-time PCR probe | | 20 |

/56-FAM/ = 5' 6-FAM (fluorescein) fluorophore; /ZEN/ = ZEN^{™} internal quencher, made by Integrated DNA Technologies; /3IABkFQ/ = 3' Iowa Black^{®} FQ terminal quencher, made by Integrated DNA Technologies; /56-ROXN/ = 5' ROX (carboxy-X-rhodamine) n-hydroxysuccinimide ester fluorophore; /5HEX/ = 5' HEX (hexachlorofluorescein) fluorophore; /5Cy5/ = 5' Cy5 (cyanine) fluorophore; /TAO/ = TAO^{™} internal quencher, made by Integrated DNA Technologies; /3IAbRQSp/ = 3' Iowa Black^{®} RQ terminal quencher, made by Integrated DNA Technologies.

To generate the *Ipg0768* gene nucleotide sequence for *L. pneumophila* serogroup 1 strains, conventional PCR was performed using the sequencing primers outlined in Table 3(a) on the thermal cycler (iCycler iQ thermal cycler; Bio-Rad Laboratories Inc., California, USA). All reactions were carried out in a final volume of 50 µl, containing 5 µl 10× buffer (15 mM MgCl₂), 1 µl forward and reverse primers (0.2 µM final conc.), 2 µl DNA polymerase (Taq; 1 U/µl, Roche Diagnostics, Basel, Switzerland), 1 µl dNTP mix (10 mM deoxynucleoside triphosphate set; Roche Diagnostics), 5 µl of template DNA and 35 µl nuclease free water (Applied Biosystems/Ambion, Texas, USA). The cycling parameters consisted of initial denaturation at 95°C for 5 minutes, followed by 35 cycles of denaturation at 95°C (1 minutes), annealing at 50°C (1 minutes), and extension at 72°C (1 minutes), with a final elongation step at 72°C for 10 minutes.

PCR products were visualised on a 1.5% agarose gel and subsequently purified using a PCR product purification kit (High Pure Product Purification Kit; Roche Diagnostics). Purified PCR products were sent for sequencing externally (Sequiserve, Vaterstetten, Germany).

### Assay design

All oligonucleotide primers and probes used in this example were designed in accordance with published guidelines. All oligonucleotide primers and probes were purchased from Integrated DNA Technologies (Leuven, Belgium).

### Development of an Internal Amplification Control (IAC) for real-time PCR

An internal amplification control (IAC) was designed and incorporated in the multiplex assay designed to monitor for PCR inhibition, thermocycler malfunction, degradation of assay components etc. As this multiplex assay is designed for use on environmental samples, a synthetic construct of random sequence was identified to be the most suitable IAC. The synthetic construct sequence is not found in nature and as such the specific oligonucleotide primers and probes will not be mopped up by competing microorganisms commonly found in the environment.

In this example, the following exemplary real-time PCR assay primers and probe for the IAC were used: IAC synthetic construct forward primer (SEQ ID NO. 18), IAC synthetic construct reverse primer (SEQ ID NO. 19), IAC synthetic construct probe (SEQ ID NO. 20). The IAC synthetic construct probe (SEQ ID NO. 20) comprised at least one marker, optionally at least one dye, optionally at least one fluorophore, optionally cyanine (5' Cy5^{™}, Integrated DNA Technologies). Optionally, the synthetic construct probe (SEQ ID NO. 20) comprised at least one fluorescence quenching dye, optionally an internal quencher (TAO^{™} quencher, Integrated DNA Technologies), optionally a terminal quencher (3' Iowa Black^{®} RQ, Integrated DNA Technologies).

Titrations of synthetic DNA were performed to determine the optimum concentration of IAC target per reaction such that the IAC is always detected without impacting on detection of the primary *Legionella* targets in the multiplex. An internal control concentration of 1000 genome equivalents per reaction allowed for the detection of the IAC all real-time PCR experiments performed.

A multiplex PCR assay was produced using a combination of oligonucleotide primers and probes selected from: the oligonucleotide primers and probes listed in Table 3(a), and the oligonucleotide primers and probes described in example 1, the oligonucleotide primers and probes described in example 2, and the oligonucleotide primers and probes for the IAC synthetic construct described in this example.

### Real-time PCR

To determine the specificity of each of the diagnostics target used in this study, Monoplex real-time PCR was performed on a thermal cycler (LightCycler 480; Roche Diagnostics, Basel, Switzerland) using a probes kit (LightCycler^{®} Probes Master kit; Roche Diagnostics). A final volume of 20 µl was used in each reaction, containing 2X PCR reaction master mixture (master mix) [LightCycler 480 Probes Master (6.4mM MgCl₂)], forward and reverse primer (0.5mM final conc.), FAM, HEX, ROX or CY5 labelled probe (0.2 mM final conc.), template DNA (2 µl) and the volume adjusted to 20 µl with the addition of nuclease free dH₂O. The cycling parameters consisted of incubation for 10 minutes at 95°C to activate enzymes and DNA denaturation followed by 50 cycles of 95°C for 10 seconds and 60°C for 30 s, followed by a cooling step at 40°C for 10 s. The temperature transition rate for all cycling steps was 4.4°C/s while heating and 2.2°C/s while cooling.

Multiplex real-time PCR reactions were carried out on a thermal cycler (LightCycler 480; Roche Diagnostics, Basel, Switzerland) using a probes kit (LightCycler^{®} Probes Master kit; Roche Diagnostics). A final volume of 20 µl was used for each multiplex experiment. The optimised master mix contained 2X PCR reaction master mixture [LightCycler 480 Probes Master (6.4 mM MgCl₂)], forward and reverse primer (0.125-0.25 mM final conc.), FAM, HEX, ROX and CY5 labelled dyes (0.2-0.4 µM final conc.), template DNA (5 µl), IAC DNA 1 µl, and the volume adjusted to 20 µl with the addition of nuclease free dH₂O. The internal control DNA was diluted to contain 1000 genome equivalents per µl and all other DNAs were tested at a range of 10*3-10*4 genome equivalents per 5 µl. The cycling parameters used were the same as those used in a monoplex format outlined above. A colour compensation file was generated using the technical note outlined in the Advanced Software Functionalities of the operator manual.

### Sensitivity and specificity of the real-time PCR assays

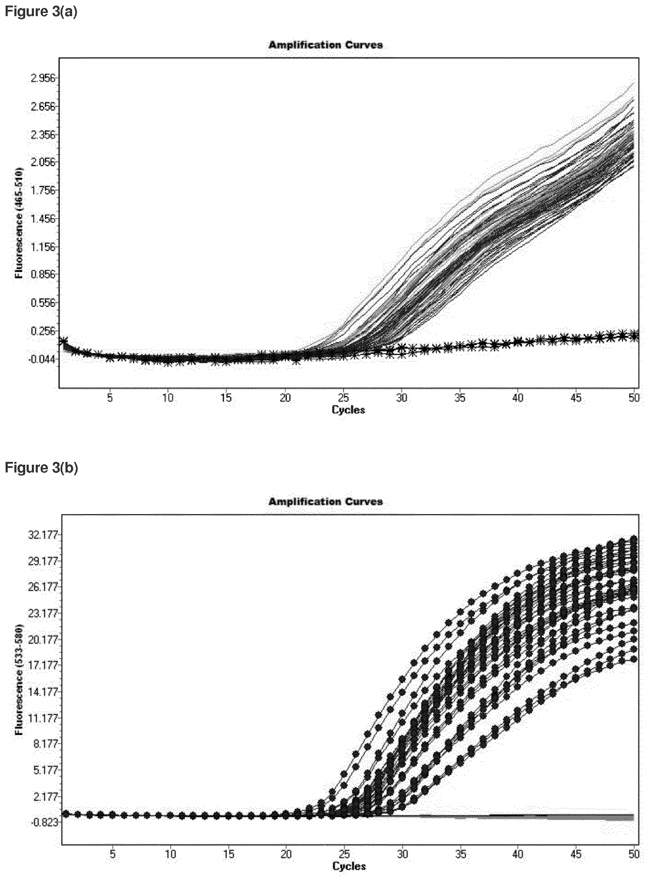

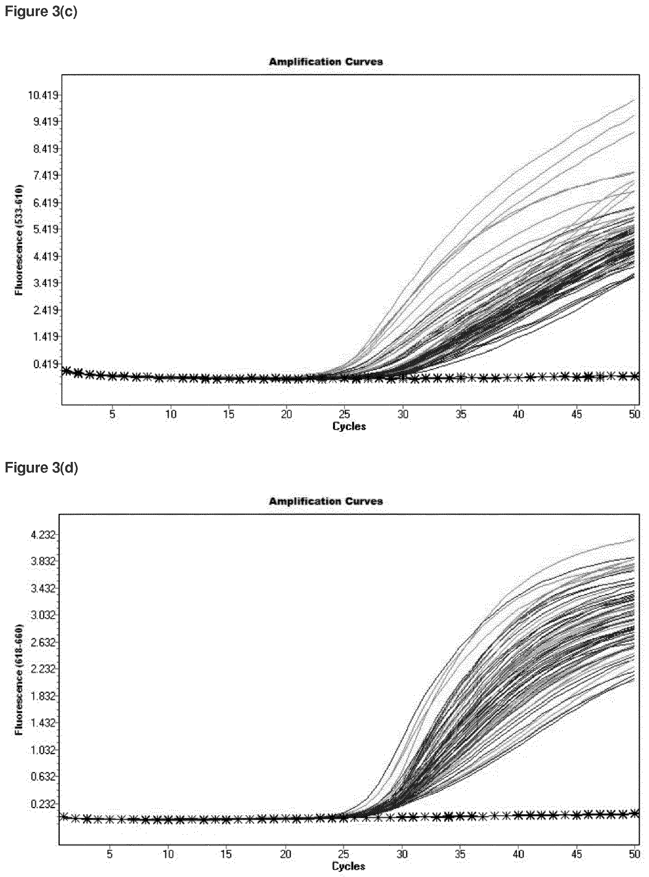

The specificity of each real-time PCR assay was confirmed both in monoplex and multiplex formats using the specificity panel listed in Table 3(b) and Table 3(c). Using the multiplex assay, all of the *Legionella* strains were detected in the *Legionella* genus assay targeting a region of the *ssrA* gene. A representation of this can be seen in Figure 3(a), in which the negative control is represented by a line marked with stars. The *L. pneumophila* serogroup 1 specific assay targeting a region of the *Ipg0768* gene was specific for the detection of *L. pneumophila* serogroup 1 isolates. A representation of this can be seen in Figure 3(b), in which the 12 *L. pneumophila* serogroup 1 strains tested are represented by lines marked with circles. All 12 *L. pneumophila* serogroup 1 strains were detected by the assay. The *L. pneumophila* species assay targeting a region of the *smpB* gene was specific for the detection of the 26 *L. pneumophila* strains tested for and the 41 other *Legionella* species were not detected by this assay. A representation of this can be seen in Figure 3(c), in which the negative control is represented by a line marked with stars. The specificity of the IAC assay was tested using the full specificity panel (Table 3(b) and Table 3(c)) and was specific for the synthetic construct. As the IAC assay is designed using a non-competitive approach, when spiked into the master mix a positive signal should always be observed in the Cy5 channel. A representation of this can be seen in Figure 3(d), in which the no template control is represented by a line marked with stars. The analytical specificity of this multiplex real-time PCR assay is 100%.

To determine the sensitivity of the multiplex real-time PCR assay developed, serial dilutions ranging from 10*4-1 cell equivalent of *L. pneumophila* were tested in triplicate. The limit of detection of the multiplex assay is between 1~10 genome equivalents.

**Table 3(b) Legionella pneumophila strains used in this study**

| Strain | Culture collection | Number | Serogroup |
|---|---|---|---|
| *Legionella pneumophila* | DSM | 7513 | 1 |
| *Legionella pneumophila* | NCTC | 11191 | 1 |
| *Legionella pneumophila* | NCTC | 11231 | 1 |
| *Legionella pneumophila* | NCTC | 11286 | 1 |
| *Legionella pneumophila* | NCTC | 11378 | 1 |
| *Legionella pneumophila* | NCTC | 11404 | 1 |
| *Legionella pneumophila* | DSM | 25019 | 1 |
| *Legionella pneumophila* | DSM | 25021 | 1 |
| *Legionella pneumophila* | DSM | 25199 | 1 |
| *Legionella pneumophila* | DSM | 25200 | 1 |
| *Legionella pneumophila* | DSM | 25213 | 1 |
| *Legionella pneumophila* | DSM | 27564 | 1 |
| *Legionella pneumophila* | NCTC | 11230 | 2 |
| *Legionella pneumophila* | NCTC | 11232 | 3 |
| *Legionella pneumophila* | DSM | 7414 | 4 |
| *Legionella pneumophila* | DSM | 7415 | 5 |
| *Legionella pneumophila* | NCTC | 11417 | 5 |
| *Legionella pneumophila* | NCTC | 11287 | 6 |
| *Legionella pneumophila* | NCTC | 11984 | 7 |
| *Legionella pneumophila* | NCTC | 11985 | 8 |
| *Legionella pneumophila* | NCTC | 11986 | 9 |
| *Legionella pneumophila* | NCTC | 12000 | 10 |
| *Legionella pneumophila* | NCTC | 12179 | 11 |
| *Legionella pneumophila* | NCTC | 12180 | 12 |
| *Legionella pneumophila* | NCTC | 12181 | 13 |
| *Legionella pneumophila* | NCTC | 12174 | 14 |

| | | | |
|---|---|---|---|
| *DSM = The German Collection of Microorganisms; *NCTC = National Collection of Type Cultures | | | |

**Table 3(c) Other Legionella species tested in this study**

| **Strain** | **Source** |
|---|---|
| *Legionella anisa* | DSM 17627 |
| *Legionella birminghamensis* | DSM 19232 |
| *Legionella birminghamensis* | ATCC 700709 |
| *Fluoribacter bozemanae* (*Legionella bozemanii*) | DSM 16523 |
| *Fluoribacter bozemanae-2* | NCTC 11975 |
| *Legionella cincinnatiensis* | DSM 19233 |
| *Fluoribacter dumoffii* (*Legionella dumoffii)* | DSM 17625 |
| *Fluoribacter dumoffii* | ATCC 33343 |
| *Legionella erythra* | DSM 17644 |
| *Legionella feeleii-1* | DSM 17645 |
| *Legionella feeleii-2* | NCTC 11978 |
| *Flouribacter gormanii* (*Legionella gormanii*) | DSM 16641 |
| *Fluoribacter gormanii* | ATCC 43769 |
| *Legionella hackeliae*-1 | DSM 19214 |
| *Legionella hackeliae*-2 | NCTC 11980 |
| *Legionella jordanis* | DSM 19212 |
| *Legionella jordanis* | ATCC 700762 |
| *Legionella lansingensis* | DSM 19556 |
| *Legionella longbeachae-*1 | DSM 10572 |
| *Legionella maceachwerii* | DSM 10572 |
| *Tatlockia micdadei* (*Legionella micdadei*) | NCTC 11403 |
| *Legionella oakridgensis* | NCTC 11531 |
| *Legionella oakridgensis* | ATCC 700515 |
| *Legionella parisiensis* | DSM 19216 |
| *Legionella wadsworthii* | NCTC 11532 |
| *Legionella adelaidensis* | DSM 19888 |
| *Legionella brunensis* | DSM 19236 |
| *Legionella busanesis* | ATCC-BAA518 |
| *Legionella cherrii* | DSM 19213 |
| *Legionella donaldsonii* | ATCC BAA-693 |
| *Legionella fairfieldensis* | NCTC 12488 |
| *Legionella fallonii* | CCUG 43887 |
| *Legionella gratiana* | DSM 21233 |
| *Legionella gresilensis* | DSM 21218 |
| *Legionella israelensi* | DSM 19235 |
| *Legionella jamestowniensis* | DSM 19215 |
| *Legionella londoniensis* | DSM 21234 |
| *Legionella moravica* | DSM 19234 |
| *Legionella quinlivanii*-1 | NCTC 12433 |
| *Legionella rubrilicens* | DSM 11884 |
| *Legionella taurinsis* | CCUG 44901 |

| | |
|---|---|
| *DSM = The German Collection of Microorganisms; *ATCC = American Type Culture Collection; *NCTC = National Collection of Type Cultures; *CCUG=Culture Collection, University of Göteborg | |

### Example 5 - Diagnostics algorithm

For determining which *Legionella* species and/or strains are present in a sample, the user must take into account the combination of results observed for each channel of the real-time *in vitro* amplification instrument. This are set out in Table 4 below, and explained below.

**Table 4 Result of multiplex PCRs associated with each diagnosis**

| **Test** | **Multiplex 1** | | | | |
|---|---|---|---|---|---|
| **(Target)** | ***(ssrA)*** | ***(*lpg07 68*)*** | ***(smpB)*** | **(IAC)** | |
| | • | • | • | • | *Legionella pneumophila* serogroup 1 |
| | • | X | • | • | *Legionella pneumophila* serogroup 2-16 |
| | • | X | X | • | *Legionella species other than Legionella pneumophila* |
| | X | X | X | • | *Legionella* species not present |
| | X | X | X | X | Result invalid |

| | | | | | |
|---|---|---|---|---|---|
| Key: • = positive signal, X = not detected | | | | | |

### Example 6: An internally controlled multiplex real-time PCR for the detection and identification of Legionella species, Legionella pneumophila (serogroup 1-16) and Legionella pneumophila serogroup 1

### Diagnostic target identification

A number of housekeeping genes which are highly conserved throughout the *Legionella* genus were evaluated to identify, *in silico,* a potential diagnostic target which could be used to detect all members of this genus and for the detection of *Legionella pneumophila* strains. The optimal diagnostic target chosen in this study for detection of the *Legionella* genus was the *ssrA* gene. The *ssrA* gene codes for tmRNA and has been identified in all bacterial species. The function of tmRNA in bacteria is to rescue stalled ribosomes and to clear the cell of incomplete polypeptides. *In silico* alignment of publically available nucleotide sequences indicated that while sufficient nucleotide sequence heterogeneity exists between different *Legionella* species, sufficient nucleotide sequence similarity existed to design genus oligonucleotide primers and probes.

In this example, the following exemplary real-time PCR assay primers and probe for the *ssrA* gene nucleotide sequence (SEQ ID NO. 5) were used: ssrA forward primer (SEQ ID NO. 10), ssrA reverse primer (SEQ ID NO. 11), ssrA probe (SEQ ID NO. 12). The ssrA probe (SEQ ID NO. 12) comprised at least one marker, optionally at least one dye, optionally at least one fluorophore, optionally fluorescein (5' 6-FAM, Integrated DNA Technologies). Optionally, the ssrA probe (SEQ ID NO. 12) comprised at least one fluorescence quenching dye, optionally an internal quencher (ZEN^{™} quencher, Integrated DNA Technologies), optionally a terminal quencher (3' Iowa Black^{®} FQ, Integrated DNA Technologies; or Black Hole Quencher 1^{®}, LGC Biosearch).

By *in silico* alignment, it was demonstrated that there is perfect, or near-perfect, conservation of the each of the following nucleotide sequences: the exemplary ssrA forward primer (SEQ ID NO. 10), the exemplary ssrA reverse primer (SEQ ID NO. 11), and the exemplary ssrA probe (SEQ ID NO. 12); among the ssrA gene nucleotide sequences of the following *Legionella* species:
*L. pneumophilaATCC43290, L. pneumophila_*ThunderBay, *L. pneumophila_*HL06041035,
*L. pneumophila_*str.Philadelphia, *L. pneumophila_*Lens, *L. pneumophila_*Paris,
*L. pneumophila_*Corby, *L. pneumophila_*2300/99Alcoy, *L. pneumophila_*Lorraine, *L. longbeachaeD-*4968, *L. longbeachae*NSW150, *L. fallonii_*LLAP-10, *L. hackeliae_LHA, L. oakridgensis_*ATCC33761
*L. micdadei_*LMI, *L. sainthelensi_*ATCC35248, *L. wadsworthii*DSM21896, *L. cherrii_*DSM19213,
*L. moravica*DSM19234, *L. norrlandica, L. shakespearei*DSM23087, *L. drancourtii*LLAP12
*L. lansingensis_*DSM19556, *L. fairfieldensis_*ATCC*_*49588, *L. massiliensis*_PRJEB110, and
*L. geestiana*DSM21217*.*

The optimal diagnostic target chosen in this study for detection of the *Legionella pneumophila* was the *smpB* gene. The *smpB* gene encodes the RNA binding protein Small Protein B (SmpB) and has been identified in all bacterial species to date. It is considered an essential component of quality control in bacteria as it facilitates the binding of tmRNA to stalled ribosomes which in turn allows for the removal of incomplete polypeptides from the cell. *In silico* alignment of publically available nucleotide sequences indicated that sufficient nucleotide sequence similarity exists between different serogroups of *Legionella pneumophila* to allow for the design of species specific oligonucleotide primers and probes.

In this example, the following exemplary real-time PCR assay primers and probe for the *smpB* gene nucleotide sequence (SEQ ID NO. 6) were used: smpB forward primer (SEQ ID NO. 49), smpB reverse primer (SEQ ID NO. 50), smpB probes (SEQ ID NO. 51 and SEQ ID NO. 52). The smpB probes (SEQ ID NO. 51 and SEQ ID NO. 52) comprised at least one marker, optionally at least one dye, optionally at least one fluorophore, optionally rhodamine (5' ROX, Integrated DNA Technologies). Optionally, the smpB probes (SEQ ID NO. 51 and SEQ ID NO. 52) comprised at least one fluorescence quenching dye, optionally a terminal quencher (3' Iowa Black^{®} RQ, Integrated DNA Technologies; or Black Hole Quencher 2^{®}, LGC Biosearch).

By *in silico* alignment, it was demonstrated that there is perfect, or near-perfect, conservation of the each of the following nucleotide sequences: the exemplary *smpB* forward sequencing primer (SEQ ID NO. 14), the exemplary *smpB* forward primer (SEQ ID NO. 49), the exemplary *smpB* reverse sequencing primer (SEQ ID NO. 15), the exemplary *smpB* reverse primer (SEQ ID NO. 50), and the exemplary *smpB* probes (SEQ ID NO. 51 and SEQ ID NO. 52); among the *smpB* nucleotide sequences of the following *Legionella* species: *L. pneumophila_*Paris, *L. pneumophila_Corby,*
*L. pneumophila_Pneu, L. pneumophila_Lens, L. pneumophila_*Alcoy, *L. pneumophila_*2868,
*L. pneumophila_*3140, *L. pneumophila_3194, L. pneumophila_*00189, *L. pneumophila_2829,*
*L. pneumophila_*NCTC11986, *L. pneumophila_NCTC12000, L. pneumophila_*NCTC12179,
*L. pneumophila_*DSM7513, *L. pneumophila*DSM7514, *L. pneumophila*DSM7515,
*L. pneumophila_*NCTC11191, *L. pneumophila_NCTC11230, L. pneumophila_*NCTC11232,
*L. pneumophila_*NCTC11287, *L. pneumophila_*NCTC11984, *L. pneumophila_*NCTC11985*.*

Based on the evaluation of housekeeping genes performed in this study, it was not possible to identify a *Legionella pneumophila* serogroup 1 specific target. As such whole genome comparisons were performed to identify a novel diagnostics target *in silico.* Publicly available whole genome sequence information was retrieved from the National Center for Biotechnology Information website (www.ncbi.nlm.nih.gov/) for *Legionella pneumophila* serogroup 1 and *Legionella pneumophila* serogroup 6 and serogroup 12. Regions potentially unique to *L. pneumophila* serogroup 1 were identified using WEBACT (www.webact.org/). This resulted in a number of regions of difference in *L. pneumophila* serogroup 1. Each identified putative target nucleotide sequence was BLAST analysed (blast.ncbi.nlm.nih.gov/Blast.cgi) to determine if they were present in all *L. pneumophila* serogroup 1 and absent in all other *L. pneumophila* serogroups or closely related *Legionella* species. One region was present in all *L. pneumophila* serogroup 1 analysed with very low or no sequence similarity to other *L. pneumophila* serogroups or closely related *Legionella* species. This region was the *Ipg0768* gene (SEQ ID NO. 1, 21-49) which encodes the spore coat polysaccharide biosynthesis protein E, NeuB. Closely related nucleotide sequences from *Legionella pneumophila* serogroup 1 strains were aligned using ClustalW (www.ebi.ac.uk/Tools/msa/clustalw2/).

By *in silico* alignment, it was demonstrated that there is partial, perfect, or near-perfect, conservation of the each of the following nucleotide sequences: the exemplary *Ipg0768*forward sequencing primer (SEQ ID NO. 16), the exemplary *Ipg0768* forward primer (SEQ ID NO. 2), the exemplary *Ipg0768* reverse sequencing primer (SEQ ID NO. 17), the exemplary *Ipg0768* reverse primer (SEQ ID NO. 3), and the exemplary *Ipg0768* probe (SEQ ID NO. 4); among the *Ipg0768* nucleotide sequences of the following *Legionella pneumophila* serogroup 1 strains: gi|395129037 *L. pneumophila_*extended,
*L. pneumophila_*str.Philadelphia1(sg1), *L. pneumophila_*str.Paris(sg1),
*L. pneumophila_*LPE509(SG1), gi|6688579 *L. pneumophila(sg1), L. pneumophila_*str.Lens(sg1), gi|410173912 *L. pneumophila(sg1), L. pneumophila_*str.Camperdown1(sg1),
*L. pneumophila_*str.Heysham1(sg1), *L. pneumophila_*str.Uppsala3(sg1),
*L. pneumophila*2300/99Alcoy(sg1), *L. pneumophila_str.Corby(sg1),*
*L. pneumophila_*str.uppsala3.b(sg1), *L. pneumophila_*str.Lorraine(sg1),
*L. pneumophila_*str.Goerlitz6543(sg1), *L. pneumophila_*str.HL06041035(sg1),
*L. pneumophila_*str.Hextuple_3a(sg1), *L. pneumophila_*str.Hextuple*_*2q(sg1),
*L. pneumophila*(sg1)_DSM7513, *L. pneumophila*(sg1)_NCTC11191,
*L. pneumophila*(sg1)_NCTC11231, *L. pneumophila*(sg1)_NCTC11378,
*L. pneumophila*(sg1)_NCTC11404, *L. pneumophila*(sg1)_DSM25019,
*L. pneumophila*(sg1)_DSM25021, *L. pneumophila*(sg1)_DSM25199,
*L. pneumophila*(sg1)_DSM25200, *L. pneumophila*(sg1)_DSM25213, and
*L. pneumophila*(sg1)_DSM37564*.*

### Bacterial strains, culture media, and growth conditions

A panel of 26 *Legionella pneumophila* strains and 41 other *Legionella* species were used in this study (Tables 2.1 and 2.2). These species and strains were purchased from the German Collection of Microorganisms and Cell Cultures (Deutsche Sammlung von Mikroorganismen und Zellkulturen GmbH, DSMZ. Braunschweig, Germany), the National Collection of Type Cultures (NCTC, Public Health England, Salisbury, United Kingdom), American Type Culture Collection (ATCC, provided by LGC standards, Middlesex United Kingdom) and Culture Collection, University of Göteborg, (CCUG, Sweden). All *Legionella* species and strains were cultured on BCYE media at 37°C for 18-24 hours or until sufficient growth was observed.

### Genomic DNA isolation and quantification.

Genomic DNA from *Legionella* cultures were isolated using a modified procedure combining mechanical lysis (IDI lysis kit; Becton Dickenson, Canada) and purification using a DNA purification kit (quick gDNA kit; Zymo Research, Irvine, CA, USA). Briefly, a loop of culture was resuspended in 250 ul lysis buffer (IDI lysis buffer). The suspension was transferred to a cell lysis tube (GeneOhm) and bead beaten (Mini-Bead-Beater-16; Stratech, United Kingdom) for 3 minutes. After bead beating, 200 µl was transferred to a nucleic acid purification spin column (Zymo-Spin^{™} Column) in a collection tube and steps 2 to 5 of the procedure for purification of total DNA from cell suspensions were followed, according to the manufacturer's instructions.

### Nucleotide Sequencing

To generate partial *smpB* nucleotide sequence for *L. pneumophila,* conventional PCR was performed using the sequencing primers outlined in Table 5(a) on a thermal cycler (iCycler iQ thermal cycler; Bio-Rad Laboratories Inc., California, USA). All reactions were carried out in a final volume of 50 µl, containing 5 µl 10x buffer (15 mM MgCl₂), 1 µl forward and reverse primers (0.2 µM final conc.), 2 µl DNA polymerase (Taq; 1 U/µl, Roche Diagnostics, Basel, Switzerland), 1 µl dNTP mix (10 mM deoxynucleoside triphosphate set; Roche Diagnostics), 5 µl of template DNA and 35 µl nuclease free water (Applied Biosystems/Ambion, Texas, USA). The cycling parameters consisted of initial denaturation at 95°C for 5 minutes, followed by 35 cycles of denaturation at 95°C (1 minutes), annealing at 50°C (1 minutes), and extension at 72°C (1 minutes), with a final elongation step at 72°C for 10 minutes.

**Table 5(a): Oligonucleotides used in this study**

| **Name** | **Target gene** | **Function** | **5'-3' Sequence** | **SEQ ID NO.** |
|---|---|---|---|---|
| LegGen-F1 | ssrA | Genus real-time PCR assay forward primer | GGCGACCTGGCTTC | 10 |
| LegGen-R1 | ssrA | Genus real-time PCR assay reverse primer | TATGACCGTTGATTCGATACC | 11 |
| LegGen-P1 | ssrA | Genus real-time PCR assay probe | | 12 |
| LgensmpB_F | smpB | *L. pneumophila* forward Seqeuncing primer | GATCAATACGAAGCAGGC | 14 |
| LgensmpB_R | smpB | *L. pneumophila* reverse Seqeuncing primer | GCCATTCTCTGTCTTTGATC | 15 |
| Legionella sg1-16 F2 | smpB | *L. pneumophila* real-time PCR assay forward primer | GATCAATACGAAGCAGGC | 49 |
| Legionella sg1-16 R2 | smpB | *L. pneumophila* real-time | CCTTGYCTTTCAACACTTCC | 50 |
| | | PCR assay reverse primer | | |
| Legionella sg1-16 P2 | smpB | *L. pneumophila* real-time | | 51 |
| | | PCR assay probe | | |
| Legionella sg1-16 P3 | smpB | *L. pneumophila* real-time PCR assay probe | | 52 |
| L.pneu(sg1)_Fseq | region 5' Ipg0768 | *L. pneumophila* sg1 forward Seqeuncing primer | CCAAGTAGGGTTTCTAACAATC | 16 |
| L.pneu(sg1)_Rseq | region 3' Ipg0768 | *L. pneumophila* sg1 reverse Seqeuncing primer | GAGTCAGCCTTTTACAATTGG | 17 |
| L.pneu(sg1)_F | Ipg0768 | *L. pneumophila* sg1 real-time PCR assay forward primer | AGCTCCCAATGCAATAGAC | 2 |
| L.pneu(sg1)_R | Ipg0768 | *L. pneumophila* sg1 real-time PCR assay reverse primer | GCAAAGCAGTTGAAACAGC | 3 |
| L.pneu(sg1)_P | Ipg0768 | *L. pneumophila* | | 4 |
| | | sg1 real-time PCR assay probe | | |
| SIACF1 | synthetic construct | IAC real-time PCR assay forward primer | ATGCCAGTCAGCATAAGGA | 18 |
| SIACF2 | synthetic construct | IAC real-time PCR assay reverse primer | CAGACCTCTGGTAGGATGTAC | 19 |
| SIACP1 | synthetic construct | IAC real-time PCR probe | | 20 |

/56-FAM/ = 5' 6-FAM (fluorescein) fluorophore; /ZEN/ = ZEN^{™} internal quencher, made by Integrated DNA Technologies; /3IABkFQ/ = 3' Iowa Black^{®} FQ terminal quencher, made by Integrated DNA Technologies; /56-ROXN/ = 5' ROX (carboxy-X-rhodamine) n-hydroxysuccinimide ester fluorophore; /5HEX/ = 5' HEX (hexachlorofluorescein) fluorophore; /5Cy5/ = 5' Cy5 (cyanine) fluorophore; /TAO/ = TAO^{™} internal quencher, made by Integrated DNA Technologies; /3IAbRQSp/ = 3' Iowa Black^{®} RQ terminal quencher, made by Integrated DNA Technologies.

To generate the *Ipg0768* gene nucleotide sequence for *L. pneumophila* serogroup 1 strains, conventional PCR was performed using the sequencing primers outlined in Table 5(a) on the thermal cycler (iCycler iQ thermal cycler; Bio-Rad Laboratories Inc., California, USA). All reactions were carried out in a final volume of 50 µl, containing 5 µl 10_{×} buffer (15 mM MgCl₂), 1 µl forward and reverse primers (0.2 µM final conc.), 2 µl DNA polymerase (Taq; 1 U/µl, Roche Diagnostics, Basel, Switzerland), 1 µl dNTP mix (10 mM deoxynucleoside triphosphate set; Roche Diagnostics), 5 µl of template DNA and 35 µl nuclease free water (Applied Biosystems/Ambion, Texas, USA). The cycling parameters consisted of initial denaturation at 95°C for 5 minutes, followed by 35 cycles of denaturation at 95°C (1 minutes), annealing at 50°C (1 minutes), and extension at 72°C (1 minutes), with a final elongation step at 72°C for 10 minutes.

PCR products were visualised on a 1.5% agarose gel and subsequently purified using a PCR product purification kit (High Pure Product Purification Kit; Roche Diagnostics). Purified PCR products were sent for sequencing externally (Sequiserve, Vaterstetten, Germany).

### Assay design

All oligonucleotide primers and probes used in this example were designed in accordance with published guidelines. All oligonucleotide primers and probes were purchased from Integrated DNA Technologies (Leuven, Belgium).

### Development of an Internal Amplification Control (IAC) for real-time PCR

An internal amplification control (IAC) was designed and incorporated in the multiplex assay designed to monitor for PCR inhibition, thermocycler malfunction, degradation of assay components etc. As this multiplex assay is designed for use on environmental samples, a synthetic construct of random sequence was identified to be the most suitable IAC. The synthetic construct sequence is not found in nature and as such the specific oligonucleotide primers and probes will not be mopped up by competing microorganisms commonly found in the environment.

In this example, the following exemplary real-time PCR assay primers and probe for the IAC were used: IAC synthetic construct forward primer (SEQ ID NO. 18), IAC synthetic construct reverse primer (SEQ ID NO. 19), IAC synthetic construct probe (SEQ ID NO. 20). The IAC synthetic construct probe (SEQ ID NO. 20) comprised at least one marker, optionally at least one dye, optionally at least one fluorophore, optionally cyanine (5' Cy5^{™}, Integrated DNA Technologies). Optionally, the synthetic construct probe (SEQ ID NO. 20) comprised at least one fluorescence quenching dye, optionally an internal quencher (TAO^{™} quencher, Integrated DNA Technologies), optionally a terminal quencher (3' Iowa Black^{®} RQ, Integrated DNA Technologies).

Titrations of synthetic DNA were performed to determine the optimum concentration of IAC target per reaction such that the IAC is always detected without impacting on detection of the primary *Legionella* targets in the multiplex. An internal control concentration of 1000 genome equivalents per reaction allowed for the detection of the IAC all real-time PCR experiments performed.

A multiplex PCR assay was produced using a combination of oligonucleotide primers and probes selected from: the oligonucleotide primers and probes listed in Table 5(a), and the oligonucleotide primers and probes described in example 1, the oligonucleotide primers and probes described in example 2, and the oligonucleotide primers and probes for the IAC synthetic construct described in this example.

### Real-time PCR

To determine the specificity of each of the diagnostics target used in this study, Monoplex real-time PCR was performed on a thermal cycler (LightCycler 480; Roche Diagnostics, Basel, Switzerland) using a probes kit (LightCycler^{®} Probes Master kit; Roche Diagnostics). A final volume of 20 µl was used in each reaction, containing 2X PCR reaction master mixture (master mix) [LightCycler 480 Probes Master (6.4mM MgCl₂)], forward and reverse primer (0. 25mM final conc. for Legionella genus and *L. pneumophila* sg1-16 assays; 0.50 mM final conc. for *L. pneumophila* sg1 assay; 0.3 mM final conc. for IAC assay), FAM (0.6 mM final conc.), HEX (0.2 mM final conc.), ROX (0.2 mM final conc.) or CY5 labelled probe (0.6 mM final conc.), template DNA (2 µl) and the volume adjusted to 20 µl with the addition of nuclease free dH₂O. The cycling parameters consisted of incubation for 10 minutes at 95°C to activate enzymes and DNA denaturation followed by 50 cycles of 95°C for 10 seconds and 60°C for 30 s, followed by a cooling step at 40°C for 10 s. The temperature transition rate for all cycling steps was 4.4°C/s while heating and 2.2°C/s while cooling.

Multiplex real-time PCR reactions were carried out on a thermal cycler (LightCycler 480; Roche Diagnostics, Basel, Switzerland) using a probes kit (LightCycler^{®} Probes Master kit; Roche Diagnostics). A final volume of 20 µl was used for each multiplex experiment. The optimised master mix contained 2X PCR reaction master mixture [LightCycler 480 Probes Master (6.4 mM MgCl₂)], forward and reverse primer (0.25-0.5 mM final conc.), FAM, HEX, ROX and CY5 labelled dyes (0.2-0.6 µM final conc.), template DNA (5 µl), IAC DNA 1 µl, and the volume adjusted to 20 µl with the addition of nuclease free dH₂O. The internal control DNA was diluted to contain 1000 genome equivalents per µl and all other DNAs were tested at a range of 10*3-10*4 genome equivalents per 5 µl. The cycling parameters used were the same as those used in a monoplex format outlined above. A colour compensation file was generated using the technical note outlined in the Advanced Software Functionalities of the operator manual.

### Sensitivity and specificity of the real-time PCR assays

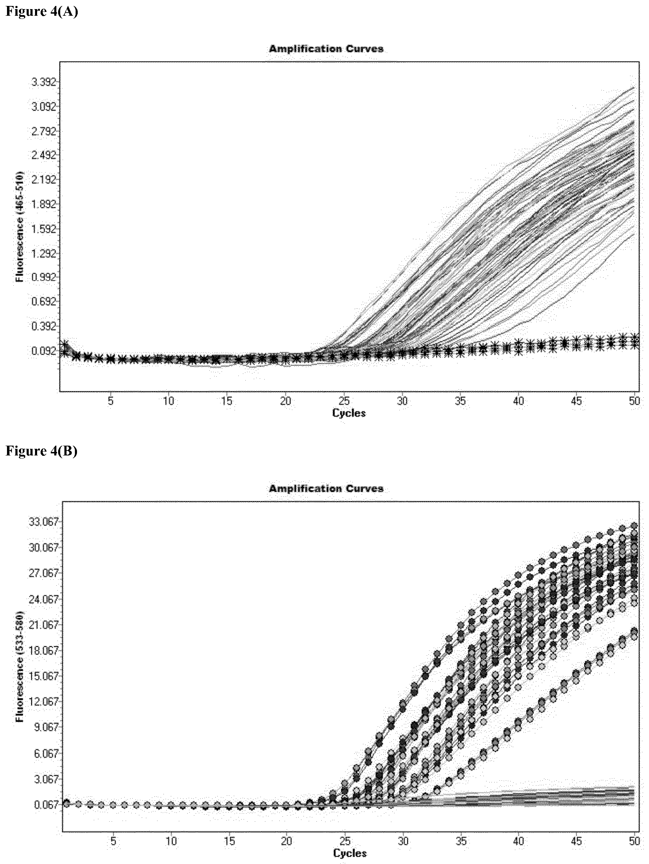

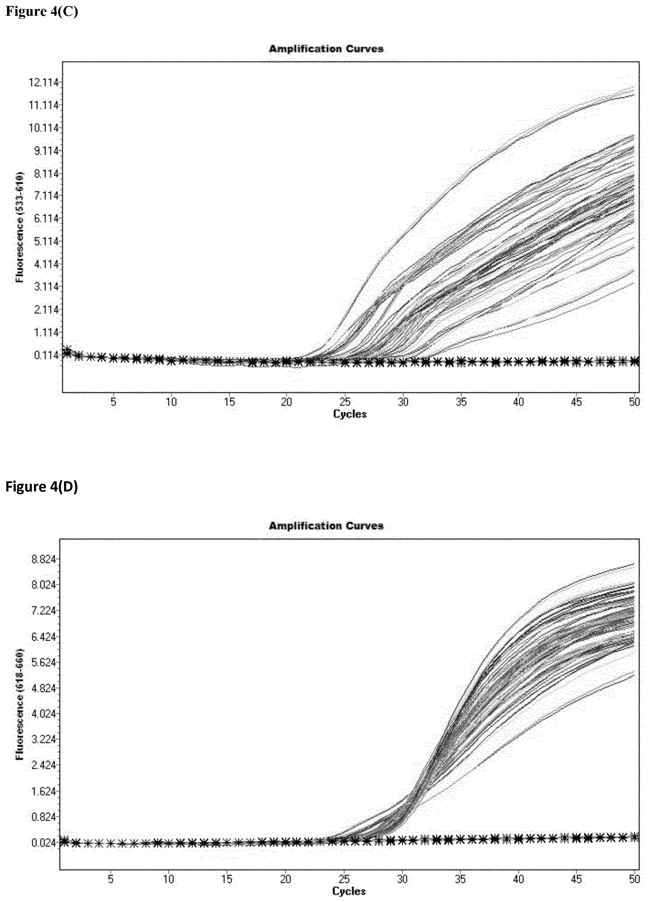

The specificity of each real-time PCR assay was confirmed both in monoplex and multiplex formats using the specificity panel listed in Table 5(b) and Table 5(c). Using the multiplex assay, all of the *Legionella* strains were detected in the *Legionella* genus assay targeting a region of the *ssrA* gene. A representation of this can be seen in Figure 4(a), in which the negative control is represented by a line marked with stars. The *L. pneumophila* serogroup 1 specific assay targeting a region of the *Ipg0768* gene was specific for the detection of *L. pneumophila* serogroup 1 isolates. A representation of this can be seen in Figure 4(b), in which the 12 *L. pneumophila* serogroup 1 strains tested are represented by lines marked with circles. All 12 *L. pneumophila* serogroup 1 strains were detected by the assay. The *L. pneumophila* species assay targeting a region of the *smpB* gene was specific for the detection of the 26 *L. pneumophila* strains tested for and the 41 other *Legionella* species were not detected by this assay. A representation of this can be seen in Figure 4(c), in which the negative control is represented by a line marked with stars. The specificity of the IAC assay was tested using the full specificity panel (Table 5(b) and Table 5(c)) and was specific for the synthetic construct. As the IAC assay is designed using a non-competitive approach, when spiked into the master mix a positive signal should always be observed in the Cy5 channel. A representation of this can be seen in Figure 4(d), in which the no template control is represented by a line marked with stars. The analytical specificity of this multiplex real-time PCR assay is 100%.

To determine the sensitivity of the multiplex real-time PCR assay developed, serial dilutions ranging from 10*4-1 cell equivalent of *L. pneumophila* were tested in triplicate. The limit of detection of the multiplex assay is between 1~10 genome equivalents.

**Table 5(b) Legionella pneumophila strains used in this study**

| Strain | Culture collection | Number | Serogroup |
|---|---|---|---|
| *Legionella pneumophila* | DSM | 7513 | 1 |
| *Legionella pneumophila* | NCTC | 11191 | 1 |
| *Legionella pneumophila* | NCTC | 11231 | 1 |
| *Legionella pneumophila* | NCTC | 11286 | 1 |
| *Legionella pneumophila* | NCTC | 11378 | 1 |
| *Legionella pneumophila* | NCTC | 11404 | 1 |
| *Legionella pneumophila* | DSM | 25019 | 1 |
| *Legionella pneumophila* | DSM | 25021 | 1 |
| *Legionella pneumophila* | DSM | 25199 | 1 |
| *Legionella pneumophila* | DSM | 25200 | 1 |
| *Legionella pneumophila* | DSM | 25213 | 1 |
| *Legionella pneumophila* | DSM | 27564 | 1 |
| *Legionella pneumophila* | NCTC | 11230 | 2 |
| *Legionella pneumophila* | NCTC | 11232 | 3 |
| *Legionella pneumophila* | DSM | 7414 | 4 |
| *Legionella pneumophila* | DSM | 7415 | 5 |
| *Legionella pneumophila* | NCTC | 11417 | 5 |
| *Legionella pneumophila* | NCTC | 11287 | 6 |
| *Legionella pneumophila* | NCTC | 11984 | 7 |
| *Legionella pneumophila* | NCTC | 11985 | 8 |
| *Legionella pneumophila* | NCTC | 11986 | 9 |
| *Legionella pneumophila* | NCTC | 12000 | 10 |
| *Legionella pneumophila* | NCTC | 12179 | 11 |
| *Legionella pneumophila* | NCTC | 12180 | 12 |
| *Legionella pneumophila* | NCTC | 12181 | 13 |
| *Legionella pneumophila* | NCTC | 12174 | 14 |
| *DSM = The German Collection of Microorganisms; *NCTC = National Collection of Type Cultures | | | |

**Table 5(c) Other Legionella species tested in this study**

| **Strain** | **Source** |
|---|---|
| *Legionella anisa* | DSM 17627 |
| *Legionella birminghamensis* | DSM 19232 |
| *Legionella birminghamensis* | ATCC 700709 |
| *Fluoribacter bozemanae* (*Legionella bozemanii*) | DSM 16523 |
| *Fluoribacter bozemanae-2* | NCTC 11975 |
| *Legionella cincinnatiensis* | DSM 19233 |
| *Fluoribacter dumoffii* (*Legionella dumoffii)* | DSM 17625 |
| *Fluoribacter dumoffii* | ATCC 33343 |
| *Legionella erythra* | DSM 17644 |
| *Legionella feeleii*-1 | DSM 17645 |
| *Legionella feeleii*-2 | NCTC 11978 |
| *Flouribacter gormanii* (*Legionella gormanii*) | DSM 16641 |
| *Fluoribacter gormanii* | ATCC 43769 |
| *Legionella hackeliae*-1 | DSM 19214 |
| *Legionella hackeliae*-2 | NCTC 11980 |
| *Legionella jordanis* | DSM 19212 |
| *Legionella jordanis* | ATCC 700762 |
| *Legionella lansingensis* | DSM 19556 |
| *Legionella longbeachae*-1 | DSM 10572 |
| *Legionella maceachwerii* | DSM 10572 |
| *Tatlockia micdadei* (*Legionella micdadei*) | NCTC 11403 |
| *Legionella oakridgensis* | NCTC 11531 |
| *Legionella oakridgensis* | ATCC 700515 |
| *Legionella parisiensis* | DSM 19216 |
| *Legionella wadsworthii* | NCTC 11532 |
| *Legionella adelaidensis* | DSM 19888 |
| *Legionella brunensis* | DSM 19236 |
| *Legionella busanesis* | ATCC-BAA518 |
| *Legionella cherrii* | DSM 19213 |
| *Legionella donaldsonii* | ATCC BAA-693 |
| *Legionella fairfieldensis* | NCTC 12488 |
| *Legionella fallonii* | CCUG 43887 |
| *Legionella gratiana* | DSM 21233 |
| *Legionella gresilensis* | DSM 21218 |
| *Legionella israelensi* | DSM 19235 |
| *Legionella jamestowniensis* | DSM 19215 |
| *Legionella londoniensis* | DSM 21234 |
| *Legionella moravica* | DSM 19234 |
| *Legionella quinlivanii*-1 | NCTC 12433 |
| *Legionella rubrilicens* | DSM 11884 |
| *Legionella taurinsis* | CCUG 44901 |

| | |
|---|---|
| *DSM = The German Collection of Microorganisms; *ATCC = American Type Culture Collection; *NCTC = National Collection of Type Cultures; *CCUG=Culture Collection, University of Göteborg | |

### Multiplex 1

### Result scenario for the identification of L. pneumophila serogroup 1

Using multiplex 1; if the *ssrA* genus assay, the *Ipg0768* assay, the *smpB* assay, and the IAC diagnostics assays each generate a positive signal, an organism selected from: *L. pneumophila serogroup* 1, is present in the sample.

### Result scenario for the identification of L. pneumophila serogroup 2-16

Using multiplex 1; if the *ssrA* genus assay, the *smpB* assay, and the IAC diagnostics assay each generate a positive signal, but the *Ipg0768* assay generates a negative signal, an organism selected from: *L. pneumophila serogroup* 2-16, is present in the sample.

### Result scenario for the identification of Legionella other than L. pneumophila

Using multiplex 1; if the *ssrA* genus assay and the IAC diagnostics assay generate a positive signal, but the *smpB* assay and the *Ipg0768* assay generate a negative signal, an organism selected from: *Legionella* species other than *L. pneumophila,* is present in the sample.

### Result scenario for when no Legionella are present in a sample

Using multiplex 1, if the *ssrA* genus assay, the *Ipg0768* assay, and the *smpB* assay are not detected, but the IAC diagnostics assays generates a positive signal, no organism of the *Legionella* genus is present in the sample

### Result scenario for invalid result

Using multiplex 1, if no positive signal is observed for any diagnostics assay tested for, including the IAC diagnostics assay, the result is considered invalid and must be repeated.

### Conclusion

The multiplex real-time PCR assay described in this example is the first description of a real-time PCR diagnostics assay for the identification of the *Legionella* genus, *L. pneumophila* (2-16) and *L. pneumophila* serogroup 1 using the novel *Ipg0768* gene diagnostics targets. This multiplex real-time PCR diagnostics assay takes approximately one hour to perform after genomic DNA extraction and purification and has an analytical specificity of 100% and a sensitivity of less than 10 genome equivalents.

### References

Halse, T. A., Edwards, J., Cunningham, P. L., Wolfgang, W. J., Dumas, N. B., Escuyer, V. E. & Musser, K. A. (2010) Combined Real-Time PCR and rpoB Gene Pyrosequencing for Rapid Identification of Mycobacterium tuberculosis and Determination of Rifampin Resistance Directly in Clinical Specimens. Journal of Clinical Microbiology, 48 (4), 1182-8.
Scheler, O., Glynn, B., Parkel, S., Palta, P., Toome, K., Kaplinski, L., Remm, M., Maher, M. & Kurg, A. (2009) Fluorescent labeling of NASBA amplified tmRNA molecules for microarray applications. BMC Biotechnology, 9 (45), Available from: https://bmcbiotechnol.biomedcentral.com/articles/ 10.1186/1472-6750-9-45 [Accessed: 23^{rd} September 2016].

## Claims

1. A method for identifying the presence or quantity of Legionella pneumophila serogroup 1 in a sample, the method comprising the steps of:
(a) detecting the presence or quantity of at least part of the nucleic acid sequence of SEQ ID NO. 1 or an expression product thereof, and
(b) correlating the presence or quantity of the at least part of the nucleic acid sequence of SEQ ID NO. 1 or expression product thereof, to the presence or quantity of Legionella pneumophila serogroup 1 in the sample.

2. A method according to Claim 1, wherein the method further comprises the steps of:
(ai) detecting the presence or quantity of at least part of the nucleic acid sequence of the smpB gene or an expression product thereof; and correlating the presence or quantity of the at least part of the nucleic acid sequence of the smpB gene or expression product thereof to the presence or quantity of Legionella pneumophila in the sample.

3. A method according to Claim 1 or 2, wherein the method further comprises the steps of:
(aii) detecting the presence or quantity of at least part of the nucleic acid sequence of the ssrA gene or an expression product thereof; and correlating the presence or quantity of the at least part of the nucleic acid sequence of the ssrA gene or expression product thereof to the presence or quantity of Legionella genus in the sample.

4. The method according to any preceding claim, wherein the detecting step (a) comprises:
contacting the sample with at least one primer having a nucleic acid sequence defined by at least one of SEQ ID NO. 2, SEQ ID NO. 3 and the reverse complement each thereof, under conditions suitable for a polymerase chain reaction.

5. The method according to any preceding claim, wherein the detecting step (a) comprises:
contacting the sample with at least one probe having a nucleic acid sequence defined by at least one of SEQ ID NO. 4 and the reverse complement thereof, under conditions suitable for hybridisation.

6. The method according to any of Claims 2-5, wherein the at least part of the nucleic acid sequence of the smpB gene comprises at least part of the nucleic acid sequence defined by SEQ ID NO. 6.

7. The method according to any of Claims 2-6, wherein the detecting step (ai) comprises:
contacting the sample with at least one primer having a nucleic acid sequence defined by at least one of SEQ ID NO. 7, SEQ ID NO. 8, SEQ ID NO. 13, SEQ ID NO. 49, SEQ ID NO. 50, and the reverse complement each thereof, under conditions suitable for a polymerase chain reaction; and/or
contacting the sample with a probe having a nucleic acid sequence defined by at least one of SEQ ID NO. 9, SEQ ID NO. 51, SEQ ID NO. 52 and the reverse complement each thereof.

8. The method according to any of Claims 3-7, wherein the at least part of the nucleic acid sequence of the ssrA gene comprises at least part of the nucleic acid sequence defined by SEQ ID NO. 5.

9. The method according to any of Claims 3-8, wherein the detecting step (aii) comprises:
contacting the sample with at least one primer having a nucleic acid sequence defined by at least one of SEQ ID NO. 10, SEQ ID NO. 11, and the reverse complement each thereof, under conditions suitable for a polymerase chain reaction; and/or
contacting the sample with a probe having a nucleic acid sequence defined by SEQ ID NO. 12 or the reverse complement thereof.

10. The method according to any of Claims 2-9, wherein the method is a multiplex polymerase chain reaction method.

11. The method according to any preceding claim wherein the sample substantially comprises a water or environmental water sample.

12. An in vitro method for detecting Legionnaires disease comprising the method of any of Claims 1-11, wherein the presence of at least part of the nucleic acid sequence of SEQ ID NO. 1 or an expression product thereof, indicates the presence of Legionnaires disease.

## Patentansprüche

1. Verfahren zum Identifizieren der Gegenwart oder der Menge von Legionella pneumophila der Serogruppe 1 in einer Probe, wobei das Verfahren die folgenden Schritte umfasst:
(a) Detektieren der Gegenwart oder der Menge von mindestens einem Teil der Nukleinsäuresequenz von SEQ ID NO. 1 oder einem Expressionsprodukt davon, und
(b) Korrelieren der Gegenwart oder der Menge des mindestens einen Teils der Nukleinsäuresequenz von SEQ ID NO. 1 oder eines Expressionsprodukts davon mit der Gegenwart oder der Menge von Legionella pneumophila der Serogruppe 1 in der Probe.

2. Verfahren nach Anspruch 1, wobei das Verfahren ferner die folgenden Schritte umfasst:
(ai) Detektieren der Gegenwart oder der Menge von mindestens einem Teil der Nukleinsäuresequenz des smpB-Gens oder eines Expressionsprodukts davon; und Korrelieren der Gegenwart oder der Menge des mindestens einen Teils der Nukleinsäuresequenz des smpB-Gens oder des Expressionsprodukts davon mit der Gegenwart oder der Menge von Legionella pneumophila in der Probe.

3. Verfahren nach Anspruch 1 oder 2, wobei das Verfahren ferner die folgenden Schritte umfasst:
(aii) Detektieren der Gegenwart oder der Menge von mindestens einem Teil der Nukleinsäuresequenz des ssrA-Gens oder eines Expressionsprodukts davon; und Korrelieren der Gegenwart oder der Menge des mindestens einen Teils der Nukleinsäuresequenz des ssrA-Gens oder des Expressionsprodukts davon mit der Gegenwart oder der Menge einer Legionellen-Gattung in der Probe.

4. Verfahren nach einem der vorhergehenden Ansprüche, wobei der Schritt des Detektierens (a) Folgendes umfasst:
Inkontaktbringen der Probe mit mindestens einem Primer mit einer Nukleinsäuresequenz, die durch mindestens eines von SEQ ID NO. 2, SEQ ID NO. 3 und dem jeweiligen reversen Komplement davon definiert ist, unter Bedingungen, die für eine Polymerase-Kettenreaktion geeignet sind.

5. Verfahren nach einem der vorhergehenden Ansprüche, wobei der Schritt des Detektierens (a) Folgendes umfasst:
Inkontaktbringen der Probe mit mindestens einer Sonde mit einer Nukleinsäuresequenz, die durch mindestens eines von SEQ ID NO. 4 und dem reversen Komplement davon definiert ist, unter Bedingungen, die für eine Hybridisierung geeignet sind.

6. Verfahren nach einem der Ansprüche 2-5, wobei der mindestens eine Teil der Nukleinsäuresequenz des smpB-Gens mindestens einen Teil der Nukleinsäuresequenz, die durch SEQ ID NO. 6 definiert ist, umfasst.

7. Verfahren nach einem der Ansprüche 2-6, wobei der Schritt des Detektierens (ai) Folgendes umfasst:
Inkontaktbringen der Probe mit mindestens einem Primer mit einer Nukleinsäuresequenz, die durch mindestens eines von SEQ ID NO. 7, SEQ ID NO. 8, SEQ ID NO. 13, SEQ ID NO. 49, SEQ ID NO. 50 und dem jeweiligen reversen Komplement davon definiert ist, unter Bedingungen, die für eine Polymerase-Kettenreaktion geeignet sind; und/oder
Inkontaktbringen der Probe mit einer Sonde mit einer Nukleinsäuresequenz, die durch mindestens eines von SEQ ID NO. 9, SEQ ID NO. 51, SEQ ID NO. 52 und dem jeweiligen reversen Komplement davon definiert ist.

8. Verfahren nach einem der Ansprüche 3-7, wobei der mindestens eine Teil der Nukleinsäuresequenz des ssrA-Gens mindestens einen Teil der Nukleinsäuresequenz, die durch SEQ ID NO. 5 definiert ist, umfasst.

9. Verfahren nach einem der Ansprüche 3-8, wobei der Schritt des Detektierens (aii) Folgendes umfasst:
Inkontaktbringen der Probe mit mindestens einem Primer mit einer Nukleinsäuresequenz, die durch mindestens eines von SEQ ID NO. 10, SEQ ID NO. 11 und dem jeweiligen reversen Komplement davon definiert ist, unter Bedingungen, die für eine Polymerase-Kettenreaktion geeignet sind; und/oder Inkontaktbringen der Probe mit einer Sonde mit einer Nukleinsäuresequenz, die durch SEQ ID NO. 12 oder das reverse Komplement davon definiert ist.

10. Verfahren nach einem der Ansprüche 2-9, wobei das Verfahren ein Multiplex-Polymerase-Kettenreaktionsverfahren ist.

11. Verfahren nach einem der vorhergehenden Ansprüche, wobei die Probe im Wesentlichen eine Wasser- oder Umgebungswasserprobe umfasst.

12. In-vitro-Verfahren zum Detektieren der Legionärskrankheit, umfassend das Verfahren nach einem der Ansprüche 1-11, wobei die Gegenwart von mindestens einem Teil der Nukleinsäuresequenz von SEQ ID NO. 1 oder einem Expressionsprodukt davon die Gegenwart der Legionärskrankheit angibt.

## Revendications

1. Procédé d'identification de la présence ou de la quantité de Legionella pneumophila sérogroupe 1 dans un échantillon, le procédé comprenant les étapes de :
(a) détection de la présence ou de la quantité d'au moins une partie de la séquence d'acide nucléique de SEQ ID NO. 1 ou un produit d'expression de celle-ci, et
(b) corrélation de la présence ou de la quantité de l'au moins une partie de la séquence d'acide nucléique de SEQ ID NO. 1 ou du produit d'expression de celle-ci, à la présence ou à la quantité de Legionella pneumophila sérogroupe 1 dans l'échantillon.

2. Procédé selon la revendication 1, dans lequel le procédé comprend en outre les étapes de :
(ai) détection de la présence ou de la quantité d'au moins une partie de la séquence d'acide nucléique du gène smpB ou d'un produit d'expression de celui-ci ; et corrélation de la présence ou de la quantité de l'au moins une partie de la séquence d'acide nucléique du gène smpB ou du produit d'expression de celui-ci à la présence ou à la quantité de Legionella pneumophila dans l'échantillon.

3. Procédé selon la revendication 1 ou 2, dans lequel le procédé comprend en outre les étapes de :
(aii) détection de la présence ou de la quantité d'au moins une partie de la séquence d'acide nucléique du gène ssrA ou d'un produit d'expression de celui-ci ; et corrélation de la présence ou de la quantité de l'au moins une partie de la séquence d'acide nucléique du gène ssrA ou du produit d'expression de celui-ci à la présence ou à la quantité du genre Legionella dans l'échantillon.

4. Procédé selon une quelconque revendication précédente, dans lequel l'étape de détection (a) comprend :
la mise en contact de l'échantillon avec au moins une amorce ayant une séquence d'acide nucléique définie par au moins l'une des SEQ ID NO. 2, SEQ ID NO. 3 et le complément inverse de chacune de celles-ci, dans des conditions adaptées à une réaction en chaîne par polymérase.

5. Procédé selon une quelconque revendication précédente, dans lequel l'étape de détection (a) comprend :
la mise en contact de l'échantillon avec au moins une sonde ayant une séquence d'acide nucléique définie par au moins l'une des SEQ ID NO. 4 et le complément inverse de celle-ci, dans des conditions adaptées à l'hybridation.

6. Procédé selon l'une quelconque des revendications 2 à 5, dans lequel l'au moins une partie de la séquence d'acide nucléique du gène smpB comprend au moins une partie de la séquence d'acide nucléique définie par SEQ ID NO. 6.

7. Procédé selon l'une quelconque des revendications 2 à 6, dans lequel l'étape de détection (ai) comprend :
la mise en contact de l'échantillon avec au moins une amorce ayant une séquence d'acide nucléique définie par au moins l'une de SEQ ID NO. 7, SEQ ID NO. 8, SEQ ID NO. 13, SEQ ID NO. 49, SEQ ID NO. 50, et le complément inverse de chacune de celles-ci, dans des conditions adaptées à une réaction en chaîne par polymérase ; et/ou
la mise en contact de l'échantillon avec une sonde ayant une séquence d'acide nucléique définie par au moins l'une de SEQ ID NO. 9, SEQ ID NO. 51, SEQ ID NO. 52 et le complément inverse de chacune de celles-ci.

8. Procédé selon l'une quelconque des revendications 3 à 7, dans lequel l'au moins une partie de la séquence d'acide nucléique du gène ssrA comprend au moins une partie de la séquence d'acide nucléique définie par SEQ ID NO. 5.

9. Procédé selon l'une quelconque des revendications 3 à 8, dans lequel l'étape de détection (aii) comprend :
la mise en contact de l'échantillon avec au moins une amorce ayant une séquence d'acide nucléique définie par au moins l'une de SEQ ID NO. 10, SEQ ID NO. 11 et le complément inverse de chacune de celles-ci, dans des conditions adaptées à une réaction en chaîne par polymérase ; et/ou
la mise en contact de l'échantillon avec une sonde ayant une séquence d'acide nucléique définie par SEQ ID NO. 12 ou le complément inverse de celle-ci.

10. Procédé selon l'une quelconque des revendications 2 à 9, dans lequel le procédé est un procédé de réaction en chaîne par polymérase multiplex.

11. Procédé selon une quelconque revendication précédente, dans lequel l'échantillon comprend essentiellement un échantillon d'eau ou d'eau environnementale.

12. Procédé in vitro pour détecter la maladie du légionnaire comprenant le procédé selon l'une quelconque des revendications 1 à 11, dans lequel la présence d'au moins une partie de la séquence d'acide nucléique de SEQ ID NO. 1 ou un produit d'expression de celle-ci, indique la présence de la maladie du légionnaire.


### SEQUENCE LISTING

<110> National University of Ireland, Galway
<120> A method for the detection of Legionella
<130> P118740PC00
<160> 52
<170> PatentIn version 3.5
<210> 1
   <211> 1092
   <212> DNA
   <213> Legionella pneumophila
<400> 1
   ctaacaatca tttttttgct taatatccga ccatttaatg ggttcaccta tagatacctt      60
   gttttgtaat tcccagccat ttaccagatc ccagtattgg gtcgggactc ccttacaagg     120
   aaaggcaaag gttgtattat caaggctaat tagagagcca ggttgaagtt tttgctttgc     180
   aaccaaacaa gttctttgag agctgccaag tactcctttt ataggagctt ctagatctcg     240
   catcggttga cctaaagctt gccaacaatt gtaaattgtt tcaagagtct tggctaattg     300
   ctcaatcccc atagtatgtg atatgtcctg atcaatatct tctggataga gatgaactcc     360
   tttttccagg attgaagctc ccaatgcaat agacatatac aacatttcaa ttccattata     420
   atggtcagat aaaccaactg gtaatgaaaa acattggttt aaagttcgta acattcttag     480
   gttatgtgct tctggcaaag caggatgtcc atcaggacta tgttcaataa taattttttc     540
   acatccggca ctttttgctg tttcaactgc tttgcatata tcagttaaag aagatcttcc     600
   agtgtcaata atgagcggta atcctgtttt tgccagatga cgaattaaag gaacatggac     660
   aatgttcgat gaagaggttt tgagtgcgca ggcaccctgc tttatcgcaa aatcaatagt     720
   gtccaattca taaatcgaaa ctatgaaggg taaattggca ttctttaata gtgaaaatag     780
   catactataa tgttctaaag gcagtacttt tctctcaatt aatgagcggt agttttcctt     840
   gcggacctca ccggatttac tggtataggt ttcttcataa tcaacaggta ggcaaatgtt     900
   tgggttattt aaaatttctg tttttagtat aagaggttgg tatggtactt tatccctgat     960
   ggttataatt ttgtgtagca attgttcagc tatgccaaga tcctgattaa aaaaagtacc    1020
   tatttctgct aaaaaaatag gaggatgatt ttcttcaaca gagaatggac caattgtaaa    1080
   aggctgactc at                                                        1092
<210> 2
   <211> 19
   <212> DNA
   <213> Unknown
<220>
   <223> Synthetic oligonucleotide
<400> 2
   agctcccaat gcaatagac 19
<210> 3
   <211> 19
   <212> DNA
   <213> Unknown
<220>
   <223> Synthetic oligonucleotide
<400> 3
   gcaaagcagt tgaaacagc 19
<210> 4
   <211> 25
   <212> DNA
   <213> Unknown
<220>
   <223> Synthetic oligonucleotide
<400> 4
   ccaactggta atgaaaaaca ttggt 25
<210> 5
   <211> 405
   <212> DNA
   <213> Legionella pneumophila
<400> 5
   ggggcgacct ggcttcgacg tgggttgcaa aaccggaagt gcatgccgag aaggagatct    60
   ctcgtaaata agactcaatt aaatataaat gcaaacgatg aaaactttgc tggtggggaa   120
   gctatcgctg cctaataagc actttagtta aaccatcact gtgtactggc caataaaccc   180
   agtatcccgt tcgaccgagc ccgcttatcg gtatcgaatc aacggtcata agagataagc   240
   tagcgtccta atctatcccg ggttatggcg cgaaactcag ggaatcgctg tgtatcatcc   300
   tgcccgtcgg aggagccaca gttaaattca aaagacaagg ctatgcatgt agagctaaag   360
   gcagaggact tgcggacgcg ggttcgattc ccgccgcctc cacca                   405
<210> 6
   <211> 471
   <212> DNA
   <213> Legionella pneumophila
<400> 6
   atgactacca aaaaacaacc agattccacc atagccttga atagaaaagc tggttttgat    60
   tactttattg aagatcaata cgaagcaggc ctggttttgg aaggctggga agtaaaaagt   120
   ctgcgtgctg gaaaaatcaa tttgtcggat tcacacgtga taatcaaata cggtgaggca   180
   ttcctattgg gcgcccaaat acagcctctt cccaccgcat ccactcattt tattcctgat 240
   ccgatcagga cgcgcaagct attgatgaat aaaaaagaat taaaccatct cataggaagt 300
   gttgaaaggc aaggctatac catagtccct ctttctttgt attggaaaaa aaataaaatt 360
   aaaataaaaa ttgctctggc taaaggaaaa aaagagcatg acaaaagaga cacgatcaaa 420
   gacagagaat ggcaaaggga tcgttcaaga ataatgaaaa agaacacttg a 471
<210> 7
   <211> 21
   <212> DNA
   <213> Unknown
<220>
   <223> Synthetic oligonucleotide
<400> 7
   cacgtgataa tmaaatacgg t 21
<210> 8
   <211> 19
   <212> DNA
   <213> Unknown
<220>
   <223> Synthetic oligonucleotide
<400> 8
   ttcatcaata gcttgcgyg 19
<210> 9
   <211> 26
   <212> DNA
   <213> Unknown
<220>
   <223> Synthetic oligonucleotide
<400> 9
   cygcatccac tcattttatt cctgat 26
<210> 10
   <211> 14
   <212> DNA
   <213> Unknown
<220>
   <223> Synthetic oligonucleotide
<400> 10
   ggcgacctgg cttc 14
<210> 11
   <211> 21
   <212> DNA
   <213> Unknown
<220>
   <223> Synthetic oligonucleotide
<400> 11
   tatgaccgtt gattcgatac c 21
<210> 12
   <211> 17
   <212> DNA
   <213> Unknown
<220>
   <223> Synthetic oligonucleotide
<400> 12
   acgtgggttg craaacc 17
<210> 13
   <211> 19
   <212> DNA
   <213> Unknown
<220>
   <223> Synthetic oligonucleotide
<400> 13
   ttcatcaaca gcttgcgtg 19
<210> 14
   <211> 18
   <212> DNA
   <213> Unknown
<220>
   <223> Synthetic oligonucleotide
<400> 14
   gatcaatacg aagcaggc 18
<210> 15
   <211> 20
   <212> DNA
   <213> Unknown
<220>
   <223> Synthetic oligonucleotide
<400> 15
   gccattctct gtctttgatc 20
<210> 16
   <211> 22
   <212> DNA
   <213> Unknown
<220>
   <223> Synthetic oligonucleotide
<400> 16
   ccaagtaggg tttctaacaa tc 22
<210> 17
   <211> 21
   <212> DNA
   <213> Unknown
<220>
   <223> Synthetic oligonucleotide
<400> 17
   gagtcagcct tttacaattg g 21
<210> 18
   <211> 19
   <212> DNA
   <213> Unknown
<220>
   <223> Synthetic oligonucleotide
<400> 18
   atgccagtca gcataagga 19
<210> 19
   <211> 21
   <212> DNA
   <213> Unknown
<220>
   <223> Synthetic oligonucleotide
<400> 19
   cagacctctg gtaggatgta c 21
<210> 20
   <211> 20
   <212> DNA
   <213> Unknown
<220>
   <223> Synthetic oligonucleotide
<400> 20
   tcggcactac cgacacgaac 20
<210> 21
   <211> 1091
   <212> DNA
   <213> Legionella pneumophila
<400> 21
   taacaatcat ttttttgctt aatatccgac catttaatgg gttcacctat agataccttg      60
   ttttgtaatt cccagccatt taccagatcc cagtattggg taggaactcc cttacaagga     120
   aaggcaaagg ttgtattatc aaggctaatt agagagccgg gttgaagttt ttgctttgca      180
   accaaacaag ttctttgaga gctgccaagt actcctttta taggggtttc tagatctcgc      240
   attggttgac ctaaagcttg ccaacaattg taaattgttt caagagtctt ggctaattga      300
   tcaatcccca tagtatgtga tatgtcctga tcgatatctt ctggatagag atgaactcct      360
   ttttccagga ttgaagctcc caatgcaata gacatataca acatttcaat tccattataa      420
   tggtcagata aaccaactgg taatgaaaaa cattggttta aagttcgtaa cattcttagg      480
   ttatgtgctt ctggcaaagc aggatgtcca tcagggctat gttcaataat aattttttca       540
   catccggcac tttttgctgt ttcaactgct ttgcatatat cagttaagga agatcttcca      600
   gtgtcaataa tgagcggtaa tcctgttttt gccagatgac gaattaaagg aatatggaca      660
   atgttcgatg aagaggtttt gagtgcgcag gccccctgct ttatcgcaaa atcaatagtg      720
   tccaattcat aaatcgaaac tatgaagggt aaattggcat tctttaatag tgaaaatagc      780
   atactataat gttctaaagg cagtactttt ctctcaatta atgagcggta gttttccttg      840
   cgaacctcac ctgatttact ggtataggtt tcttcataat caacaggtag gcaaatgttt      900
   gggttattta aaatttctgt ttttagtata agaggttggt atggtacttt atccctgatg      960
   gttataattt tgtgtagcaa ttgttcagct atgccaagat cctgattaaa aaaagtacct     1020
   atttctgcta aaaaaatagg aggatgattt tcttcaacag agaatggacc aattgtaaaa     1080
   ggctgactca t                                                          1091
<210> 22
   <211> 1091
   <212> DNA
   <213> Legionella pneumophila
<400> 22
   taacaatcat ttttttgctt aatatccgac catttaatgg gttcacctat agataccttg      60
   ttttgtaatt cccagccatt taccagatcc cagtattggg taggaactcc cttacaagga     120
   aaggcaaagg ttgtattatc aaggctaatt agagagccgg gttgaagttt ttgctttgca     180
   accaaacaag ttctttgaga gctgccaagt actcctttta taggggtttc tagatctcgc     240
   attggttgac ctaaagcttg ccaacaattg taaattgttt caagagtctt ggctaattga     300
   tcaatcccca tagtatgtga tatgtcctga tcgatatctt ctggatagag atgaactcct     360
   ttttccagga ttgaagctcc caatgcaata gacatataca acatttcaat tccattataa     420
   tggtcagata aaccaactgg taatgaaaaa cattggttta aagttcgtaa cattcttagg     480
   ttatgtgctt ctggcaaagc aggatgtcca tcagggctat gttcaataat aattttttca     540
   catccggcac tttttgctgt ttcaactgct ttgcatatat cagttaagga agatcttcca     600
   gtgtcaataa tgagcggtaa tcctgttttt gccagatgac gaattaaagg aatatggaca     660
   atgttcgatg aagaggtttt gagtgcgcag gccccctgct ttatcgcaaa atcaatagtg     720
   tccaattcat aaatcgaaac tatgaagggt aaattggcat tctttaatag tgaaaatagc     780
   atactataat gttctaaagg cagtactttt ctctcaatta atgagcggta gttttccttg     840
   cgaacctcac ctgatttact ggtataggtt tcttcataat caacaggtag gcaaatgttt     900
   gggttattta aaatttctgt ttttagcata agaggttggt atggtacttt atccctgatg     960
   gttataattt tgtgtagcaa ttgttcagct atgccaagat cctgattaaa aaaagtacct    1020
   atttctgcta aaaaaatagg aggatgattt tcttcaacag agaatggacc aattgtaaaa    1080
   ggctgactca t                                                         1091
<210> 23
   <211> 1091
   <212> DNA
   <213> Legionella pneumophila
<400> 23
   ctaacaatca tttttttgct taatatccga ccatttaatg ggttcaccta tagatacctt       60
   gttttgtaat tcccacccat ttaccagatc ccagtattgg gtcggtactc ccttgcaagg      120
   aaaggcaaag gttgtattat caaggctaat tagagtacca ggatgaagtt cttgttttgc      180
   aaccaaacaa gttctttgag agctaccaag tactcctttt atgggagctt ctagatctcg      240
   catcggttga cctaaagctt gccaacaatt gtaaattgtt taagagtctt ggctaattgc      300
   tcaatcccca tagtatgtga tatgtcctga tcaatatctt ctggatagag atgaactcct      360
   ttttccagga ttgaagctcc caatgcaata gacatataca acatttcaat tccattataa      420
   tggtcagata aaccaactgg taatgaaaaa cattggttta aagttcgtaa cattcttagg      480
   ttatgtgctt ctggcaaagc aggatgtcca tcaggactat gttcaataat aattttttca      540
   catccggcac tttttgctgt ttcaactgct ttgcatatat cagttaaaga agatcttcca      600
   gtgtcaataa tgagcggtaa tcctgttttt gccagatgac gaattaaagg aacatggaca      660
   atgttcgatg aagaggtttt gagtgcgcag gcaccctgct ttatcgcaaa atcaatagtg      720
   tccaattcat aaatcgaaac tatgaagggt aaattggcat tctttaatag tgaaaatagc      780
   atactataat gttctaaagg cagtactttt ctctcaatta atgagcggta gttttccttg      840
   cggacctcac ctgatttact ggtataggtt tcttcataat caacaggtag gcaaatgttt      900
   gggttattta aaatttctgt ttttagtata agaggttgat acggtacttt atccctgatg      960
   gttataattt tgtgtagtaa ttgttcggct acaccaagat cctgattaaa aaaagtacca     1020
   atttctgcca aaaaaatagg aggatgattt tctccaatag agaatggacc aattgtaaaa     1080
   ggctgactca t                                                          1091
<210> 24
   <211> 1074
   <212> DNA
   <213> Legionella pneumophila
<400> 24
   taacaatcat ttttttgctt aatatccgac catttaatgg gttcacctat agataccttg        60
   ttttgtaatt cccagccatt taccagatcc cagtattggg taggaactcc cttacaagga       120
   aaggcaaagg ttgtattatc aaggctaatt agagagccgg gttgaagttt ttgctttgca       180
   accaaacaag ttctttgaga gctgccaagt actcctttta taggggtttc tagatctcgc       240
   attggttgac ctaaagcttg ccaacaattg taaattgttt caagagtctt ggctaattga       300
   tcaatcccca tagtatgtga tatgtcctga tcgatatctt ctggatagag atgaactcct       360
   ttttccagga ttgaagctcc caatgcaata gacatataca acatttcaat tccattataa       420
   tggtcagata aaccaactgg taatgaaaaa cattggttta aagttcgtaa cattcttagg       480
   ttatgtgctt ctggcaaagc aggatgtcca tcaggactat gttcaataat aattttttca        540
   catccggcac tttttgctgt ttcaactgct ttgcatatat cagttaagga agatcttcca       600
   gtgtcaataa tgagcggtaa tcctgttttt gccagatgac gaattaaagg aatatggaca       660
   atgttcgatg aagaggtttt gagtgcgcag gccccctgct ttatcgcaaa atcaatagtg       720
   tccaattcat aaatcgaaac tatgaagggt aaattggcat tctttaatag tgaaaatagc       780
   atactataat gttctaaagg cagtactttt ctctcaatta atgagcggta gttttccttg       840
   cgaacctcac ctgatttact ggtataggtt tcttcataat caacaggtag gcaaatgttt       900
   gggttattta aaatttctgt ttttagtata agaggttgat acggtatttt atccctgata       960
   cttataattt tgtgtagcaa ttgttcagct atgccaagat cctgattaaa aaaagtacct      1020
   atttctgcta aaaaaatagg aggatgattt tctccaatag agtaggggcc aatt            1074
<210> 25
   <211> 1074
   <212> DNA
   <213> Legionella pneumophila
<400> 25
   taacaatcat ttttttgctt aatatccgac catttaatgg gttcacctat agataccttg        60
   ttttgtaatt cccagccatt taccagatcc cagtattggg taggaactcc cttacaagga       120
   aaggcaaagg ttgtattatc aaggctaatt agagagccgg gttgaagttt ttgctttgca       180
   accaaacaag ttctttgaga gctgccaagt actcctttta taggggtttc tagatctcgc       240
   attggttgac ctaaagcttg ccaacaattg taaattgttt caagagtctt ggctaattga       300
   tcaatcccca tagtatgtga tatgtcctga tcgatatctt ctggatagag atgaactcct       360
   ttttccagga ttgaagctcc caatgcaata gacatataca acatttcaat tccattataa       420
   tggtcagata aaccaactgg taatgaaaaa cattggttta aagttcgtaa cattcttagg       480
   ttatgtgctt ctggcaaagc aggatgtcca tcaggactat gttcaataat aattttttca       540
   catccggcac tttttgctgt ttcaactgct ttgcatatat cagttaagga agatcttcca      600
   gtgtcaataa tgagcggtaa tcctgttttt gccagatgac gaattaaagg aatatggaca      660
   atgttcgatg aagaggtttt gagtgcgcag gccccctgct ttatcgcaaa atcaatagtg      720
   tccaattcat aaatcgaaac tatgaagggt aaattggcat tctttaatag tgaaaatagc      780
   atactataat gttctaaagg cagtactttt ctctcaatta atgagcggta gttttccttg      840
   cgaacctcac ctgatttact ggtataggtt tcttcataat caacaggtag gcaaatgttt      900
   gggttattta aaatttctgt ttttagtata agaggttgat acggtatttt atccctgata      960
   cttataattt tgtgtagcaa ttgttcagct atgccaagat cctgattaaa aaaagtacct     1020
   atttctgcta aaaaaatagg aggatgattt tctccaatag agtaggggcc aatt           1074
<210> 26
   <211> 1074
   <212> DNA
   <213> Legionella pneumophila
<400> 26
   taacaatcat ttttttgctt aatatccgac catttaatgg gttcacctat agataccttg      60
   ttttgtaatt cccagccatt taccagatcc cagtattggg taggaactcc cttacaagga     120
   aaggcaaagg ttgtattatc aaggctaatt agagagccgg gttgaagttt ttgctttgca     180
   accaaacaag ttctttgaga gctgccaagt actcctttta taggggtttc tagatctcgc     240
   attggttgac ctaaagcttg ccaacaattg taaattgttt caagagtctt ggctaattga     300
   tcaatcccca tagtatgtga tatgtcctga tcgatatctt ctggatagag atgaactcct     360
   ttttccagga ttgaagctcc caatgcaata gacatataca acatttcaat tccattataa     420
   tggtcagata aaccaactgg taatgaaaaa cattggttta aagttcgtaa cattcttagg     480
   ttatgtgctt ctggcaaagc aggatgtcca tcaggactat gttcaataat aattttttca    540
   catccggcac tttttgctgt ttcaactgct ttgcatatat cagttaagga agatcttcca     600
   gtgtcaataa tgagcggtaa tcctgttttt gccagatgac gaattaaagg aatatggaca     660
   atgttcgatg aagaggtttt gagtgcgcag gccccctgct ttatcgcaaa atcaatagtg    720
   tccaattcat aaatcgaaac tatgaagggt aaattggcat tctttaatag tgaaaatagc     780
   atactataat gttctaaagg cagtactttt ctctcaatta atgagcggta gttttccttg     840
   cgaacctcac ctgatttact ggtataggtt tcttcataat caacaggtag gcaaatgttt     900
   gggttattta aaatttctgt ttttagtata agaggttgat acggtatttt atccctgata     960
   cttataattt tgtgtagcaa ttgttcagct atgccaagat cctgattaaa aaaagtacct    1020
   atttctgcta aaaaaatagg aggatgattt tctccaatag agtaggggcc aatt          1074
<210> 27
   <211> 1074
   <212> DNA
   <213> Legionella pneumophila
<400> 27
   taacaatcat ttttttgctt aatatccgac catttaatgg gttcacctat agataccttg 60
   ttttgtaatt cccagccatt taccagatcc cagtattggg taggaactcc cttacaagga 120
   aaggcaaagg ttgtattatc aaggctaatt agagagccgg gttgaagttt ttgctttgca 180
   accaaacaag ttctttgaga gctgccaagt actcctttta taggggtttc tagatctcgc 240
   attggttgac ctaaagcttg ccaacaattg taaattgttt caagagtctt ggctaattga 300
   tcaatcccca tagtatgtga tatgtcctga tcgatatctt ctggatagag atgaactcct 360
   ttttccagga ttgaagctcc caatgcaata gacatataca acatttcaat tccattataa 420
   tggtcagata aaccaactgg taatgaaaaa cattggttta aagttcgtaa cattcttagg 480
   ttatgtgctt ctggcaaagc aggatgtcca tcaggactat gttcaataat aattttttca 540
   catccggcac tttttgctgt ttcaactgct ttgcatatat cagttaagga agatcttcca 600
   gtgtcaataa tgagcggtaa tcctgttttt gccagatgac gaattaaagg aatatggaca 660
   atgttcgatg aagaggtttt gagtgcgcag gccccctgct ttatcgcaaa atcaatagtg 720
   tccaattcat aaatcgaaac tatgaagggt aaattggcat tctttaatag tgaaaatagc 780
   atactataat gttctaaagg cagtactttt ctctcaatta atgagcggta gttttccttg 840
   cgaacctcac ctgatttact ggtataggtt tcttcataat caacaggtag gcaaatgttt 900
   gggttattta aaatttctgt ttttagtata agaggttgat acggtatttt atccctgata 960
   cttataattt tgtgtagcaa ttgttcagct atgccaagat cctgattaaa aaaagtacct 1020
   atttctgcta aaaaaatagg aggatgattt tctccaatag agtaggggcc aatt 1074
<210> 28
   <211> 1074
   <212> DNA
   <213> Legionella pneumophila
<400> 28
   taacaatcat ttttttgctt aatatccgac catttaatgg gttcacctat agataccttg 60
   ttttgtaatt cccagccatt taccagatcc cagtattggg taggaactcc cttacaagga 120
   aaggcaaagg ttgtattatc aaggctaatt agagagccgg gttgaagttt ttgctttgca 180
   accaaacaag ttctttgaga gctgccaagt actcctttta taggggtttc tagatctcgc 240
   attggttgac ctaaagcttg ccaacaattg taaattgttt caagagtctt ggctaattga 300
   tcaatcccca tagtatgtga tatgtcctga tcgatatctt ctggatagag atgaactcct 360
   ttttccagga ttgaagctcc caatgcaata gacatataca acatttcaat tccattataa      420
   tggtcagata aaccaactgg taatgaaaaa cattggttta aagttcgtaa cattcttagg      480
   ttatgtgctt ctggcaaagc aggatgtcca tcaggactat gttcaataat aattttttca       540
   catccggcac tttttgctgt ttcaactgct ttgcatatat cagttaagga agatcttcca      600
   gtgtcaataa tgagcggtaa tcctgttttt gccagatgac gaattaaagg aatatggaca      660
   atgttcgatg aagaggtttt gagtgcgcag gccccctgct ttatcgcaaa atcaatagtg      720
   tccaattcat aaatcgaaac tatgaagggt aaattggcat tctttaatag tgaaaatagc      780
   atactataat gttctaaagg cagtactttt ctctcaatta atgagcggta gttttccttg      840
   cgaacctcac ctgatttact ggtataggtt tcttcataat caacaggtag gcaaatgttt      900
   gggttattta aaatttctgt ttttagtata agaggttgat acggtatttt atccctgata      960
   cttataattt tgtgtagcaa ttgttcagct atgccaagat cctgattaaa aaaagtacct     1020
   atttctgcta aaaaaatagg aggatgattt tctccaatag agtaggggcc aatt           1074
<210> 29
   <211> 1074
   <212> DNA
   <213> Legionella pneumophila
<400> 29
   taacaatcat ttttttgctt aatatccgac catttaatgg gttcacctat agataccttg      60
   ttttgtaatt cccagccatt taccagatcc cagtattggg taggaactcc cttacaagga     120
   aaggcaaagg ttgtattatc aaggctaatt agagagccgg gttgaagttt ttgctttgca     180
   accaaacaag ttctttgaga gctgccaagt actcctttta taggggtttc tagatctcgc     240
   attggttgac ctaaagcttg ccaacaattg taaattgttt caagagtctt ggctaattga     300
   tcaatcccca tagtatgtga tatgtcctga tcgatatctt ctggatagag atgaactcct     360
   ttttccagga ttgaagctcc caatgcaata gacatataca acatttcaat tccattataa     420
   tggtcagata aaccaactgg taatgaaaaa cattggttta aagttcgtaa cattcttagg     480
   ttatgtgctt ctggcaaagc aggatgtcca tcaggactat gttcaataat aattttttca      540
   catccggcac tttttgctgt ttcaactgct ttgcatatat cagttaagga agatcttcca     600
   gtgtcaataa tgagcggtaa tcctgttttt gccagatgac gaattaaagg aatatggaca     660
   atgttcgatg aagaggtttt gagtgcgcag gccccctgct ttatcgcaaa atcaatagtg     720
   tccaattcat aaatcgaaac tatgaagggt aaattggcat tctttaatag tgaaaatagc     780
   atactataat gttctaaagg cagtactttt ctctcaatta atgagcggta gttttccttg     840
   cgaacctcac ctgatttact ggtataggtt tcttcataat caacaggtag gcaaatgttt     900
   gggttattta aaatttctgt ttttagtata agaggttgat acggtatttt atccctgata     960
   cttataattt tgtgtagcaa ttgttcagct atgccaagat cctgattaaa aaaagtacct    1020
   atttctgcta aaaaaatagg aggatgattt tctccaatag agtaggggcc aatt          1074
<210> 30
   <211> 1074
   <212> DNA
   <213> Legionella pneumophila
<400> 30
   taacaatcat ttttttgctt aatatccgac catttaatgg gttcacctat agataccttg      60
   ttttgtaatt cccagccatt taccagatcc cagtattggg taggaactcc cttacaagga     120
   aaggcaaagg ttgtattatc aaggctaatt agagagccgg gttgaagttt ttgctttgca     180
   accaaacaag ttctttgaga gctgccaagt actcctttta taggggtttc tagatctcgc     240
   attggttgac ctaaagcttg ccaacaattg taaattgttt caagagtctt ggctaattga     300
   tcaatcccca tagtatgtga tatgtcctga tcgatatctt ctggatagag atgaactcct     360
   ttttccagga ttgaagctcc caatgcaata gacatataca acatttcaat tccattataa     420
   tggtcagata aaccaactgg taatgaaaaa cattggttta aagttcgtaa cattcttagg     480
   ttatgtgctt ctggcaaagc aggatgtcca tcaggactat gttcaataat aattttttca     540
   catccggcac tttttgctgt ttcaactgct ttgcatatat cagttaagga agatcttcca     600
   gtgtcaataa tgagcggtaa tcctgttttt gccagatgac gaattaaagg aatatggaca     660
   atgttcgatg aagaggtttt gagtgcgcag gccccctgct ttatcgcaaa atcaatagtg     720
   tccaattcat aaatcgaaac tatgaagggt aaattggcat tctttaatag tgaaaatagc     780
   atactataat gttctaaagg cagtactttt ctctcaatta atgagcggta gttttccttg     840
   cgaacctcac ctgatttact ggtataggtt tcttcataat caacaggtag gcaaatgttt     900
   gggttattta aaatttctgt ttttagtata agaggttgat acggtatttt atccctgata     960
   cttataattt tgtgtagcaa ttgttcagct atgccaagat cctgattaaa aaaagtacct    1020
   atttctgcta aaaaaatagg aggatgattt tctccaatag agtaggggcc aatt          1074
<210> 31
   <211> 1092
   <212> DNA
   <213> Legionella pneumophila
<400> 31
   ctaacaatca tttttttgct taatatctga ccatttaatg ggctcaccta tagatacctt      60
   gttttgtagt tcccagccat ttaccagatc ccagtattgg gtaggaactc ccttacaagg     120
   aaaggcaaag gttgtattat caaggctaat tagagagccg ggttgaagct tttgctttgc     180
   aaccaaacaa gttctttgag agctgccaag tactcctttt ataggggttt ctagatctcg     240
   catcggttga cctaaagctt gccaacaatt gtaaattgtt tcaagagtct tggctaattg     300
   atcaatcccc atagtatgtg atatgtcctg atcaatatct tctggataga gatgaacccc      360
   tttttccagg attgaagctc ccaatgcaat agacatatac aacatttcaa ttccattata      420
   atggtcagat aatccaactg gtaatgaaaa acattggttt aaagttcgta acattcttaa      480
   gttatgtgct tctggcaaag caggatgtcc atcaggacta tgttcaataa taattttttc      540
   acatccggca ttttttgctg tttcaactgc tttgcatata tcagttaagg aagatcttcc      600
   agtgtcaata ataagcggta atcctgtttt tgccagatga cgaattaaag gaatatggac      660
   aatgttcgat gaagaggttt tgagtgcgca ggccccttgc tttattgcaa aatcaatagt      720
   gtccaattca taaattgaaa ctatgaaggg taaattggca ttctttaata gtgagaatag      780
   catactgtaa tgttctaaag gcagtatttt tctctcaatt aatgagcggt agttttcctt      840
   gcgaacctca cctgatttac tagtataggt ttcttcataa tcaacaggta ggcaaatgtt      900
   tgggttattt aaaatttctg tttttagtat aagaggttga tacggtactt tatctctgat      960
   ggttataatt ttgtgtagca attgttcagc tatgccaaga tcctgattaa aaaaagtacc     1020
   tatttctgct aaaaaaatag gaggatgatt ttctccaata gagaatggtc caattgtaaa     1080
   aggctgactc at                                                         1092
<210> 32
   <211> 1092
   <212> DNA
   <213> Legionella pneumophila
<400> 32
   ctaacaatca tttttttgct taatatccga ccatttaatg ggttcaccta tagatacctt 60
   gttttgtaat tcccagccat ttaccagatc ccagtattgg gtcgggactc ccttacaagg 120
   aaaggcaaag gttgtattat caaggctaat tagagagcca ggttgaagtt tttgctttgc 180
   aaccaaacaa gttctttgag agctgccaag tactcctttt ataggagctt ctagatctcg 240
   catcggttga cctaaagctt gccaacaatt gtaaattgtt tcaagagtct tggctaattg 300
   ctcaatcccc atagtatgtg atatgtcctg atcaatatct tctggataga gatgaactcc 360
   tttttccagg attgaagctc ccaatgcaat agacatatac aacatttcaa ttccattata 420
   atggtcagat aaaccaactg gtaatgaaaa acattggttt aaagttcgta acattcttag 480
   gttatgtgct tctggcaaag caggatgtcc atcaggacta tgttcaataa taattttttc 540
   acatccggca ctttttgctg tttcaactgc tttgcatata tcagttaaag aagatcttcc 600
   agtgtcaata atgagcggta atcctgtttt tgccagatga cgaattaaag gaacatggac 660
   aatgttcgat gaagaggttt tgagtgcgca ggcaccctgc tttatcgcaa aatcaatagt 720
   gtccaattca taaatcgaaa ctatgaaggg taaattggca ttctttaata gtgaaaatag 780
   catactataa tgttctaaag gcagtacttt tctctcaatt aatgagcggt agttttcctt 840
   gcggacctca ccggatttac tggtataggt ttcttcataa tcaacaggta ggcaaatgtt 900
   tgggttattt aaaatttctg tttttagtat aagaggttgg tatggtactt tatccctgat 960
   ggttataatt ttgtgtagca attgttcagc tatgccaaga tcctgattaa aaaaagtacc 1020
   tatttctgct aaaaaaatag gaggatgatt ttcttcaaca gagaatggac caattgtaaa 1080
   aggctgactc at 1092
<210> 33
   <211> 1092
   <212> DNA
   <213> Legionella pneumophila
<400> 33
   ctaacaatca tttttttgct taatatccga ccatttaatg ggttcaccta tagatacctt          60
   gttttgtaat tcccagccat ttaccagatc ccagtattgg gtcgggactc ccttacaagg         120
   aaaggcaaag gttgtattat caaggctaat tagagagcca ggttgaagtt tttgctttgc         180
   aaccaaacaa gttctttgag agctgccaag tactcctttt ataggagctt ctagatctcg         240
   catcggttga cctaaagctt gccaacaatt gtaaattgtt tcaagagtct tggctaattg         300
   ctcaatcccc atagtatgtg atatgtcctg atcaatatct tctggataga gatgaactcc         360
   tttttccagg attgaagctc ccaatgcaat agacatatac aacatttcaa ttccattata         420
   atggtcagat aaaccaactg gtaatgaaaa acattggttt aaagttcgta acattcttag         480
   gttatgtgct tctggcaaag caggatgtcc atcaggacta tgttcaataa taattttttc         540
   acatccggca ctttttgctg tttcaactgc tttgcatata tcagttaaag aagatcttcc        600
   agtgtcaata atgagcggta atcctgtttt tgccagatga cgaattaaag gaacatggac         660
   aatgttcgat gaagaggttt tgagtgcgca ggcaccctgc tttatcgcaa aatcaatagt         720
   gtccaattca taaatcgaaa ctatgaaggg taaattggca ttctttaata gtgaaaatag         780
   catactataa tgttctaaag gcagtacttt tctctcaatt aatgagcggt agttttcctt         840
   gcggacctca ccggatttac tggtataggt ttcttcataa tcaacaggta ggcaaatgtt         900
   tgggttattt aaaatttctg tttttagtat aagaggttgg tatggtactt tatccctgat         960
   ggttataatt ttgtgtagca attgttcagc tatgccaaga tcctgattaa aaaaagtacc        1020
   tatttctgct aaaaaaatag gaggatgatt ttcttcaaca gagaatggac caattgtaaa        1080
   aggctgactc at                                                            1092
<210> 34
   <211> 1092
   <212> DNA
   <213> Legionella pneumophila
<400> 34
   ctaacaatca tttttttgct taatatccga ccatttaatg ggttcaccta tagatacctt        60
   gttttgtaat tcccagccat ttaccagatc ccagtattgg gtcgggactc ccttacaagg       120
   aaaggcaaag gttgtattat caaggctaat tagagagcca ggttgaagtt tttgctttgc       180
   aaccaaacaa gttctttgag agctgccaag tactcctttt ataggagctt ctagatctcg       240
   catcggttga cctaaagctt gccaacaatt gtaaattgtt tcaagagtct tggctaattg       300
   ctcaatcccc atagtatgtg atatgtcctg atcaatatct tctggataga gatgaactcc       360
   tttttccagg attgaagctc ccaatgcaat agacatatac aacatttcaa ttccattata       420
   atggtcagat aaaccaactg gtaatgaaaa acattggttt aaagttcgta acattcttag       480
   gttatgtgct tctggcaaag caggatgtcc atcaggacta tgttcaataa taattttttc       540
   acatccggca ctttttgctg tttcaactgc tttgcatata tcagttaaag aagatcttcc       600
   agtgtcaata atgagcggta atcctgtttt tgccagatga cgaattaaag gaacatggac       660
   aatgttcgat gaagaggttt tgagtgcgca ggcaccctgc tttatcgcaa aatcaatagt       720
   gtccaattca taaatcgaaa ctatgaaggg taaattggca ttctttaata gtgaaaatag       780
   catactataa tgttctaaag gcagtacttt tctctcaatt aatgagcggt agttttcctt       840
   gcggacctca ccggatttac tggtataggt ttcttcataa tcaacaggta ggcaaatgtt       900
   tgggttattt aaaatttctg tttttagtat aagaggttgg tatggtactt tatccctgat       960
   ggttataatt ttgtgtagca attgttcagc tatgccaaga tcctgattaa aaaaagtacc      1020
   tatttctgct aaaaaaatag gaggatgatt ttcttcaaca gagaatggac caattgtaaa      1080
   aggctgactc at                                                          1092
<210> 35
   <211> 1092
   <212> DNA
   <213> Legionella pneumophila
<400> 35
   ctaacaatca tttttttgct taatatccga ccatttaatg ggttcaccta tagatacctt      60
   gttttgtaat tcccagccat ttaccagatc ccagtattgg gtcgggactc ccttacaagg     120
   aaaggcaaag gttgtattat caaggctaat tagagagcca ggttgaagtt tttgctttgc     180
   aaccaaacaa gttctttgag agctgccaag tactcctttt ataggagctt ctagatctcg     240
   catcggttga cctaaagctt gccaacaatt gtaaattgtt tcaagagtct tggctaattg     300
   ctcaatcccc atagtatgtg atatgtcctg atcaatatct tctggataga gatgaactcc     360
   tttttccagg attgaagctc ccaatgcaat agacatatac aacatttcaa ttccattata     420
   atggtcagat aaaccaactg gtaatgaaaa acattggttt aaagttcgta acattcttag     480
   gttatgtgct tctggcaaag caggatgtcc atcaggacta tgttcaataa taattttttc      540
   acatccggca ctttttgctg tttcaactgc tttgcatata tcagttaaag aagatcttcc     600
   agtgtcaata atgagcggta atcctgtttt tgccagatga cgaattaaag gaacatggac     660
   aatgttcgat gaagaggttt tgagtgcgca ggcaccctgc tttatcgcaa aatcaatagt     720
   gtccaattca taaatcgaaa ctatgaaggg taaattggca ttctttaata gtgaaaatag     780
   catactataa tgttctaaag gcagtacttt tctctcaatt aatgagcggt agttttcctt     840
   gcggacctca ccggatttac tggtataggt ttcttcataa tcaacaggta ggcaaatgtt     900
   tgggttattt aaaatttctg tttttagtat aagaggttgg tatggtactt tatccctgat     960
   ggttataatt ttgtgtagca attgttcagc tatgccaaga tcctgattaa aaaaagtacc    1020
   tatttctgct aaaaaaatag gaggatgatt ttcttcaaca gagaatggac caattgtaaa    1080
   aggctgactc at                                                         1092
<210> 36
   <211> 1060
   <212> DNA
   <213> Legionella pneumophila
<400> 36
   atttttttgc ttaatatccg accatttaat gggttcacct atagatacct tgttttgtaa      60
   ttcccagcca tttaccagat cccagtattg ggtcgggact cccttacaag gaaaggcaaa     120
   ggttgtatta tcaaggctaa ttagagagcc aggttgaagt ttttgctttg caaccaaaca     180
   agttctttga gagctgccaa gtactccttt tataggagct tctagatctc gcatcggttg     240
   acctaaagct tgccaacaat tgtaaattgt ttcaagagtc ttggctaatt gctcaatccc     300
   catagtatgt gatatgtcct gatcaatatc ttctggatag agatgaactc ctttttccag     360
   gattgaagct cccaatgcaa tagacatata caacatttca attccattat aatggtcaga     420
   taaaccaact ggtaatgaaa aacattggtt taaagttcgt aacattctta ggttatgtgc     480
   ttctggcaaa gcaggatgtc catcaggact atgttcaata ataatttttt cacatccggc     540
   actttttgct gtttcaactg ctttgcatat atcagttaaa gaagatcttc cagtgtcaat     600
   aatgagcggt aatcctgttt ttgccagatg acgaattaaa ggaacatgga caatgttcga     660
   tgaagaggtt ttgagtgcgc aggcaccctg ctttatcgca aaatcaatag tgtccaattc     720
   ataaatcgaa actatgaagg gtaaattggc attctttaat agtgaaaata gcatactata     780
   atgttctaaa ggcagtactt ttctctcaat taatgagcgg tagttttcct tgcggacctc     840
   accggattta ctggtatagg tttcttcata atcaacaggt aggcaaatgt ttgggttatt     900
   taaaatttct gtttttagta taagaggttg gtatggtact ttatccctga tggttataat     960
   tttgtgtagc aattgttcag ctatgccaag atcctgatta aaaaaagtac ctatttctgc    1020
   taaaaaaata ggaggatgat tttcttcaac agagaatgga                          1060
<210> 37
   <211> 1060
   <212> DNA
   <213> Legionella pneumophila
<400> 37
   atttttttgc ttaatatctg accatttaat gggctcacct atagatacct tgttttgtag       60
   ttcccagcca tttaccagat cccagtattg ggtaggaaat cccttacaag gaaaggcaaa      120
   ggttgtatta tcaaggctaa ttagagagcc gggttgaagc ttttgctttg caaccaaaca      180
   agttctttga gagctgccaa gtactccttt tataggggtt tctagatctc gcatcggttg      240
   acctaaagct tgccaacaat tgtaaattgt ttcaagagtc ttggctaatt gatcaatccc      300
   catagtatgt gatatgtcct gatcaatatc ttctggatag agatgaaccc ctttttccag      360
   gattgaagct cccaatgcaa tagacatata caacatttca attccattat aatggtcaga      420
   taatccaact ggtaatgaaa aacattggtt taaagttcgt aacattctta agttatgtgc      480
   ttctggcaaa gcaggatgtc catcaggact atgttcaata ataatttttt cacatccggc      540
   attttttgct gtttcaactg ctttgcatat atcagttaag gaagatcttc cagtgtcaat      600
   aataagcggt aatcctgttt ttgccagatg acgaattaaa ggaatatgga caatgttcga      660
   tgaagaggtt ttgagtgcgc aggccccttg ctttattgca aaatcaatag tgtccaattc      720
   ataaattgaa actatgaagg gtaaattggc attctttaat agtgagaata gcatactgta      780
   atgttctaaa ggcagtattt ttctctcaat taatgagcgg tagttttcct tgcgaacctc      840
   acctgattta ctagtatagg tttcttcata atcaacaggt aggcaaatgt ttgggttatt      900
   taaaatttct gtttttagta taagaggttg atacggtact ttatctctga tggttataat      960
   tttgtgtagc aattgttcag ctatgccaag atcctgatta aaaaaagtac ctatttctgc     1020
   taaaaaaata ggaggatgat tttctccaat agagaatggt                           1060
<210> 38
   <211> 1060
   <212> DNA
   <213> Legionella pneumophila
<400> 38
   atttttttgc ttaatatccg accatttaat gggttcacct atagatacct tgttttgtaa       60
   ttcccagcca tttaccagat cccagtattg ggtcgggact cccttacaag gaaaggcaaa      120
   ggttgtatta tcaaggctaa ttagagagcc aggttgaagt ttttgctttg caaccaaaca      180
   agttctttga gagctgccaa gtactccttt tataggagct tctagatctc gcatcggttg      240
   acctaaagct tgccaacaat tgtaaattgt ttcaagagtc ttggctaatt gctcaatccc      300
   catagtatgt gatatgtcct gatcaatatc ttctggatag agatgaactc ctttttccag      360
   gattgaagct cccaatgcaa tagacatata caacatttca attccattat aatggtcaga      420
   taaaccaact ggtaatgaaa aacattggtt taaagttcgt aacattctta ggttatgtgc      480
   ttctggcaaa gcaggatgtc catcaggact atgttcaata ataatttttt cacatccggc      540
   actttttgct gtttcaactg ctttgcatat atcagttaaa gaagatcttc cagtgtcaat      600
   aatgagcggt aatcctgttt ttgccagatg acgaattaaa ggaacatgga caatgttcga      660
   tgaagaggtt ttgagtgcgc aggcaccctg ctttatcgca aaatcaatag tgtccaattc      720
   ataaatcgaa actatgaagg gtaaattggc attctttaat agtgaaaata gcatactata      780
   atgttctaaa ggcagtactt ttctctcaat taatgagcgg tagttttcct tgcggacctc      840
   accggattta ctggtatagg tttcttcata atcaacaggt aggcaaatgt ttgggttatt      900
   taaaatttct gtttttagta taagaggttg gtatggtact ttatccctga tggttataat      960
   tttgtgtagc aattgttcag ctatgccaag atcctgatta aaaaaagtac ctatttctgc     1020
   taaaaaaata ggaggatgat tttcttcaac agagaatgga                           1060
<210> 39
   <211> 1060
   <212> DNA
   <213> Legionella pneumophila
<400> 39
   atttttttgc ttaatatccg accatttaat gggttcacct atagatacct tgttttgtaa 60
   ttcccagcca tttaccagat cccagtattg ggtcgggact cccttacaag gaaaggcaaa 120
   ggttgtatta tcaaggctaa ttagagagcc aggttgaagt ttttgctttg caaccaaaca 180
   agttctttga gagctgccaa gtactccttt tataggagct tctagatctc gcatcggttg 240
   acctaaagct tgccaacaat tgtaaattgt ttcaagagtc ttggctaatt gctcaatccc 300
   catagtatgt gatatgtcct gatcaatatc ttctggatag agatgaactc ctttttccag 360
   gattgaagct cccaatgcaa tagacatata caacatttca attccattat aatggtcaga 420
   taaaccaact ggtaatgaaa aacattggtt taaagttcgt aacattctta ggttatgtgc 480
   ttctggcaaa gcaggatgtc catcaggact atgttcaata ataatttttt cacatccggc 540
   actttttgct gtttcaactg ctttgcatat atcagttaaa gaagatcttc cagtgtcaat 600
   aatgagcggt aatcctgttt ttgccagatg acgaattaaa ggaacatgga caatgttcga 660
   tgaagaggtt ttgagtgcgc aggcaccctg ctttatcgca aaatcaatag tgtccaattc 720
   ataaatcgaa actatgaagg gtaaattggc attctttaat agtgaaaata gcatactata 780
   atgttctaaa ggcagtactt ttctctcaat taatgagcgg tagttttcct tgcggacctc 840
   accggattta ctggtatagg tttcttcata atcaacaggt aggcaaatgt ttgggttatt 900
   taaaatttct gtttttagta taagaggttg gtatggtact ttatccctga tggttataat 960
   tttgtgtagc aattgttcag ctatgccaag atcctgatta aaaaaagtac ctatttctgc 1020
   taaaaaaata ggaggatgat tttcttcaac agagaatgga 1060
<210> 40
   <211> 1060
   <212> DNA
   <213> Legionella pneumophila
<400> 40
   atttttttgc ttaatatccg accatttaat gggttcacct atagatacct tgttttgtaa      60
   ttcccagcca tttaccagat cccagtattg ggtcgggact cccttacaag gaaaggcaaa     120
   ggttgtatta tcaaggctaa ttagagagcc aggttgaagt ttttgctttg caaccaaaca     180
   agttctttga gagctgccaa gtactccttt tataggagct tctagatctc gcatcggttg     240
   acctaaagct tgccaacaat tgtaaattgt ttcaagagtc ttggctaatt gctcaatccc     300
   catagtatgt gatatgtcct gatcaatatc ttctggatag agatgaactc ctttttccag     360
   gattgaagct cccaatgcaa tagacatata caacatttca attccattat aatggtcaga     420
   taaaccaact ggtaatgaaa aacattggtt taaagttcgt aacattctta ggttatgtgc     480
   ttctggcaaa gcaggatgtc catcaggact atgttcaata ataatttttt cacatccggc     540
   actttttgct gtttcaactg ctttgcatat atcagttaaa gaagatcttc cagtgtcaat     600
   aatgagcggt aatcctgttt ttgccagatg acgaattaaa ggaacatgga caatgttcga     660
   tgaagaggtt ttgagtgcgc aggcaccctg ctttatcgca aaatcaatag tgtccaattc     720
   ataaatcgaa actatgaagg gtaaattggc attctttaat agtgaaaata gcatactata     780
   atgttctaaa ggcagtactt ttctctcaat taatgagcgg tagttttcct tgcggacctc     840
   accggattta ctggtatagg tttcttcata atcaacaggt aggcaaatgt ttgggttatt     900
   taaaatttct gtttttagta taagaggttg gtatggtact ttatccctga tggttataat     960
   tttgtgtagc aattgttcag ctatgccaag atcctgatta aaaaaagtac ctatttctgc    1020
   taaaaaaata ggaggatgat tttcttcaac agagaatgga                          1060
<210> 41
   <211> 1060
   <212> DNA
   <213> Legionella pneumophila
<400> 41
   atttttttgc ttaatatccg accatttaat gggttcacct atagatacct tgttttgtaa      60
   ttcccagcca tttaccagat cccagtattg ggtcgggact cccttacaag gaaaggcaaa     120
   ggttgtatta tcaaggctaa ttagagagcc aggttgaagt ttttgctttg caaccaaaca     180
   agttctttga gagctgccaa gtactccttt tataggagct tctagatctc gcatcggttg     240
   acctaaagct tgccaacaat tgtaaattgt ttcaagagtc ttggctaatt gctcaatccc     300
   catagtatgt gatatgtcct gatcaatatc ttctggatag agatgaactc ctttttccag 360
   gattgaagct cccaatgcaa tagacatata caacatttca attccattat aatggtcaga 420
   taaaccaact ggtaatgaaa aacattggtt taaagttcgt aacattctta ggttatgtgc 480
   ttctggcaaa gcaggatgtc catcaggact atgttcaata ataatttttt cacatccggc 540
   actttttgct gtttcaactg ctttgcatat atcagttaaa gaagatcttc cagtgtcaat 600
   aatgagcggt aatcctgttt ttgccagatg acgaattaaa ggaacatgga caatgttcga 660
   tgaagaggtt ttgagtgcgc aggcaccctg ctttatcgca aaatcaatag tgtccaattc 720
   ataaatcgaa actatgaagg gtaaattggc attctttaat agtgaaaata gcatactata 780
   atgttctaaa ggcagtactt ttctctcaat taatgagcgg tagttttcct tgcggacctc 840
   accggattta ctggtatagg tttcttcata atcaacaggt aggcaaatgt ttgggttatt 900
   taaaatttct gtttttagta taagaggttg gtatggtact ttatccctga tggttataat 960
   tttgtgtagc aattgttcag ctatgccaag atcctgatta aaaaaagtac ctatttctgc 1020
   taaaaaaata ggaggatgat tttcttcaac agagaatgga 1060
<210> 42
   <211> 1060
   <212> DNA
   <213> Legionella pneumophila
<400> 42
   atttttttgc ttaatatctg accatttaat gggctcacct atagatacct tgttttgtag      60
   ttcccagcca tttaccagat cccagtattg ggtaggaaat cccttacaag gaaaggcaaa     120
   ggttgtatta tcaaggctaa ttagagagcc gggttgaagc ttttgctttg caaccaaaca     180
   agttctttga gagctgccaa gtactccttt tataggggtt tctagatctc gcatcggttg     240
   acctaaagct tgccaacaat tgtaaattgt ttcaagagtc ttggctaatt gatcaatccc     300
   catagtatgt gatatgtcct gatcaatatc ttctggatag agatgaaccc ctttttccag     360
   gattgaagct cccaatgcaa tagacatata caacatttca attccattat aatggtcaga     420
   taatccaact ggtaatgaaa aacattggtt taaagttcgt aacattctta agttatgtgc     480
   ttctggcaaa gcaggatgtc catcaggact atgttcaata ataatttttt cacatccggc     540
   attttttgct gtttcaactg ctttgcatat atcagttaag gaagatcttc cagtgtcaat      600
   aataagcggt aatcctgttt ttgccagatg acgaattaaa ggaatatgga caatgttcga     660
   tgaagaggtt ttgagtgcgc aggccccttg ctttattgca aaatcaatag tgtccaattc     720
   ataaattgaa actatgaagg gtaaattggc attctttaat agtgagaata gcatactgta     780
   atgttctaaa ggcagtattt ttctctcaat taatgagcgg tagttttcct tgcgaacctc     840
   acctgattta ctagtatagg tttcttcata atcaacaggt aggcaaatgt ttgggttatt     900
   taaaatttct gtttttagta taagaggttg atacggtact ttatctctga tggttataat     960
   tttgtgtagc aattgttcag ctatgccaag atcctgatta aaaaaagtac ctatttctgc    1020
   taaaaaaata ggaggatgat tttctccaat agagaatggt                          1060
<210> 43
   <211> 1060
   <212> DNA
   <213> Legionella pneumophila
<400> 43
   atttttttgc ttaatatctg accatttaat gggctcacct atagatacct tgttttgtag 60
   ttcccagcca tttaccagat cccagtattg ggtaggaact cccttacaag gaaaggcaaa 120
   ggttgtatta tcaaggctaa ttagagagcc gggttgaagc ttttgctttg caaccaaaca 180
   agttctttga gagctgccaa gtactccttt tataggggtt tccagatctc gcattggttg 240
   acctaaagct tgccaacaat tgtaaattgt ttcaagagtc ttggctaatt gatcaatccc 300
   catagtatgt gatatgtcct gatcaatatc ttctggatag agatgaaccc ctttttccag 360
   gattgaagct cccaatgcaa tagacatata caacatttca attccattat aatggtcaga 420
   taatccaact ggtaatgaaa aacattggtt taaagttcgt aacattctta agttatgtgc 480
   ttctggcaaa gcaggatggc catcaggact atgttcaata ataatttttt cacatccggc 540
   attttttgct gtttcaactg ctttgcatat atcagttaag gaagatcttc cagtgtcaat 600
   aatgagcggt aatcctgttt ttgccagatg acgaattaaa ggaatatgga caatgtttga 660
   tgaagaggtt ttgagtgcgc aggccccttg ctttattgca aaatcaatag tgtccaattc 720
   ataaattgaa actatgaagg gtaaattggc attctttaat agtgagaata gcatactgta 780
   atgttctaaa ggcagtattt ttctctcaat taatgagcgg tagttttcct tgcgaacctc 840
   acctgattta ctagtgtagg tttcttcata atcaacaggt aggcaaatgt ttgggttatt 900
   taaaatttct gtttttagta taagaggttg atacggtact ttatctctga tggttataat 960
   tttgtgtagc aattgttcag ctatgccaag atcctgatta aaaaaagtac ctatttctgc 1020
   taaaaaaata ggaggatgat tttctccaat agagaatggt 1060
<210> 44
   <211> 1060
   <212> DNA
   <213> Legionella pneumophila
<400> 44
   atttttttgc ttaatatccg accatttaat gggttcacct atagatacct tgttttgtaa 60
   ttcccagcca tttaccagat cccagtattg ggtcgggact cccttacaag gaaaggcaaa 120
   ggttgtatta tcaaggctaa ttagagagcc aggttgaagt ttttgctttg caaccaaaca 180
   agttctttga gagctgccaa gtactccttt tataggagct tctagatctc gcatcggttg 240
   acctaaagct tgccaacaat tgtaaattgt ttcaagagtc ttggctaatt gctcaatccc      300
   catagtatgt gatatgtcct gatcaatatc ttctggatag agatgaactc ctttttccag      360
   gattgaagct cccaatgcaa tagacatata caacatttca attccattat aatggtcaga      420
   taaaccaact ggtaatgaaa aacattggtt taaagttcgt aacattctta ggttatgtgc      480
   ttctggcaaa gcaggatgtc catcaggact atgttcaata ataatttttt cacatccggc      540
   actttttgct gtttcaactg ctttgcatat atcagttaaa gaagatcttc cagtgtcaat      600
   aatgagcggt aatcctgttt ttgccagatg acgaattaaa ggaacatgga caatgttcga      660
   tgaagaggtt ttgagtgcgc aggcaccctg ctttatcgca aaatcaatag tgtccaattc      720
   ataaatcgaa actatgaagg gtaaattggc attctttaat agtgaaaata gcatactata      780
   atgttctaaa ggcagtactt ttctctcaat taatgagcgg tagttttcct tgcggacctc      840
   accggattta ctggtatagg tttcttcata atcaacaggt aggcaaatgt ttgggttatt      900
   taaaatttct gtttttagta taagaggttg gtatggtact ttatccctga tggttataat      960
   tttgtgtagc aattgttcag ctatgccaag atcctgatta aaaaaagtac ctatttctgc     1020
   taaaaaaata ggaggatgat tttcttcaac agagaatgga                           1060
<210> 45
   <211> 1060
   <212> DNA
   <213> Legionella pneumophila
<400> 45
   atttttttgc ttaatatccg accatttaat gggttcacct atagatacct tgttttgtaa 60
   ttcccagcca tttaccagat cccagtattg ggtaggaact cccttacaag gaaaggcaaa 120
   ggttgtatta tcaaggctaa ttagagagcc gggttgaagt ttttgctttg caaccaaaca 180
   agttctttga gagctgccaa gtactccttt tataggggtt tctagatctc gcattggttg 240
   acctaaagct tgccaacaat tgtaaattgt ttcaagagtc ttggctaatt gatcaatccc 300
   catagtatgt gatatgtcct gatcgatatc ttctggatag agatgaactc ctttttccag 360
   gattgaagct cccaatgcaa tagacatata caacatttca attccattat aatggtcaga 420
   taaaccaact ggtaatgaaa aacattggtt taaagttcgt aacattctta ggttatgtgc 480
   ttctggcaaa gcaggatgtc catcagggct atgttcaata ataatttttt cacatccggc 540
   actttttgct gtttcaactg ctttgcatat atcagttaag gaagatcttc cagtgtcaat 600
   aatgagcggt aatcctgttt ttgccagatg acgaattaaa ggaatatgga caatgttcga 660
   tgaagaggtt ttgagtgcgc aggccccctg ctttatcgca aaatcaatag tgtccaattc 720
   ataaatcgaa actatgaagg gtaaattggc attctttaat agtgaaaata gcatactata 780
   atgttctaaa ggcagtactt ttctctcaat taatgagcgg tagttttcct tgcgaacctc 840
   acctgattta ctggtatagg tttcttcata atcaacaggt aggcaaatgt ttgggttatt 900
   taaaatttct gtttttagta taagaggttg gtatggtact ttatccctga tggttataat 960
   tttgtgtagc aattgttcag ctatgccaag atcctgatta aaaaaagtac ctatttctgc 1020
   taaaaaaata ggaggatgat tttcttcaac agagaatgga 1060
<210> 46
   <211> 1060
   <212> DNA
   <213> Legionella pneumophila
<400> 46
   atttttttgc ttaatatctg accatttaat gggctcacct atagatacct tgttttgtag 60
   ttcccagcca tttaccagat cccagtattg ggtaggaact cccttacaag gaaaggcaaa 120
   ggttgtatta tcaaggctaa ttagagagcc gggttgaagc ttttgctttg caaccaaaca 180
   agttctttga gagctgccaa gtactccttt tataggggtt tccagatctc gcattggttg 240
   acctaaagct tgccaacaat tgtaaattgt ttcaagagtc ttggctaatt gatcaatccc 300
   catagtatgt gatatgtcct gatcaatatc ttctggatag agatgaaccc ctttttccag 360
   gattgaagct cccaatgcaa tagacatata caacatttca attccattat aatggtcaga 420
   taatccaact ggtaatgaaa aacattggtt taaagttcgt aacattctta agttatgtgc 480
   ttctggcaaa gcaggatggc catcaggact atgttcaata ataatttttt cacatccggc 540
   attttttgct gtttcaactg ctttgcatat atcagttaag gaagatcttc cagtgtcaat 600
   aatgagcggt aatcctgttt ttgccagatg acgaattaaa ggaatatgga caatgtttga 660
   tgaagaggtt ttgagtgcgc aggccccttg ctttattgca aaatcaatag tgtccaattc 720
   ataaattgaa actatgaagg gtaaattggc attctttaat agtgagaata gcatactgta 780
   atgttctaaa ggcagtattt ttctctcaat taatgagcgg tagttttcct tgcgaacctc 840
   acctgattta ctagtgtagg tttcttcata atcaacaggt aggcaaatgt ttgggttatt 900
   taaaatttct gtttttagta taagaggttg atacggtact ttatctctga tggttataat 960
   tttgtgtagc aattgttcag ctatgccaag atcctgatta aaaaaagtac ctatttctgc 1020
   taaaaaaata ggaggatgat tttctccaat agagaatggt 1060
<210> 47
   <211> 1113
   <212> DNA
   <213> Legionella pneumophila
<400> 47
   gatagtcaat gagtcagcct tttacaattg gtccattctc tgttgaagaa aatcatcctc 60
   ctattttttt agcagaaata ggtacttttt ttaatcagga tcttggcata gctgaacaat 120
   tgctacacaa aattataacc atcagggata aagtaccata ccaacctctt atactaaaaa      180
   cagaaatttt aaataaccca aacatttgcc tacctgttga ttatgaagaa acctatacca      240
   gtaaatccgg tgaggtccgc aaggaaaact accgctcatt aattgagaga aaagtactgc      300
   ctttagaaca ttatagtatg ctattttcac tattaaagaa tgccaattta cccttcatag      360
   tttcgattta tgaattggac actattgatt ttgcgataaa gcagggtgcc tgcgcactca      420
   aaacctcttc atcgaacatt gtccatgttc ctttaattcg tcatctggca aaaacaggat      480
   taccgctcat tattgacact ggaagatctt ctttaactga tatatgcaaa gcagttgaaa      540
   cagcaaaaag tgccggatgt gaaaaaatta ttattgaaca tagtcctgat ggacatcctg      600
   ctttgccaga agcacataac ctaagaatgt tacgaacttt aaaccaatgt ttttcattac      660
   cagttggttt atctgaccat tataatggaa ttgaaatgtt gtatatgtct attgcattgg      720
   gagcttcaat cctggaaaaa ggagttcatc tctatccaga agatattgat caggacatat      780
   cacatactat ggggattgag caattagcca agactcttga aacaatttac aattgttggc      840
   aagctttagg tcaaccgatg cgagatctag aagctcctat aaaaggagta cttggcagct      900
   ctcaaagaac ttgtttggtt gcaaagcaaa aacttcaacc tggctctcta attagccttg      960
   ataatacaac ctttgccttt ccttgtaagg gagtcccgac ccaatactgg gatctggtaa     1020
   atggctggga attacaaaac aaggtatcta taggtgaacc cattaaatgg tcggatatta     1080
   agcaaaaaaa tgattgttag aaaccctact tgg                                 1113
<210> 48
   <211> 1113
   <212> DNA
   <213> Legionella pneumophila
<400> 48
   gatagtcaat gagtcagcct tttacaattg gtccattctc tgttgaagaa aatcatcctc      60
   ctattttttt agcagaaata ggtacttttt ttaatcagga tcttggcata gctgaacaat     120
   tgctacacaa aattataacc atcagggata aagtaccata ccaacctctt atactaaaaa     180
   cagaaatttt aaataaccca aacatttgcc tacctgttga ttatgaagaa acctatacca     240
   gtaaatccgg tgaggtccgc aaggaaaact accgctcatt aattgagaga aaagtactgc     300
   ctttagaaca ttatagtatg ctattttcac tattaaagaa tgccaattta cccttcatag     360
   tttcgattta tgaattggac actattgatt ttgcgataaa gcagggtgcc tgcgcactca     420
   aaacctcttc atcgaacatt gtccatgttc ctttaattcg tcatctggca aaaacaggat     480
   taccgctcat tattgacact ggaagatctt ctttaactga tatatgcaaa gcagttgaaa     540
   cagcaaaaag tgccggatgt gaaaaaatta ttattgaaca tagtcctgat ggacatcctg     600
   ctttgccaga agcacataac ctaagaatgt tacgaacttt aaaccaatgt ttttcattac     660
   cagttggttt atctgaccat tataatggaa ttgaaatgtt gtatatgtct attgcattgg     720
   gagcttcaat cctggaaaaa ggagttcatc tctatccaga agatattgat caggacatat     780
   cacatactat ggggattgag caattagcca agactcttga aacaatttac aattgttggc     840
   aagctttagg tcaaccgatg cgagatctag aagctcctat aaaaggagta cttggcagct     900
   ctcaaagaac ttgtttggtt gcaaagcaaa aacttcaacc tggctctcta attagccttg     960
   ataatacaac ctttgccttt ccttgtaagg gagtcccgac ccaatactgg gatctggtaa    1020
   atggctggga attacaaaac aaggtatcta taggtgaacc cattaaatgg tcggatatta    1080
   agcaaaaaaa tgattgttag aaaccctact tgg                                 1113
<210> 49
   <211> 18
   <212> DNA
   <213> Unknown
<220>
   <223> Synthetic oligonucleotide
<400> 49
   gatcaatacg aagcaggc 18
<210> 50
   <211> 20
   <212> DNA
   <213> Unknown
<220>
   <223> Synthetic oligonucleotide
<400> 50
   ccttgycttt caacacttcc 20
<210> 51
   <211> 25
   <212> DNA
   <213> Unknown
<220>
   <223> Synthetic oligonucleotide
<400> 51
   catccactca ttttattcct gatcc 25
<210> 52
   <211> 25
   <212> DNA
   <213> Unknown
<220>
   <223> Synthetic oligonucleotide
<400> 52
   catctactca ttttattcct gatcc 25